# EUROPEAN PATENT SPECIFICATION

(11) **EP 3 406 585 B1**
(45) Date of publication and mention of the grant of the patent: **29.07.2020**
(21) Application number: 16886514.5
(22) Date of filing: 19.12.2016
(51) Int. Cl.: C07C 43/225, C07D 309/06, C09K 19/12, C09K 19/14, C09K 19/18, C09K 19/20, C09K 19/30, C09K 19/32, C09K 19/34, C09K 19/42, G02F 1/13, C09K 19/04

(54) **TETRACYCLIC LIQUID CRYSTAL COMPOUND HAVING DIATOMIC BONDING GROUP AND 2,3-DIFLUOROPHENYLENE, LIQUID CRYSTAL COMPOSITION AND LIQUID CRYSTAL DISPLAY DEVICE**
TETRACYCLISCHE FLUSSIGKRISTALLINE VERBINDUNG MIT DIATOMISCHE BINDUNGSGRUPPE UND 2,3-DIFLUORPHENYLEN, FLUSSIGKRISTALLZUSAMMENSETZUNG UND FLUSSIGKRISTALLANZEIGEELEMENT
CCOMPOSE CRISTALLIN LIQUID TETRACYCLIQUE AYANT UN GROUPE DE LIAISON DIATOMIQUE ET DU 2,3-DIFLUOROPHENYLENE, COMPOSITION À BASE DE CRISTAUX LIQUIDES ET ELEMENT D'AFFICHAGE À BASE DE CRISTAUX LIQUIDES

(30) Priority: 20.01.2016 JP 2016008895
(43) Date of publication of application: 28.11.2018
(73) Proprietor: JNC Corporation, Chiyoda-ku Tokyo 100-8105 (JP); JNC Petrochemical Corporation, Tokyo 100-0004 (JP)
(72) Inventor: MORI, Ayako, Ichihara-shi Chiba 290-8551 (JP); KIMURA, Keiji, Ichihara-shi Chiba 290-8551 (JP); SATOU, Teizi, Minamata-shi Kumamoto 867-0053 (JP)
(74) Representative: Müller-Boré & Partner Patentanwälte PartG mbB
(86) International application number: PCT/JP2016/087798
(87) International publication number: WO 2017/126275

(56) References cited:
- WO-A1-2014/192656
- CN-A- 101 928 199
- CN-A- 104 419 427
- JP-A- H09 157 202
- JP-A- 2001 500 894
- JP-A- 2008 088 165
- JP-A- 2008 273 957
- JP-A- 2010 215 609
- JP-A- 2014 234 357
- JP-A- 2015 063 684
- JP-A- 2015 507 672
- US-A1- 2015 368 558

## Description

The invention relates to a liquid crystal compound, a liquid crystal composition and a liquid crystal display device. More specifically, the invention relates to a liquid crystal compound having 2,3-difluorophenylene and negative dielectric anisotropy, a liquid crystal composition containing the liquid crystal compound, and a liquid crystal display device including the composition.

In a liquid crystal display device, a classification based on an operating mode for liquid crystal molecules includes a phase change (PC) mode, a twisted nematic (TN) mode, a super twisted nematic (STN) mode, an electrically controlled birefringence (ECB) mode, an optically compensated bend (OCB) mode, an in-plane switching (IPS) mode, a vertical alignment (VA) mode, a fringe field switching (FFS) mode and a field-induced photo-reactive alignment (FPA) mode. A classification based on a driving mode in the device includes a passive matrix (PM) and an active matrix (AM). The PM is classified into static, multiplex and so forth, and the AM is classified into a thin film transistor (TFT), a metal insulator metal (MIM) and so forth.

A liquid crystal composition is sealed into the device. Physical properties of the composition relate to characteristics in the device. Specific examples of the physical properties in the composition include stability to heat or light, a temperature range of a nematic phase, viscosity, optical anisotropy, dielectric anisotropy, specific resistance and an elastic constant. The composition is prepared by mixing many liquid crystal compounds. Physical properties required for a compound include high stability to environment such as water, air, heat and light, a wide temperature range of a liquid crystal phase, small viscosity, suitable optical anisotropy, large dielectric anisotropy, a suitable elastic constant and good compatibility with other liquid crystal compounds. A compound having high maximum temperature of the nematic phase is preferred. A compound having low minimum temperature in the liquid crystal phase such as the nematic phase and a smectic phase is preferred. A compound having small viscosity contributes to a short response time in the device. A suitable value of optical anisotropy depends on a mode of the device. A compound having large positive or negative dielectric anisotropy is preferred for driving the device at low voltage. A compound having good compatibility with other liquid crystal compounds is preferred for preparing the composition. The device may be occasionally used at a temperature below freezing point, and therefore a compound having good compatibility at low temperature is preferred.

Many liquid crystal compounds have been so far prepared. Development of a new liquid crystal compound has been still continued. The reason is that good physical properties that are not found in conventional compounds are expected from a new compound. The reason is that the new compound may be occasionally provided with a suitable balance regarding at least two physical properties in the composition. Compounds each having a tetracyclic ring as described below are reported.

WO 2010/082558 A discloses compound (A) on page 300.

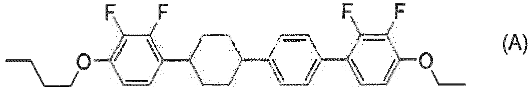

WO 98/27036 A discloses compound (B) on page 65.

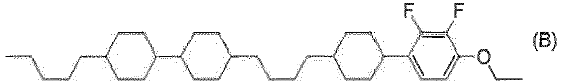

WO 2009/150966 A discloses compound (C) on page 213.

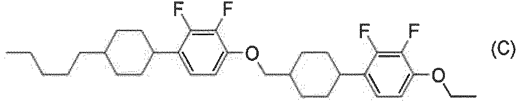

WO 2009/031437 A discloses compound (D) on page 233.

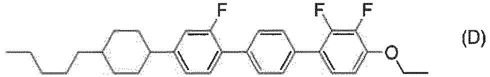

JP 2014-114276 A discloses compound (E) on page 63.

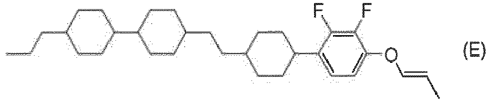

JP 2002-193853 A discloses compound (F) on page 103.

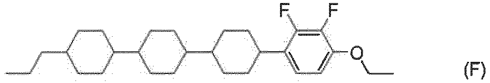

CN 102888226 A1 discloses compound (G) on page 6.

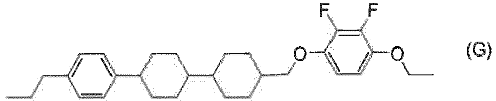

CN 101928199 A1 discloses compound (H) on page 10.

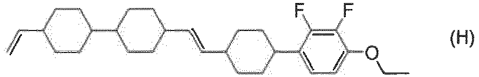

WO 2010/095506 A discloses compound (I) on page 64.

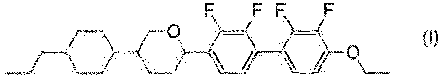

Compound No.1-3-51 described in JP 2010-215609 A on page 54 is compound (J).

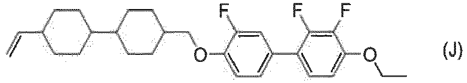

Compounds (A) to (J) are disclosed, but liquid crystal performance is not known.

Patent literature No. 1: WO 2010/082558 A.
Patent literature No. 2: WO 98/27036 A.
Patent literature No. 3: WO 2009/150966 A.
Patent literature No. 4: WO 2009/031437 A.
Patent literature No. 5: JP 2014-114276 A.
Patent literature No. 6: JP 2002-193853 A.
Patent literature No. 7: CN 102888226 A1.
Patent literature No. 8: CN 101928199 A1.
Patent literature No. 9: WO 2010/095506 A.
Patent literature No. 10: JP 2010-215609 A.

JP 2014 234357 A describes a compound, a liquid crystal composition, and a display element.

JP 2015 063684 A describes a liquid crystal composition and an optical element.

WO 2014/192656 A1 describes a difluorophenyl liquid crystal composition.

JP 2008 273957 A describes an alkadienyl-group-containing compound and a liquid crystal composition using the same.

CN 104 419 427 A describes a liquid crystal composition and the application thereof.

US 2015/368558 A1 describes a polymer stable alignment type liquid crystal composition and the use thereof.

JP 2015 507672 A describes a high scattering smectic liquid crystal material and a display device using the same.

CN 101 928 199 A describes a divinyl liquid crystal compound and a preparation method thereof.

JP H09 157202 A describes an alkenyl compound having an oxygen atom-containing binding group, a liquid crystal composition and a liquid crystal display element.

JP 2010 215609 A describes a liquid crystal compound having negative dielectric anisotropy and a liquid crystal composition and a liquid crystal display element each comprising the same.

JP 2008 088165 A describes a cyclohexene derivative having an alkenyl group, a liquid crystal composition and a liquid crystal display device.

JP 2001 500894 A describes a compound having an alkadienyl group as a side chain, and a liquid crystal composition using the same.

A first object is to provide a liquid crystal compound satisfying at least one of physical properties such as high stability to heat or light, a high clearing point (or high maximum temperature of a nematic phase), low minimum temperature of a liquid crystal phase, small viscosity, suitable optical anisotropy, large negative dielectric anisotropy, a suitable elastic constant and good compatibility with other liquid crystal compounds. The object is to provide a compound having better physical properties in comparison with a similar compound. A second object is to provide a liquid crystal composition that contains the compound and satisfies at least one of physical properties such as high stability to heat or light, high maximum temperature of a nematic phase, low minimum temperature of the nematic phase, small viscosity, suitable optical anisotropy, large negative dielectric anisotropy, large specific resistance and a suitable elastic constant. The object is to provide a liquid crystal composition having a suitable balance regarding at least two of the physical properties. A third object is to provide a liquid crystal display device including the composition, and having a wide temperature range in which the device can be used, a short response time, a large voltage holding ratio, low threshold voltage, a large contrast ratio, a small flicker rate and a long service life.

The invention concerns a compound represented by formula (1), a liquid crystal composition containing the compound, and a liquid crystal display device including the composition: 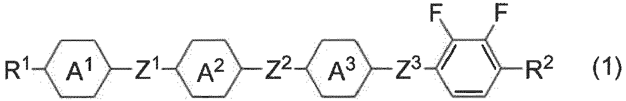 wherein, in formula (1),
R¹ is alkyl having 1 to 15 carbons, and in the alkyl, at least one piece of -CH₂- may be replaced by -O-, and one piece of -CH₂CH₂- may be replaced by -CH=CH-, and R² is alkyl having 1 to 15 carbons, alkoxy having 1 to 15 carbons or alkenyl having 2 to 15 carbons;
ring A¹ and ring A² are independently 1,4-cyclohexylene, 1,4-phenylene, tetrahydropyran-2,5-diyl, 1,3-dioxane-2,5-diyl pyridine-2,5-diyl or pyrimidine-2,5-diyl, and ring A3 is 1,4-cyclohexylene, 1,4-phenylene, 2-fluoro-1,4-phenylene, tetrahydropyran-2,5-diyl, 1,3-dioxane-2,5-diyl, pyridine-2,5-diyl or pyrimidine-2,5-diyl; and
Z¹, Z² and Z³ are independently a single bond, -COO-, -OCO-, -CH₂CH₂- or -CH=CH-, and at least one of Z¹, Z² and Z³ is -COO-, -OCO-, -CH₂CH₂- or -CH=CH-.

A first advantage is to provide a liquid crystal compound satisfying at least one of physical properties such as high stability to heat or light, a high clearing point (or high maximum temperature of a nematic phase), low minimum temperature of a liquid crystal phase, small viscosity, suitable optical anisotropy, large negative dielectric anisotropy, a suitable elastic constant and good compatibility with other liquid crystal compounds. The advantage is to provide a compound having larger dielectric anisotropy in comparison with a similar compound. The advantage is to provide a compound having better compatibility with other liquid crystal compounds, larger negative dielectric anisotropy and smaller viscosity in comparison with a similar compound (Comparative Examples 1 and 2). A second advantage is to provide a liquid crystal composition that contains the compound and satisfies at least one of physical properties such as high stability to heat or light, high maximum temperature of a nematic phase, low minimum temperature of the nematic phase, small viscosity, suitable optical anisotropy, large negative dielectric anisotropy, large specific resistance and a suitable elastic constant. The advantage is to provide a liquid crystal composition having a suitable balance regarding at least two of the physical properties. A third advantage is to provide a liquid crystal display device including the composition, and having a wide temperature range in which the device can be used, a short response time, a large voltage holding ratio, low threshold voltage, a large contrast ratio, a small flicker rate and a long service life.

Usage of terms herein is as described below. Terms "liquid crystal compound," "liquid crystal composition" and "liquid crystal display device" may be occasionally abbreviated as "compound," "composition" and "device," respectively. "Liquid crystal compound" is a generic term for a compound having a liquid crystal phase such as a nematic phase and a smectic phase, and a compound having no liquid crystal phase but to be added for the purpose of adjusting physical properties of a composition such as maximum temperature, minimum temperature, viscosity and dielectric anisotropy. The compound has a six-membered ring such as 1,4-cyclohexylene and 1,4-phenylene, and has rod-like molecular structure. "Liquid crystal display device" is a generic term for a liquid crystal display panel and a liquid crystal display module. "Polymerizable compound" is a compound to be added for the purpose of forming a polymer in the composition.

The liquid crystal composition is prepared by mixing a plurality of liquid crystal compounds. An additive is added to the composition for the purpose of further adjusting the physical properties. The additive such as the polymerizable compound, a polymerization initiator, a polymerization inhibitor, an optically active compound, an antioxidant, an ultraviolet light absorber, a light stabilizer, a heat stabilizer, a dye and an antifoaming agent is added thereto when necessary. The liquid crystal compound and the additive are mixed in such a procedure. A proportion (content) of the liquid crystal compound is expressed in terms of weight percent (% by weight) based on the weight of the liquid crystal composition containing no additive, even after the additive is added. A proportion (amount of addition) of the additive is expressed in terms of weight percent (% by weight) based on the weight of the liquid crystal composition containing no additive. Weight parts per million (ppm) may be occasionally used. A proportion of the polymerization initiator and the polymerization inhibitor is exceptionally expressed based on the weight of the polymerizable compound.

"Clearing point" is a transition temperature between the liquid crystal phase and an isotropic phase in the liquid crystal compound. "Minimum temperature of the liquid crystal phase" is a transition temperature between a solid and the liquid crystal phase (the smectic phase, the nematic phase or the like) in the liquid crystal compound. "Maximum temperature of the nematic phase" is a transition temperature between the nematic phase and the isotropic phase in a mixture of the liquid crystal compound and a base liquid crystal or in the liquid crystal composition, and may be occasionally abbreviated as "maximum temperature." "Minimum temperature of the nematic phase" may be occasionally abbreviated as "minimum temperature." An expression "increase the dielectric anisotropy" means that a value of dielectric anisotropy positively increases in a composition having positive dielectric anisotropy, and the value of dielectric anisotropy negatively increases in a composition having negative dielectric anisotropy. An expression "having a large voltage holding ratio" means that the device has a large voltage holding ratio at room temperature and also at a temperature close to the maximum temperature in an initial stage, and the device has the large voltage holding ratio at room temperature and also at a temperature close to the maximum temperature even after the device has been used for a long period of time. In the composition or the device, the characteristics may be occasionally examined before and after an aging test (including an acceleration deterioration test).

A compound represented by formula (1) may be occasionally abbreviated as compound (1). At least one compound selected from the group of compounds represented by formula (1) may be occasionally abbreviated as compound (1). "Compound (1)" means one compound, a mixture of two compounds or a mixture of three or more compounds represented by formula (1). A same rule applies also to any other compound represented by any other formula. In formulas (1) to (15), a symbol of A¹, B¹, C¹ or the like surrounded by a hexagonal shape correspond to a ring such as ring A¹, ring B¹ and ring C¹, respectively. The hexagonal shape represents a six-membered ring such as cyclohexane or benzene. The hexagonal shape may occasionally represents a fused ring such as naphthalene or a bridged ring such as adamantane.

A symbol of terminal group R¹¹ is used in a plurality of compounds in chemical formulas of component compounds. In the compounds, two groups represented by two pieces of arbitrary R¹¹ may be identical or different. For example, in one case, R¹¹ of compound (2) is ethyl and R¹¹ of compound (3) is ethyl. In another case, R¹¹ of compound (2) is ethyl and R¹¹ of compound (3) is propyl. A same rule applies also to a symbol of R¹², R¹³, Z¹¹ or the like. In compound (15), when i is 2, two of ring E¹ exists. In the compound, two groups represented by two of ring E¹ may be identical or different. A same rule applies also to two of arbitrary ring E¹ when i is larger than 2. A same rule applies also to other symbols.

An expression "at least one piece of 'A'" means that the number of 'A' is arbitrary. An expression "at least one piece of 'A' may be replaced by 'B'" means that, when the number of 'A' is 1, a position of 'A' is arbitrary, and also when the number of 'A' is 2 or more, positions thereof can be selected without restriction. A same rule applies also to an expression "at least one piece of 'A' is replaced by 'B'." An expression "at least one piece of 'A' may be replaced by 'B', 'C' or 'D'" includes a case where arbitrary 'A' is replaced by 'B', a case where arbitrary 'A' is replaced by 'C', and a case where arbitrary 'A' is replaced by 'D', and also a case where a plurality of pieces of 'A' are replaced by at least two pieces of 'B', 'C' and/or 'D'. For example, "alkyl in which at least one piece of -CH₂- may be replaced by -O- or -CH=CH-" includes alkyl, alkoxy, alkoxyalkyl, alkenyl, alkoxyalkenyl and alkenyloxyalkyl. In addition, a case where two pieces of consecutive -CH₂- are replaced by -O- to form -O-O- is not preferred. In alkyl or the like, a case where -CH₂- of a methyl part (-CH₂-H) is replaced by -O- to form -O-H is not preferred, either.

An expression "R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine" may be occasionally used. In the expression, "in the groups" may be interpreted according to wording. In the expression, "the groups" means alkyl, alkenyl, alkoxy, alkenyloxy or the like. More specifically, "the groups" represents all of the groups described before the term "in the groups." The common interpretation is applied also to terms of "in the monovalent groups" or "in the divalent groups. " For example, "the monovalent groups" represents all of the groups described before the term "in the monovalent groups."

Alkyl of the liquid crystal compound is straight-chain alkyl or branched-chain alkyl, but includes no cyclic alkyl. In general, straight-chain alkyl is preferred to branched-chain alkyl. A same rule applies also to a terminal group such as alkoxy and alkenyl. With regard to a configuration of 1, 4-cyclohexylene, trans is preferred to cis for increasing the maximum temperature. Then, 2-fluoro-1,4-phenylene means two divalent groups described below. In a chemical formula, fluorine may be leftward (L) or rightward (R). A same rule applies also to an asymmetrical divalent group formed by eliminating two hydrogens from a ring, such as tetrahydropyran-2,5-diyl.

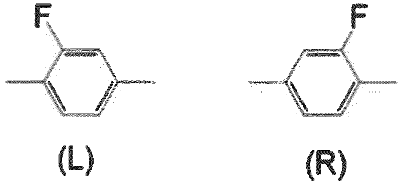

The invention includes items described below.

Item 1. A compound, represented by formula (1): 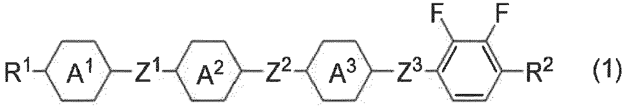 wherein, in formula (1),
R¹ is alkyl having 1 to 15 carbons, and in the alkyl, at least one piece of -CH₂- may be replaced by -O-, and one piece of -CH₂CH₂- may be replaced by -CH=CH-, and R² is alkyl having 1 to 15 carbons, alkoxy having 1 to 15 carbons or alkenyl having 2 to 15 carbons;
ring A¹ and ring A² are independently 1,4-cyclohexylene, 1,4-phenylene, tetrahydropyran-2,5-diyl, 1,3-dioxane-2,5-diyl, pyridine-2,5-diyl or pyrimidine-2,5-diyl, and ring A³ is 1,4-cyclohexylene, 1,4-phenylene, 2-fluoro-1,4-phenylene, tetrahydropyran-2,5-diyl, 1,3-dioxane-2,5-diyl, pyridine-2,5-diylor pyrimidine-2,5-diyl; and
Z¹, Z² and Z³ are independently a single bond, -COO-, -OCO-, -CH₂CH₂- or -CH=CH-, and at least one of Z¹, Z² and Z³ is -COO-, -OCO-, -CH₂CH₂- or -CH=CH-,
in which, when ring A³ is 2-fluoro-1,4-phenylene, at least one of ring A¹ and ring A² is 1,4-phenylene, tetrahydropyran-2,5-diyl, 1,3-dioxane-2,5-diyl, pyridine-2,5-diyl or pyrimidine-2,5-diyl, and Z³ is a single bond,
in which, when R¹ is CH₂=CH- or alkenyloxy, Z¹ and Z³ each are a single bond, and when Z² is -CH=CH-, at least one of ring A¹, ring A² and ring A³ is 1,4-phenylene, tetrahydropyran-2,5-diyl, 1,3-dioxane-2,5-diyl, pyridine-2,5-diyl or pyrimidine-2,5-diyl.

Item 2. The compound according to item 1, wherein, in formula (1), R¹ is alkenyl having 2 to 10 carbons, and R² is alkyl having 1 to 15 carbons, alkoxy having 1 to 15 carbons or alkenyl having 2 to 15 carbons; ring A¹ is 1, 4-cyclohexylene, and ring A² and ring A³ are independently 1,4-cyclohexylene, 1,4-phenylene, tetrahydropyran-2,5-diyl, 1,3-dioxane-2,5-diyl, pyridine-2,5-diyl or pyrimidine-2,5-diyl; and Z¹, Z² and Z³ are independently a single bond, -COO-, -OCO- or -CH₂CH₂-, and at least one of Z¹, Z² and Z³ is -COO-, -OCO- or -CH₂CH₂-.

Item 3. The compound according to item 1, represented by formula (1-1), formula (1-2) or formula (1-3): 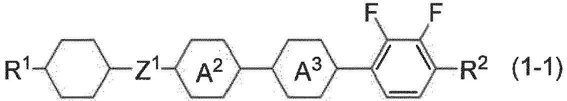 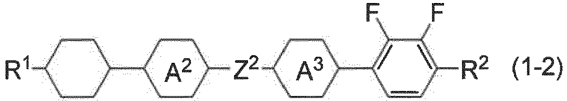 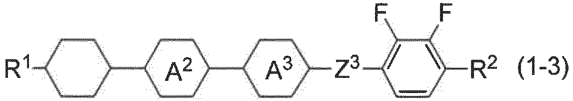 wherein, in formula (1-1) to formula (1-3),
R¹ is alkyl having 1 to 10 carbons, and in the alkyl, at least one piece of -CH₂- may be replaced by -O-, and one piece of -CH₂CH₂- may be replaced by -CH=CH-, and R² is alkyl having 1 to 10 carbons, alkoxy having 1 to 10 carbons or alkenyl having 2 to 10 carbons;
ring A² and ring A³ are independently 1,4-cyclohexylene, 1,4-phenylene, tetrahydropyran-2,5-diyl, 1,3-dioxane-2,5-diyl, pyridine-2,5-diyl or pyrimidine-2,5-diyl; and
Z¹, Z² and Z³ are independently -COO-, -OCO- or -CH₂CH₂-.

Item 4. The compound according to item 1, represented by any one of formula (1-4) to formula (1-7): 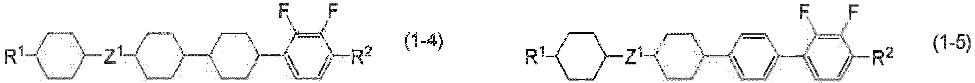 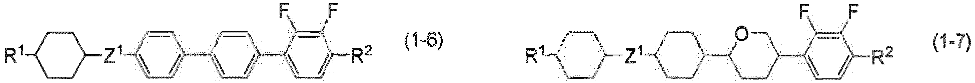 wherein, in formula (1-4) to formula (1-7), R¹ and R² are independently alkyl having 1 to 10 carbons, alkoxy having 1 to 10 carbons or alkenyl having 2 to 10 carbons; and Z¹ is -COO-, -OCO- or -CH₂CH₂-.

Item 5. The compound according to item 1, represented by any one of formula (1-8) to formula (1-10): 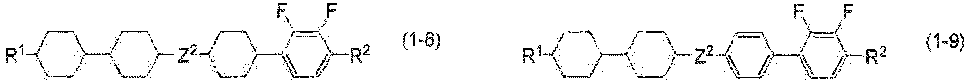 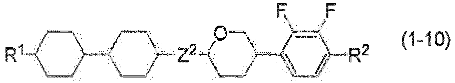 wherein, in formula (1-8) to formula (1-10), R¹ and R² are independently alkyl having 1 to 10 carbons, alkoxy having 1 to 10 carbons or alkenyl having 2 to 10 carbons; and Z² is -COO-, -OCO- or -CH₂CH₂-.

Item 6. The compound according to item 1, represented by any one of formula (1-11) or formula (1-12): 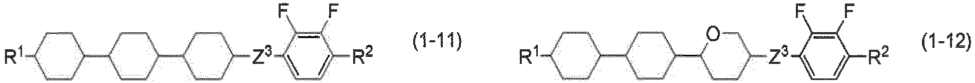 wherein, in formula (1-11) or formula (1-12), R¹ and R² are alkyl having 1 to 10 carbons, alkoxy having 1 to 10 carbons or alkenyl having 2 to 10 carbons; and Z³ is -COO-, -OCO- or -CH₂CH₂-.

Item 7. The compound according to item 1, represented by any one of formula (1-13) to formula (1-15), formula (1-19) to formula (1-22) and formula (1-24): 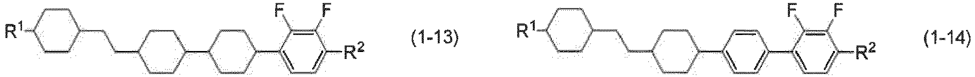 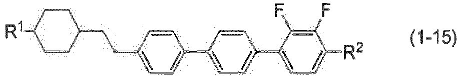 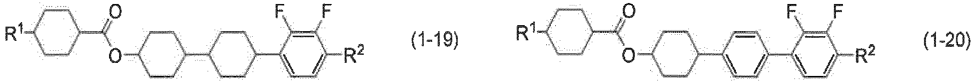 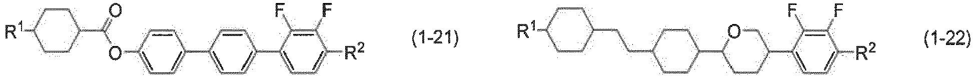 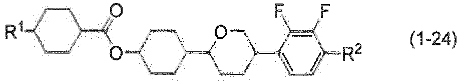 wherein, in formula (1-13) to formula (1-15), formula (1-19) to formula (1-22) and formula (1-24), R¹ is alkenyl having 2 to 5 carbons, and R² is alkyl having 1 to 5 carbons, alkoxy having 1 to 5 carbons or alkenyl having 2 to 5 carbons.

Item 8. The compound according to item 1, represented by any one of formula (1-25), formula (1-26) and formula (1-29) to formula (1-31) : 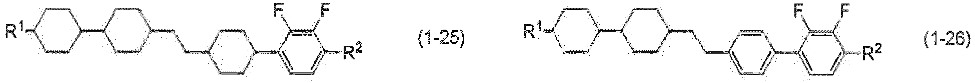 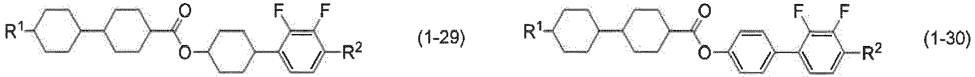 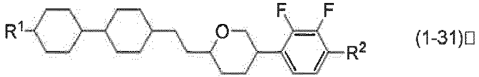 wherein, in formula (1-25), formula (1-26) and formula (1-29) to formula (1-31), R¹ is alkenyl having 2 to 5 carbons, and R² is alkyl having 1 to 5 carbons, alkoxy having 1 to 5 carbons or alkenyl having 2 to 5 carbons.

Item 9. The compound according to item 1, represented by any one of formula (1-32), formula (1-34) and formula (1-36): 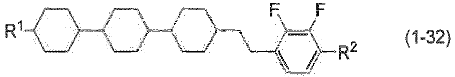 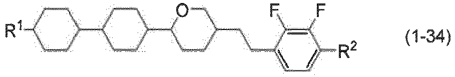 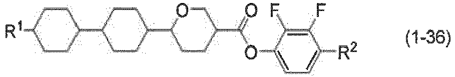 wherein, in formula (1-32), formula (1-34) and formula (1-36), R¹ is alkenyl having 2 to 5 carbons, and R² is alkyl having 1 to 5 carbons, alkoxy having 1 to 5 carbons or alkenyl having 2 to 5 carbons.

Item 10. A liquid crystal composition containing at least one compound according to any one of items 1 to 9.

Item 11. The liquid crystal composition according to item 10, further containing at least one compound selected from the group of compounds represented by formulas (2) to (4): 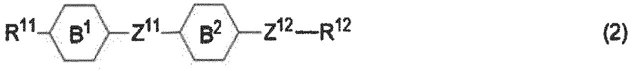 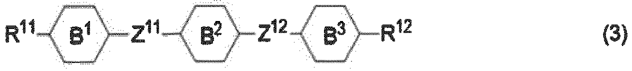 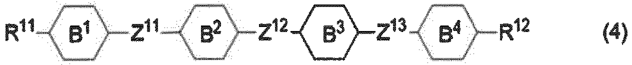 wherein, in formulas (2) to (4),
R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -COO-, -CH₂CH₂-, -CH=CH- or -C≡C-.

Item 12. The liquid crystal composition according to item 11, further containing at least one compound selected from the group of compounds represented by formulas (5) to (11): 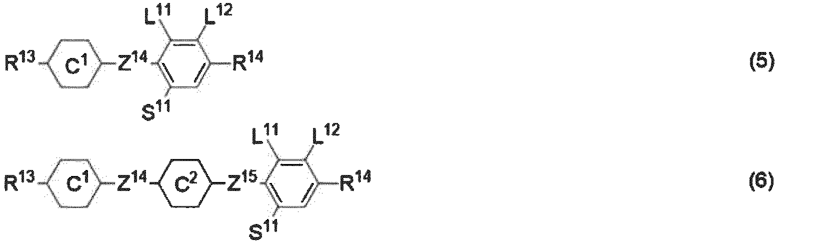 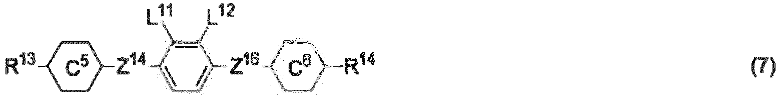 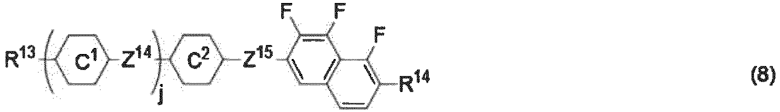 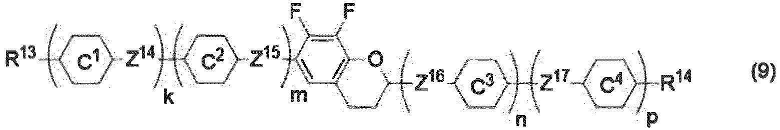 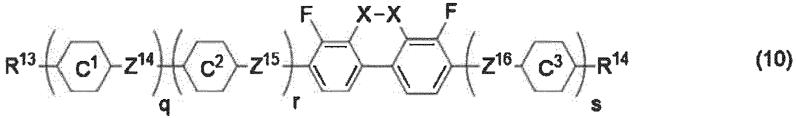 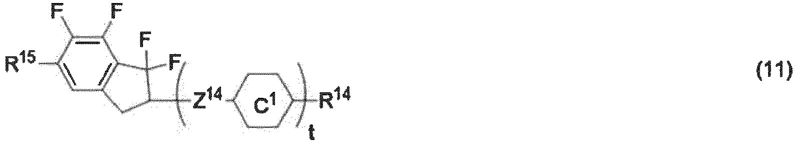 wherein, in formulas (5) to (11),
R¹³, R¹⁴ and R¹⁵ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁵ may be hydrogen or fluorine;
ring C¹, ring C², ring C³ and ring C⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
ring C⁵ and ring C⁶ are independently 1,4-cyclohexylene, 1,4-cyclohexenylene, 1,4-phenylene, tetrahydropyran-2,5-diyl or decahydronaphthalene-2,6-diyl;
Z¹⁴, Z¹⁵, Z¹⁶ and Z¹⁷ are independently a single bond, -COO-, -CH₂O-, -OCF₂-, -CH₂CH₂- or -OCF₂CH₂CH₂-;
L¹¹ and L¹² are independently fluorine or chlorine;
S¹¹ is hydrogen or methyl;
X is -CHF- or -CF₂-; and
j, k, m, n, p, q, r and s are independently 0 or 1, a sum of k, m, n and p is 1 or 2, a sum of q, r and s is 0, 1, 2 or 3, and t is 1, 2 or 3.

Item 13. The liquid crystal composition according to item 11 or 12, further containing at least one compound selected from the group of compounds represented by formulas (12) to (14): 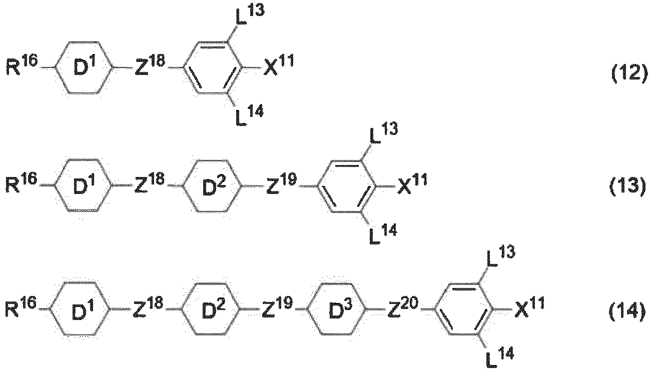 wherein, in formulas (12) to (14),
R¹⁶ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹¹ is fluorine, chlorine, -CF₃, -CHF₂, -CH₂F, -OCF₃, -OCHF₂, -OCF₂CHF₂ or -OCF₂CHFCF₃;
ring D¹, ring D² and ring D³ are independently 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁸, Z¹⁹ and Z²⁰ are independently a single bond, -COO-, -CH₂O-, -CF₂O-, -OCF₂-, -CH₂CH₂-, -CH=CH-, -C≡C- or -(CH₂)₄-; and
L¹³ and L¹⁴ are independently hydrogen or fluorine.

Item 14. The liquid crystal composition according to any one of items 11 to 13, further containing at least one compound selected from the group of compounds represented by formula (15): 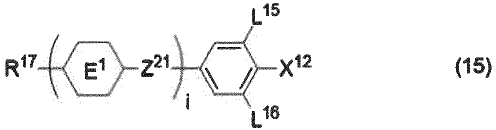 wherein, in formula (15),
R¹⁷ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹² is -C≡N or -C≡C-C≡N;
ring E¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z²¹ is a single bond, -COO-, -CH₂O-, -CF₂O-, -OCF₂-, -CH₂CH₂- or -C≡C-;
L¹⁵ and L¹⁶ are independently hydrogen or fluorine; and
i is 1, 2, 3 or 4.

Item 15. A liquid crystal display device, including the liquid crystal composition according to any one of items 10 to 14.

The invention further includes the following items: (a) the composition, further containing at least one optically active compound and/or at least one polymerizable compound; and (b) the composition, further containing at least one antioxidant and/or at least one ultraviolet light absorber.

The invention further includes the following items: (c) the composition, further containing one, two or at least three additives selected from the group of a polymerizable compound, a polymerization initiator, a polymerization inhibitor, an optically active compound, an antioxidant, an ultraviolet light absorber, a light stabilizer, a heat stabilizer, a dye and an antifoaming agent; and (d) the composition, wherein a maximum temperature of a nematic phase is 70°C or higher, an optical anisotropy (measured at 25°C) at a wavelength of 589 nanometers is 0.08 or more and a dielectric anisotropy (measured at 25°C) at a frequency of 1 kHz is -2 or less.

The invention still further includes the following items: (e) a device including the composition and having a PC mode, a TN mode, an STN mode, an ECB mode, an OCB mode, an IPS mode, a VA mode, an FFS mode, an FPA mode or a PSA mode; (f) an AM device including the composition; (g) a transmissive device including the composition; (h) use of the composition as the composition having the nematic phase; and (i) use as an optically active composition by adding the optically active compound to the composition.

An aspect of compound (1), synthesis of compound (1), the liquid crystal composition and the liquid crystal display device will be described in the order.

### 1. Aspect of compound (1)

Compound (1) has a feature of having a single bond, in which at least one of Z¹, Z² and Z³ is a diatomic bonding group such as -COO-, -OCO-, -CH₂CH₂- or -CH=CH-. One of Z¹, Z² and Z³ may be -COO-, -OCO-, -CH₂CH₂- or -CH=CH-, and a remainder thereof may be a single bond. Compound (1) has features such that the compatibility is good, the dielectric anisotropy is large and the viscosity is small in comparison with a similar compound (see Comparative Examples 1 and 2).

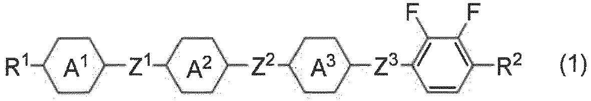

The compound is physically and chemically significantly stable under conditions in which the device is ordinarily used, and has good compatibility with other liquid crystal compounds. A composition containing the compound is stable under conditions in which the device is ordinarily used. When the composition is stored at low temperature, the compound has small tendency of precipitation as a crystal (or a smectic phase) . The compound has general physical properties required for a component of the composition, suitable optical anisotropy and suitable dielectric anisotropy.

Preferred examples of terminal group R, ring A and bonding group Z in compound (1) are as described below. The examples described above are applied also to the subordinate formula of compound (1). In compound (1), physical properties can be arbitrarily adjusted by suitably combining the groups. Compound (1) may contain a larger amount of isotope such as ²H (deuterium) and ¹³C than the amount of natural abundance because no significant difference exists in the physical properties of the compound. In addition, definitions of symbols of compound (1) are as described in item 1.

In formula (1), R¹ is alkyl having 1 to 15 carbons, and in the alkyl, at least one piece of -CH₂- may be replaced by -O-, and one piece of -CH₂CH₂- maybe replaced by -CH=CH-, and R² is alkyl having 1 to 15 carbons, alkoxy having 1 to 15 carbons or alkenyl having 2 to 15 carbons.

An example of R¹ is alkyl, alkoxy, alkoxyalkyl, alkoxyalkoxy, alkenyl, alkenyloxy, alkenyloxyalkyl or alkoxyalkenyl. In the groups, a straight chain is preferred to a branched chain. However, if R¹ has the branched chain, the group is preferred when the group has optical activity. Preferred R¹ is alkyl, alkoxy, alkoxyalkyl, alkenyl or alkenyloxy. Further preferred R¹ is fluorine, alkyl, alkoxy or alkenyl. Particularly preferred R¹ is alkenyl.

A preferred configuration of -CH=CH- in the alkenyl depends on a position of a double bond. A trans configuration is preferred in the alkenyl such as 1-propenyl, 1-butenyl, 1-pentenyl, 1-hexenyl, 3-pentenyl and 3-hexenyl. A cis configuration is preferred in the alkenyl such as 2-butenyl, 2-pentenyl and 2-hexenyl.

Specific R¹ is methyl, ethyl, propyl, butyl, pentyl, hexyl, heptyl, octyl, methoxy, ethoxy, propoxy, butoxy, pentyloxy, hexyloxy, heptyloxy, methoxymethyl, methoxyethyl, methoxypropyl, ethoxymethyl, ethoxyethyl, ethoxypropyl, propoxymethyl, butoxymethyl, pentoxymethyl, vinyl, 1-propenyl, 2-propenyl, 1-butenyl, 2-butenyl, 3-butenyl, 2-pentenyl, 3-pentenyl, 4-pentenyl, 2-propenyloxy, 2-butenyloxy, 2-pentenyloxy, 1-propynyl or 1-pentenyl.

Preferred R¹ is methyl, ethyl, propyl, butyl, pentyl, methoxy, ethoxy, propoxy, butoxy, pentyloxy, methoxymethyl, ethoxymethyl, propoxymethyl, vinyl, 1-propenyl, 2-propenyl, 1-butenyl, 2-butenyl, 3-butenyl, 1-pentenyl, 2-pentenyl, 3-pentenyl, 4-pentenyl, 2-propenyloxy, 2-butenyloxy or 2-pentenyloxy. Further preferred R¹ is vinyl, 1-propenyl, 2-propenyl, 1-butenyl, 2-butenyl, 3-butenyl, 1-pentenyl, 2-pentenyl, 3-pentenyl or 4-pentenyl. Particularly preferred R¹ is vinyl, 1-propenyl, 2-propenyl, 1-butenyl or 3- butenyl.

Preferred R² is alkyl or alkoxy. Further preferred R² is alkoxy. Particularly preferred R² is methyl or ethoxy. Most preferred R² is ethoxy.

In formula (1), ring A¹ and ring A² are independently 1,4-cyclohexylene, 1,4-phenylene, tetrahydropyran-2,5-diyl, 1,3-dioxane-2,5-diyl, pyridine-2,5-diyl or pyrimidine-2,5-diyl, and ring A³ is 1,4-cyclohexylene, 1,4-phenylene, 2-fluoro-1,4-phenylene, tetrahydropyran-2,5-diyl, 1,3-dioxane-2,5-diyl, pyridine-2,5-diyl or pyrimidine-2,5-diyl.

Preferred ring A¹ or ring A² is 1,4-cyclohexylene, 1,4-phenylene or tetrahydropyran-2,5-diyl. Further preferred ring A¹ is 1,4-cyclohexylene. Further preferred ring A² is 1,4-cyclohexylene or 1,4-phenylene. Preferred ring A³ is 1,4-cyclohexylene, 1,4-phenylene, 2-fluoro-1,4-phenylene or tetrahydropyran-2,5-diyl. Further preferred ring A³ is 1,4-cyclohexylene, 1,4-phenylene or tetrahydropyran-2,5-diyl. Particularly preferred ring A³ is 1,4-cyclohexylene or 1,4-phenylene.

In formula (1), Z¹, Z² and Z³ are independently a single bond, -COO-, -OCO-, -CH₂CH₂- or -CH=CH-. At least one of Z¹, Z² and Z³ is preferably -COO-, -OCO-, -CH₂CH₂- or -CH=CH-. Two of Z¹, Z² and Z³ are further preferably a single bond, and a remainder is -COO-, -OCO-, -CH₂CH₂- or -CH=CH-.

Preferred Z¹, Z² or Z³ is a single bond, -COO-, -OCO- or -CH₂CH₂-. Further preferred Z¹, Z² or Z³ is a single bond, -COO- or -CH₂CH₂-. Particularly preferred Z¹, Z² or Z³ is a single bond, but at least one of Z¹, Z² and Z³ is not a single bond. In a most preferred aspect, two of Z¹, Z² and Z³ is a single bond.

Physical properties such as optical anisotropy and dielectric anisotropy can be arbitrarily adjusted by suitably selecting a terminal group, a ring and a bonding group in compound (1). An effect of kinds of terminal groups R, ring A, and bonding group Z on physical properties of compound (1) will be described below.

In compound (1), when R¹ or R² has the straight chain, a temperature range of the liquid crystal phase is wide, and the viscosity is small. When R¹ or R² has the branched chain, the compatibility with other liquid crystal compounds is good. A compound in which R¹ or R² is an optically active group is useful as a chiral dopant. A reverse twisted domain to be generated in the device can be prevented by adding the compound to the composition. A compound in which R¹ or R² is not the optically active group is useful as a component of the composition. When R¹ or R² is alkenyl, a preferred configuration depends on a position of a double bond. An alkenyl compound having the preferred configuration has high maximum temperature or a wide temperature range of the liquid crystal phase. A detailed description is found in Mol. Cryst. Liq. Cryst., 1985, 131, 109 and Mol. Cryst. Liq. Cryst., 1985, 131, 327.

When ring A¹, ring A² or ring A³ is 1,4-phenylene in which at least one hydrogen may be replaced by fluorine, pyridine-2,5-diyl or pyrimidine-2,5-diyl, the optical anisotropy is large. When ring A¹, ring A² or ring A³ is 1,4-cyclohexylene or 1,3-dioxane-2,5-diyl, the optical anisotropy is small.

When at least two rings are 1,4-cyclohexylene, the maximum temperature is high, the optical anisotropy is small, and the viscosity is small. When at least one ring is 1,4-phenylene, the optical anisotropy is comparatively large and an orientational order parameter is large. When at least two rings are 1,4-phenylene, the optical anisotropy is large, the temperature range of the liquid crystal phase is wide, and the maximum temperature is high.

When bonding group Z¹, Z² or Z³ is a single bond, -CH₂O-, -CH₂CH₂- or -CH=CH-, the viscosity is small. When the bonding group is a single bond, -CH₂CH₂- or -CH=CH-, the viscosity is further smaller. When the bonding group is -CH=CH-, the temperature range of the liquid crystal phase is wide, and an elastic constant ratio K₃₃ / K₁₁ (K₃₃: a bend elastic constant, K₁₁: a splay elastic constant) is large. When the bonding group is -C≡C-, the optical anisotropy is large.

### 2. Synthesis of compound (1)

A synthetic method of compound (1) will be described. Compound (1) can be prepared by suitably combining methods in synthetic organic chemistry. A method for introducing a required terminal group, ring and bonding group into a starting material is described in books such as "Organic Syntheses" (John Wiley & Sons, Inc.), "Organic Reactions" (John Wiley & Sons, Inc.), "Comprehensive Organic Synthesis" (Pergamon Press) and "New Experimental Chemistry Course (Shin Jikken Kagaku Koza in Japanese)" (Maruzen Co., Ltd.).

### 2-1. Formation of bonding group Z

First, a scheme is shown with regard to a method for forming bonding groups Z¹ to Z³. Next, reactions described in the scheme in methods (1) to (11) will be described. In the scheme, MSG¹ (or MSG²) is a monovalent organic group having at least one ring. The monovalent organic groups represented by a plurality of MSG¹ (or MSG²) used in the scheme may be identical or different. Compounds (1A) to (1J) correspond to compound (1).

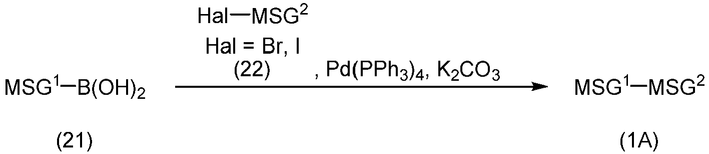

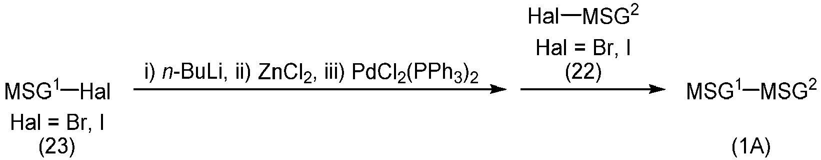

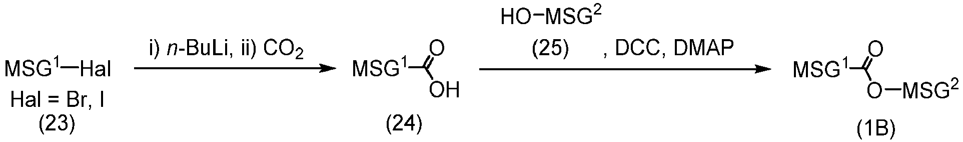

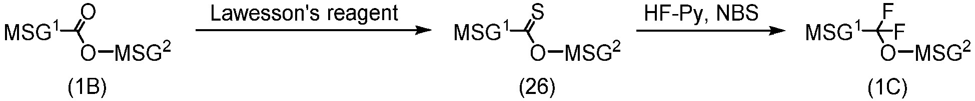

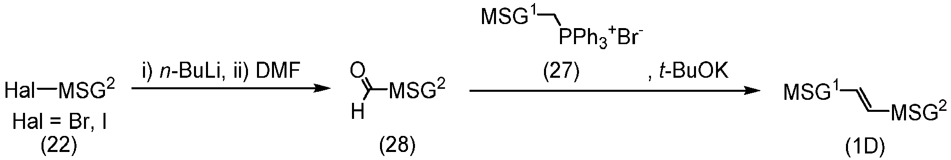

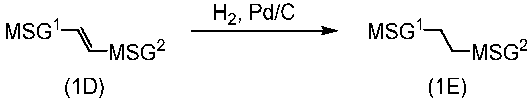

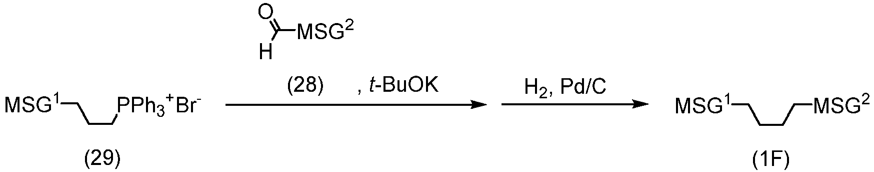

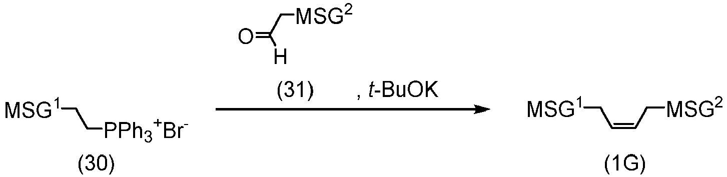

### (1) Formation of a single bond

Compound (1A) is prepared by allowing aryl boronic acid (21) prepared according to a publicly known method to react with halide (22), in the presence of carbonate and a catalyst such as tetrakis(triphenylphosphine)palladium. Compound (1A) is also prepared by allowing halide (23) prepared according to a publicly known method to react with n-butyllithium and subsequently with zinc chloride, and further with halide (22) in the presence of a catalyst such as dichlorobis(triphenylphosphine)palladium.

### (2) Formation of -COO-

Carboxylic acid (24) is obtained by allowing halide (23) to react with n-butyllithium and subsequently with carbon dioxide. Compound (1B) is prepared by dehydration of compound (25) prepared according to a publicly known method and carboxylic acid (24) in the presence of 1,3-dicyclohexylcarbodiimide (DDC) and 4-dimethylaminopyridine (DMAP).

### (3) Formation of -CF₂O-

Thionoester (26) is obtained by treating compound (1B) with a thiation reagent such as Lawesson's reagent. Compound (1C) is prepared by fluorinating thionoester (26) with a hydrogen fluoride-pyridine complex and N-bromosuccinimide (NBS). Refer to M. Kuroboshi et al., Chem. Lett., 1992, 827. Compound (1C) is also prepared by fluorinating thionoester (26) with (diethylamino) sulfur trifluoride (DAST). Refer to W. H. Bunnelle et al., J. Org. Chem. 1990, 55, 768. The bonding group can also be formed according to the method described in Peer. Kirsch et al., Angew. Chem. Int. Ed. 2001, 40, 1480.

### (4) Formation of -CH=CH-

Aldehyde (28) is obtained by treating halide (22) with n-butyllithium and then allowing the treated halide to react with N,N-dimethylformamide (DMF). Phosphorus ylide is generated by treating phosphonium salt (27) prepared according to a publicly known method with a base such as potassium t-butoxide. Compound (1D) is prepared by allowing the phosphorus ylide to react with aldehyde (28). A cis isomer may be formed depending on reaction conditions, and therefore the cis isomer is isomerized into a trans isomer according to a publicly known method when necessary.

### (5) Formation of -CH₂CH₂-

Compound (1E) is prepared by hydrogenating compound (1D) in the presence of a catalyst such as palladium on carbon.

### (6) Formation of -(CH₂)₄-

A compound having - (CH₂)₂-CH=CH- is obtained by using phosphonium salt (29) in place of phosphonium salt (27) according to the method in method (4). Compound (1F) is prepared by performing catalytic hydrogenation of the compound obtained.

### (7) Formation of -CH₂CH=CHCH₂-

Compound (1G) is prepared by using phosphonium salt (30) in place of phosphonium salt (27) and aldehyde (31) in place of aldehyde (28) according to the method of method (4). A trans isomer may be formed depending on reaction conditions, and therefore the trans isomer is isomerized into a cis isomer according to a publicly known method, when necessary.

### (8) Formation of -C≡C-

Compound (32) is obtained by allowing halide (23) to react with 2-methyl-3-butyn-2-ol in the presence of a catalyst of dichloropalladium and copper halide, and then performing deprotection under basic conditions. Compound (1H) is prepared by allowing compound (32) to react with halide (22) in the presence of the catalyst of dichloropalladium and copper halide.

### (9) Formation of -CF=CF-

Compound (33) is obtained by treating halide (23) with n-butyllithium and then allowing the treated halide to react with tetrafluoroethylene. Compound (1I) is prepared by treating halide (22) with n-butyllithium, and then allowing the treated halide to react with compound (33).

### (10) Formation of -OCH₂-

Compound (34) is obtained by reducing aldehyde (28) with a reducing agent such as sodium borohydride. Bromide (35) is obtained by brominating compound (34) with hydrobromic acid or the like. Compound (1J) is prepared by allowing bromide (35) to react with compound (36) in the presence of a base such as potassium carbonate.

### (11) Formation of -(CF₂)₂-

A compound having -(CF₂)₂- is obtained by fluorinating diketone (-COCO-) with sulfur tetrafluoride, in the presence of a hydrogen fluoride catalyst, according to a method described in J. Am. Chem. Soc., 2001, 123, 5414.

### 3. Liquid crystal composition

### 3-1. Component compound

A liquid crystal composition of the invention will be described. The composition contains at least one compound (1) as component (a). The composition may contain two, three or more compounds (1). A component in the composition may be only compound (1). The composition preferably contains at least one of compounds (1) in a range of 1% by weight to 99% by weight in order to develop good physical properties. In a composition having negative dielectric anisotropy, a preferred content of compound (1) is in a range of 5% by weight to 60% by weight. In a composition having positive dielectric anisotropy, a preferred content of compound (1) is 30% by weight or less.

**Table 1. Component compounds in composition**

| Component | Component compound | Dielectric anisotropy |
|---|---|---|
| Component (a) | Compound (1) | Negatively large |
| Component (b) | Compound (2) to Compound (4) | Small |
| Component (c) | Compound (5) to Compound (11) | Negatively large |
| Component (d) | Compound (12) to Compound (14) | Positively large |
| Component (e) | Compound (15) | Positively large |

The composition contains compound (1) as component (a). The composition further preferably contains a liquid crystal compound selected from components (b) to (e) described in Table 1. When the composition is prepared, components (b) to (e) are preferably selected by taking into account the positive or negative dielectric anisotropy and magnitude of the dielectric anisotropy. The composition may contain a liquid crystal compound different from compounds (1) to (15) . The composition may not contain such a liquid crystal compound.

Component (b) includes a compound in which two terminal groups are alkyl or the like. Specific examples of preferred component (b) include compounds (2-1) to (2-11), compounds (3-1) to (3-19) and compounds (4-1) to (4-7) . In the compounds, R¹¹ and R¹² are independently, alkyl having 1 to 10 carbons or alkenyl having 2 to 10 carbons, and in the alkyl and the alkenyl, at least one piece of -CH₂-may be replaced by -O-, and in the groups, at least one hydrogen may be replaced by fluorine.

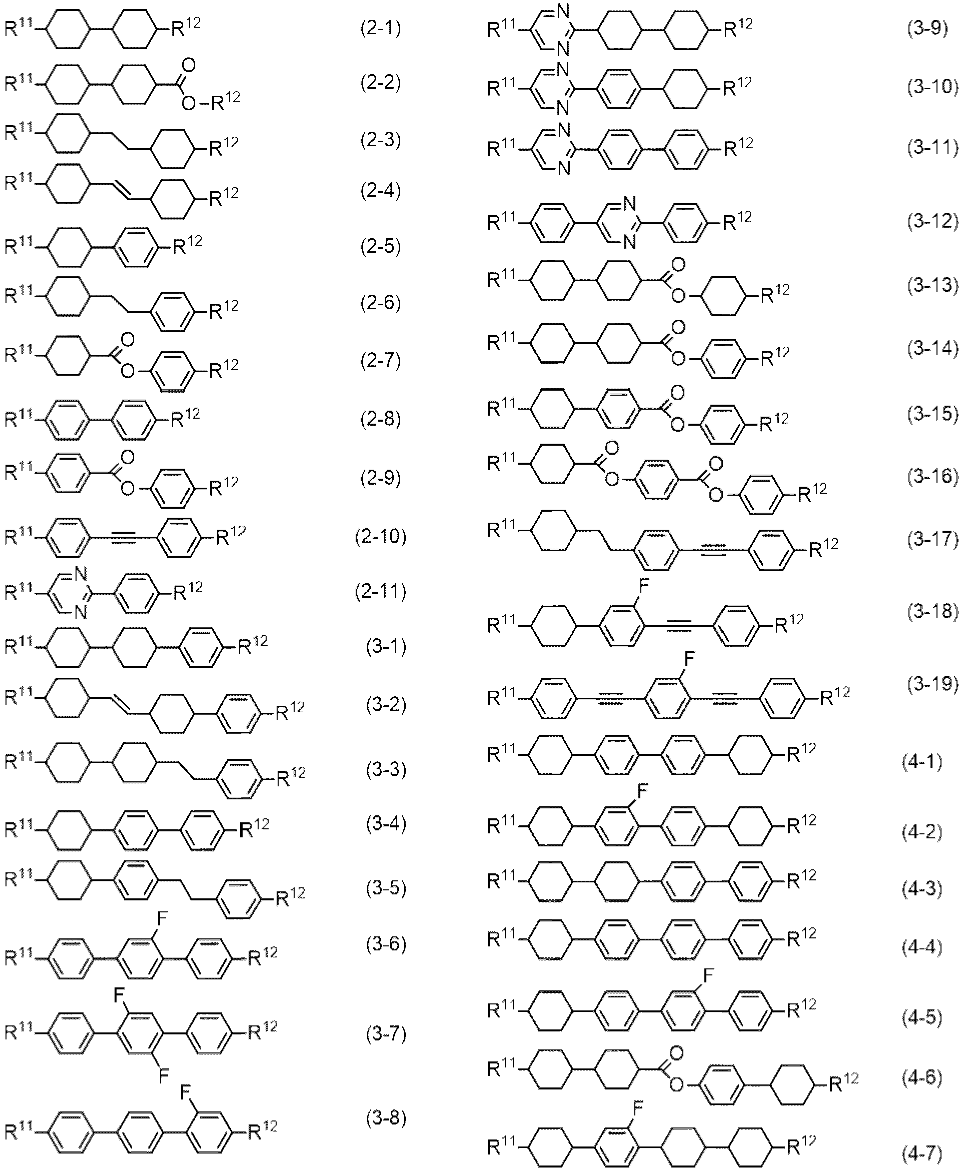

Component (b) has small dielectric anisotropy. Component (b) is close to neutrality. Compound (2) is effective in decreasing the viscosity or adjusting the optical anisotropy. Compounds (3) and (4) are effective in extending the temperature range of the nematic phase by increasing the maximum temperature, or in adjusting the optical anisotropy.

As a content of component (b) is increased, the viscosity of the composition is decreased, but the dielectric anisotropy is decreased. Thus, as long as a desired value of threshold voltage of a device is met, the content is preferably as large as possible. When a composition for the IPS mode, the VA mode or the like is prepared, the content of component (b) is preferably 30% by weight or more, and further preferably 40% by weight or more, based on the weight of the liquid crystal composition.

Component (c) includes compounds (5) to (11). The compounds have phenylene in which hydrogen in lateral positions are replaced by two halogens, such as 2,3-difluoro-1,4-phenylene. Preferred examples of component (c) include compounds (5-1) to (5-8), compounds (6-1) to (6-17), compound (7-1), compounds (8-1) to (8-3), compounds (9-1) to (9-11), compounds (10-1) to (10-3) and compounds (11-1) to (11-3). In the compounds, R¹³, R¹⁴ and R¹⁵ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁵ may be hydrogen or fluorine.

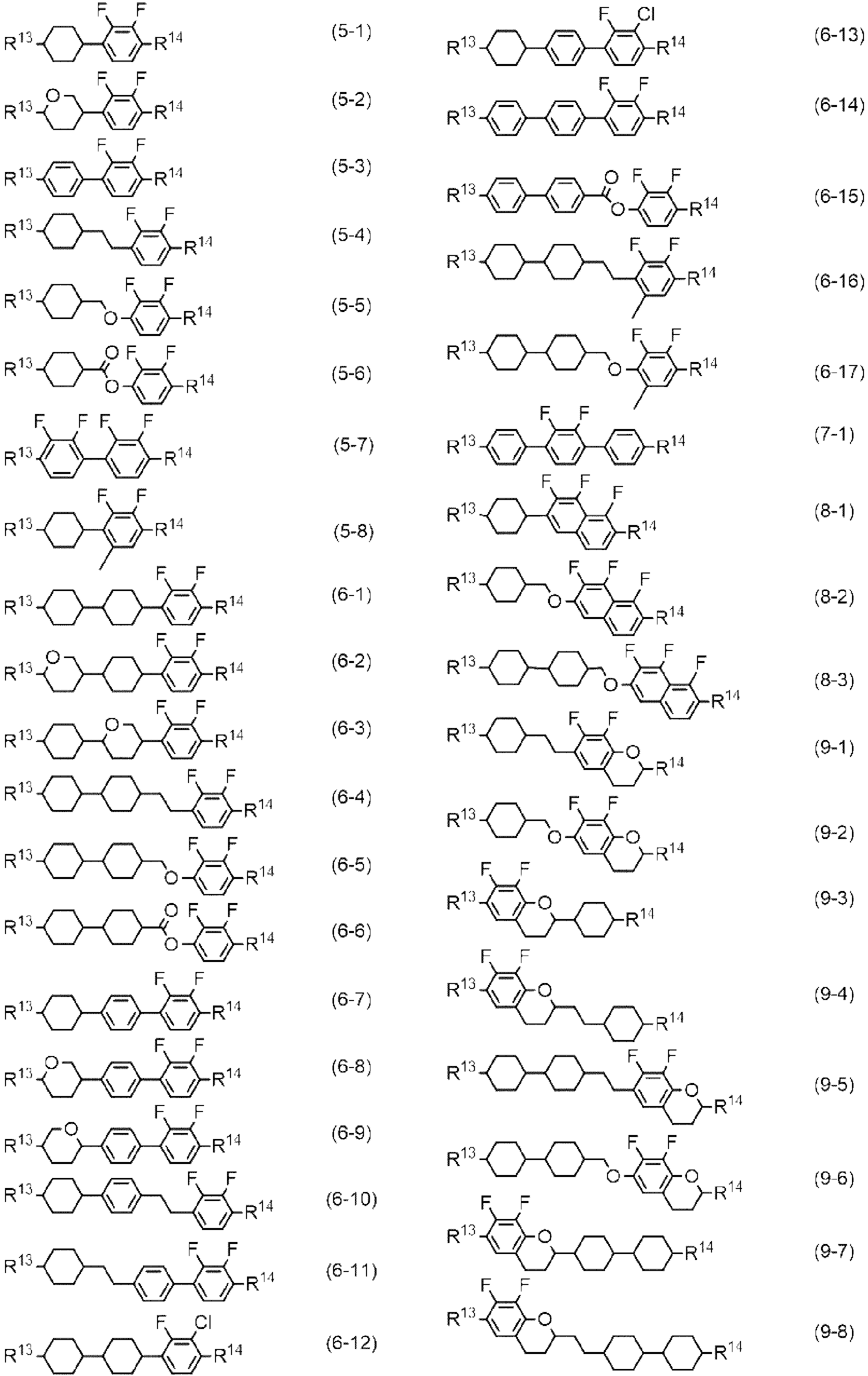

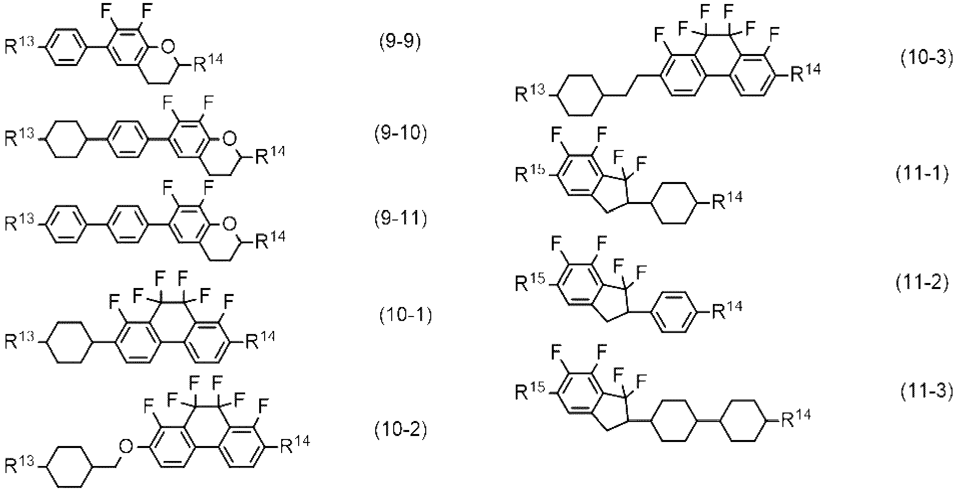

Component (c) has negatively large dielectric anisotropy. Component (c) is used when a composition for the IPS mode, the VA mode, the PSA mode or the like is prepared. As a content of component (c) is increased, the dielectric anisotropy of the composition is negatively increased, but the viscosity is increased. Thus, as long as a desired value of threshold voltage of the device is met, the content is preferably as small as possible. When the dielectric anisotropy at a degree of -5 is taken into account, the content is preferably 40% by weight or more in order to allow a sufficient voltage driving.

Among types of component (c), compound (5) is a bicyclic compound, and therefore is effective in decreasing the viscosity, adjusting the optical anisotropy or increasing the dielectric anisotropy. Compounds (5) and (6) are a tricyclic compound, and therefore are effective in increasing the maximum temperature, the optical anisotropy or the dielectric anisotropy. Compounds (8) to (11) are effective in increasing the dielectric anisotropy.

When a composition for the IPS mode, the VA mode, the PSA mode or the like is prepared, the content of component (c) is preferably 40% by weight or more, and further preferably in the range of 50% by weight to 95% by weight, based on the weight of the liquid crystal composition. When component (c) is added to the composition having positive dielectric anisotropy, the content of component (c) is preferably 30% by weight or less. Addition of component (c) allows adjustment of the elastic constant of the composition and adjustment of a voltage-transmittance curve of the device.

Component (d) is a compound having a halogen-containing group or a fluorine-containing group at a right terminal. Preferred examples of component (d) include compounds (12-1) to (12-16), compounds (13-1) to (13-113) and compounds (14-1) to (14-57). In the compounds, R¹⁶ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine. X¹¹ is fluorine, chlorine, -OCF₃, -OCHF₂, -CF₃, -CHF₂, -CH₂F, -OCF₂CHF₂ or -OCF₂CHFCF₃.

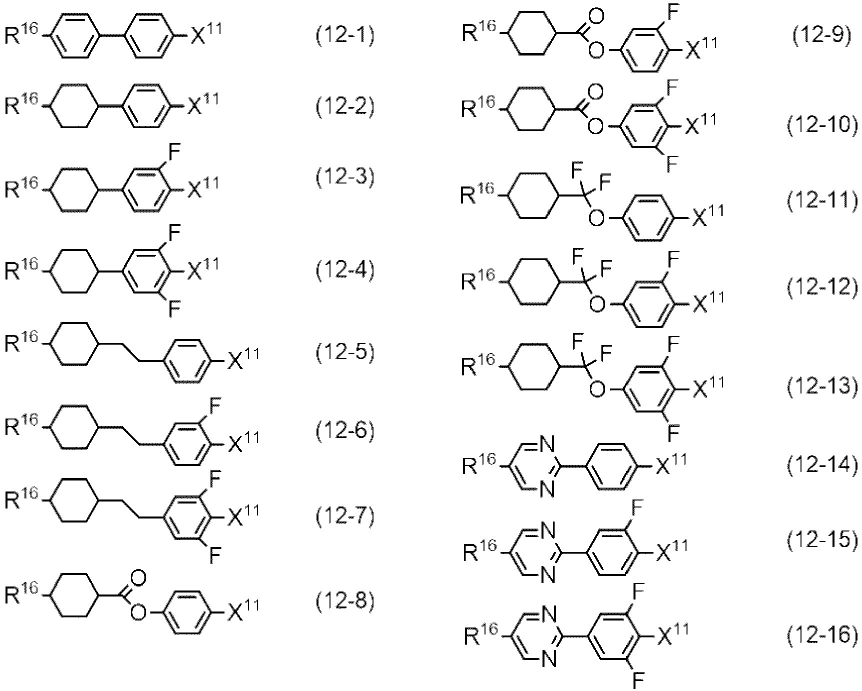

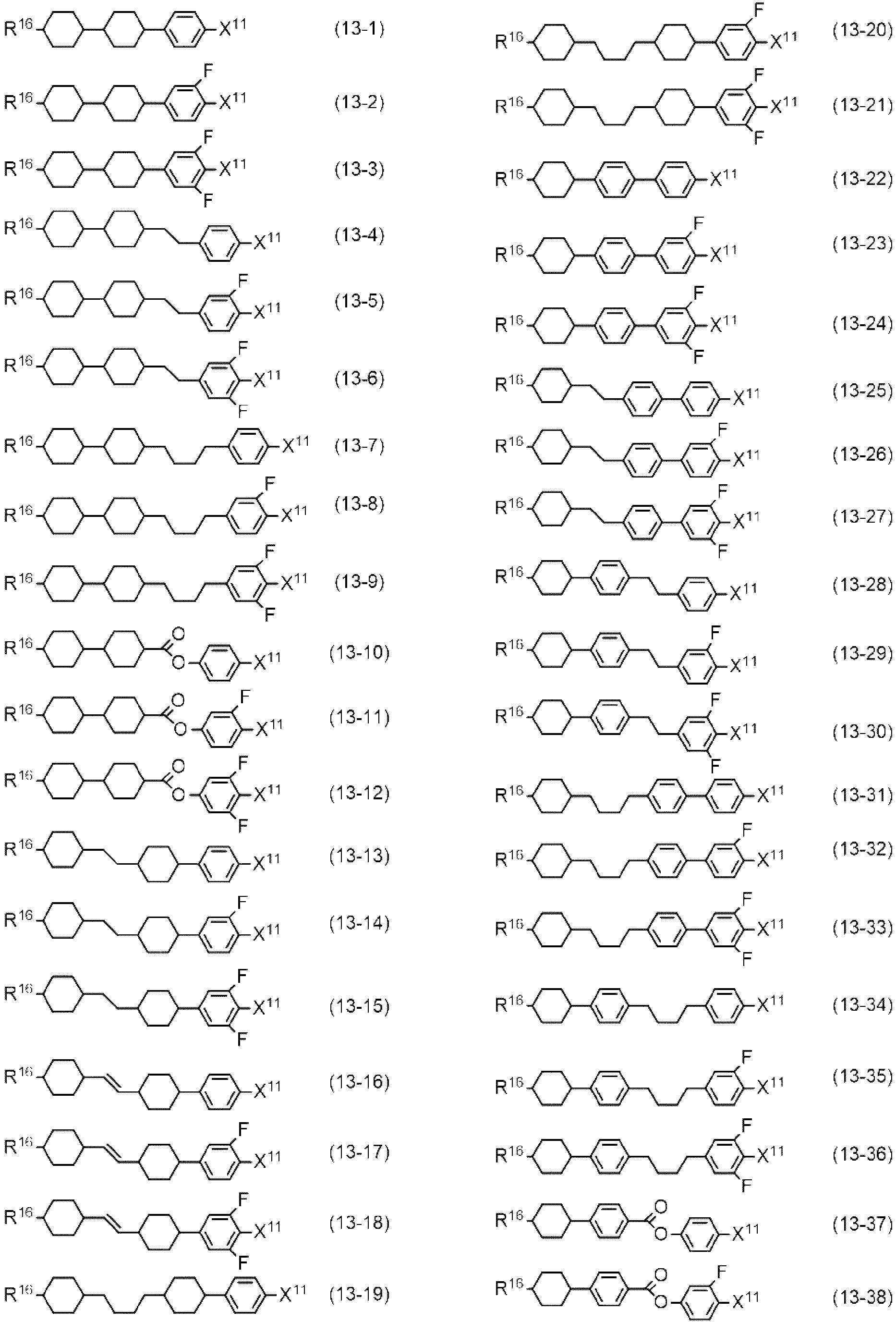

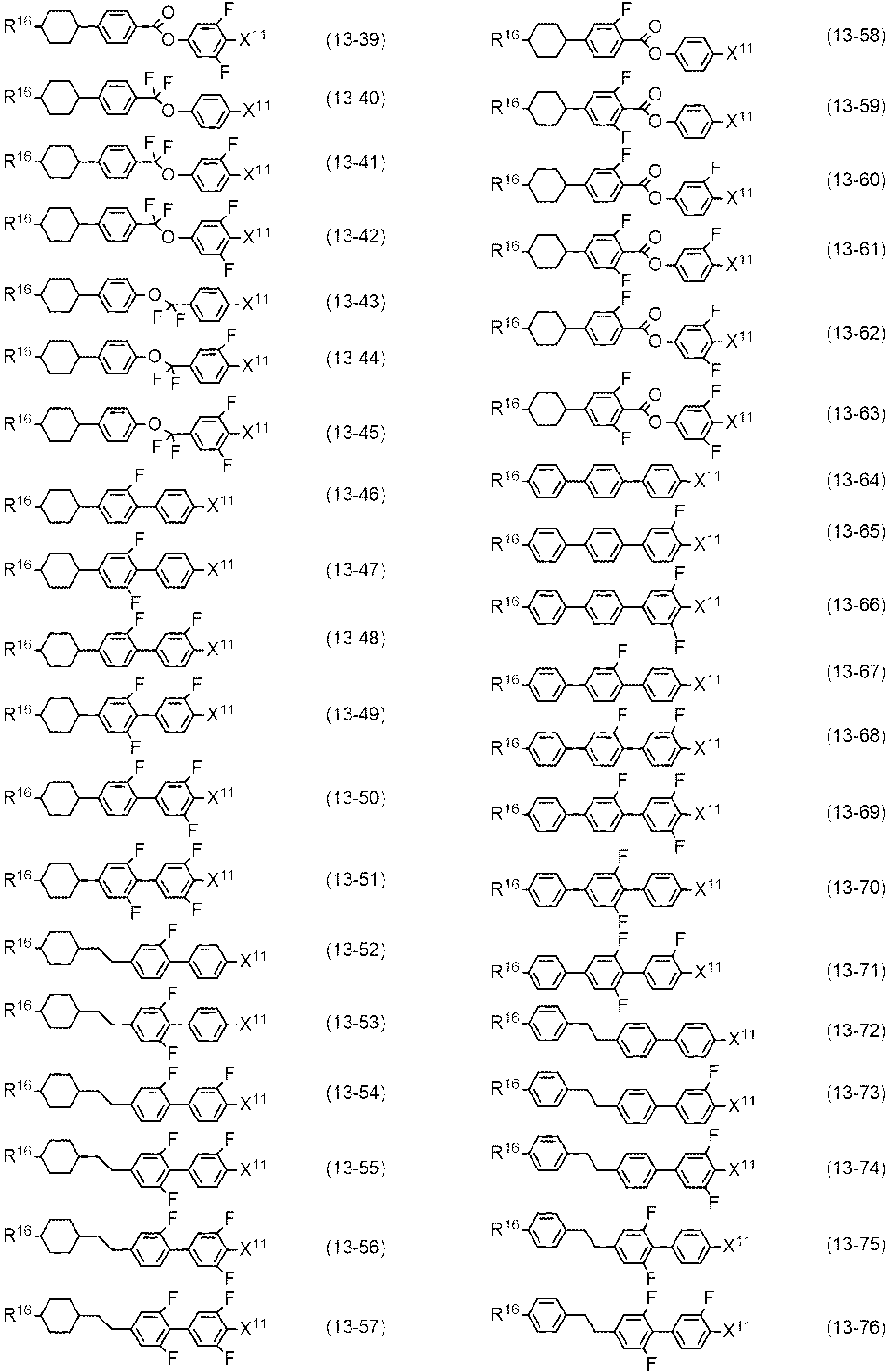

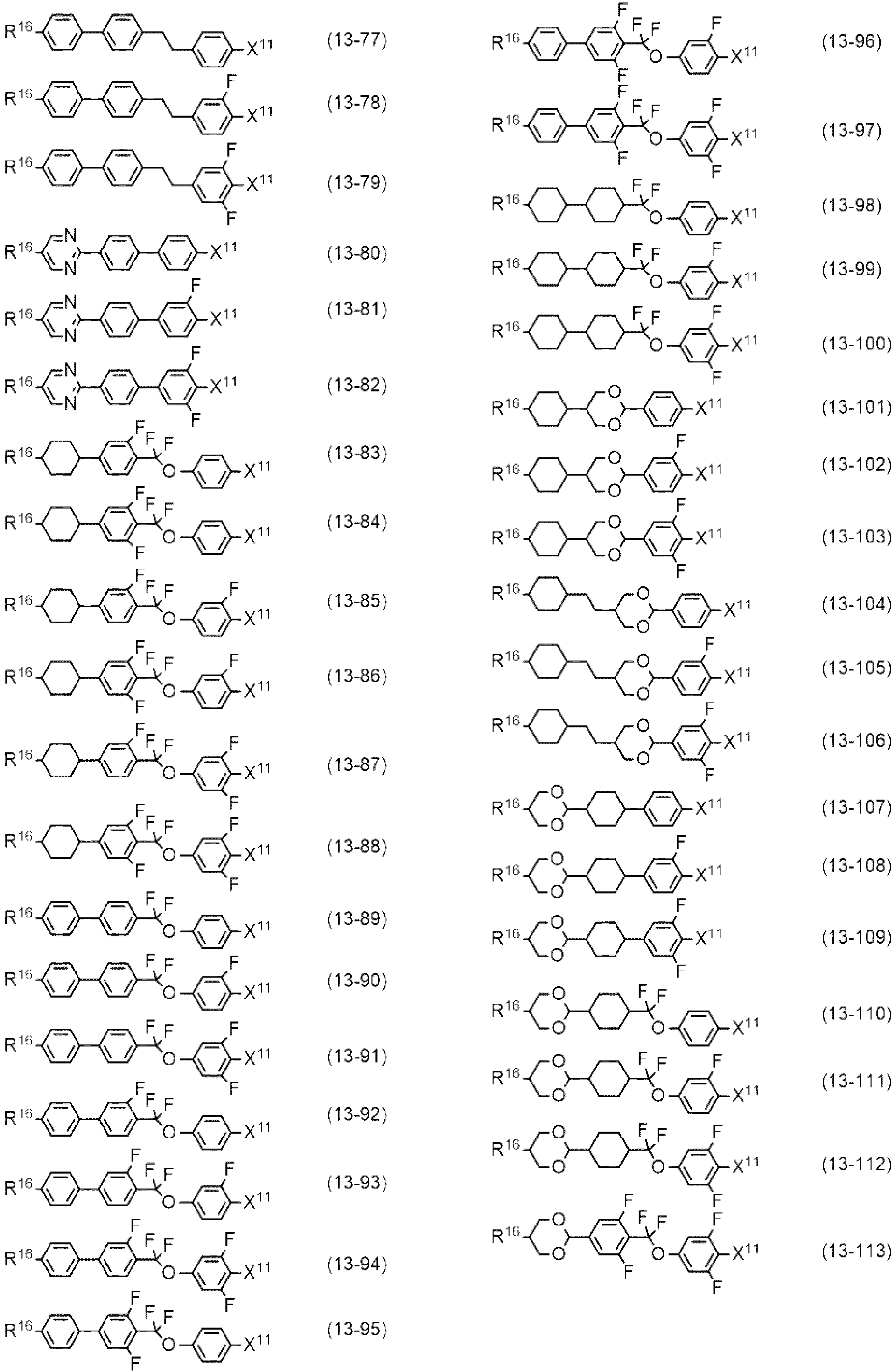

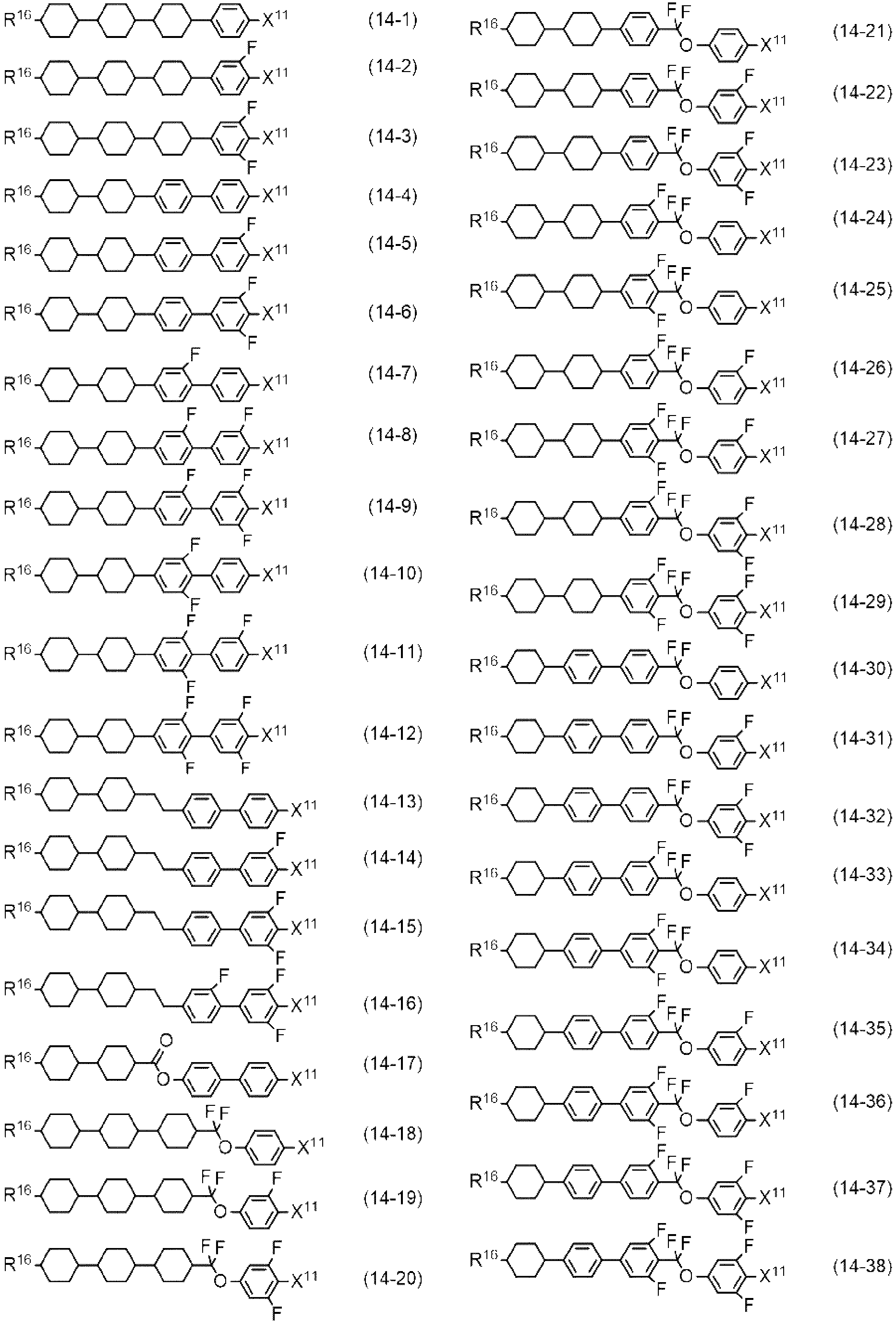

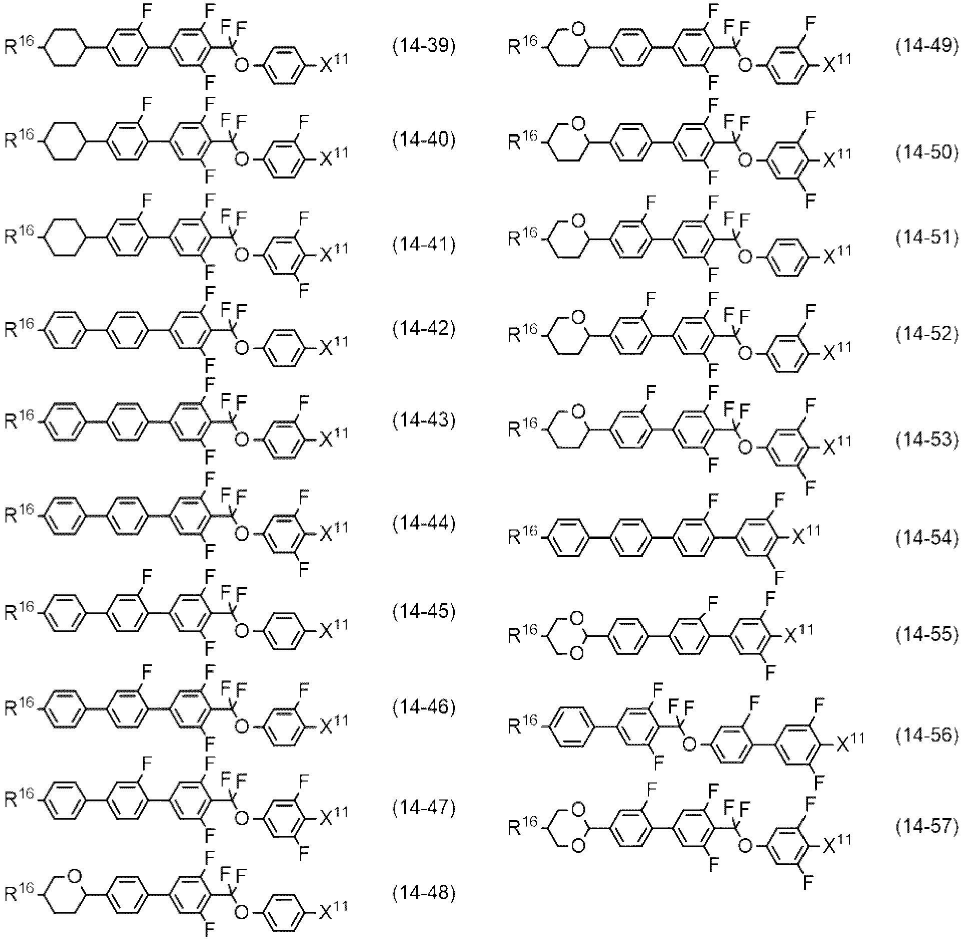

Component (d) has positive dielectric anisotropy, and significantly satisfactory stability to heat or light, and therefore is used when a composition for the IPS mode, the FFS mode, the OCB mode or the like is prepared. A content of component (d) is suitably in the range of 1% by weight to 99% by weight, preferably in the range of 10% by weight to 97% by weight, and further preferably in the range of 40% by weight to 95% by weight, based on the weight of the liquid crystal composition. When component (d) is added to the composition having negative dielectric anisotropy, the content of component (d) is preferably 30% by weight or less. Addition of component (d) allows adjustment of the elastic constant of the composition and adjustment of the voltage-transmittance curve of the device.

Component (e) is compound (15) in which a right-terminal group is -C≡N or -C≡C-C≡N. Specific examples of preferred component (e) include compounds (15-1) to (15-64). In the compounds, R¹⁷ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine. X¹² is -C≡N or -C≡C-C≡N.

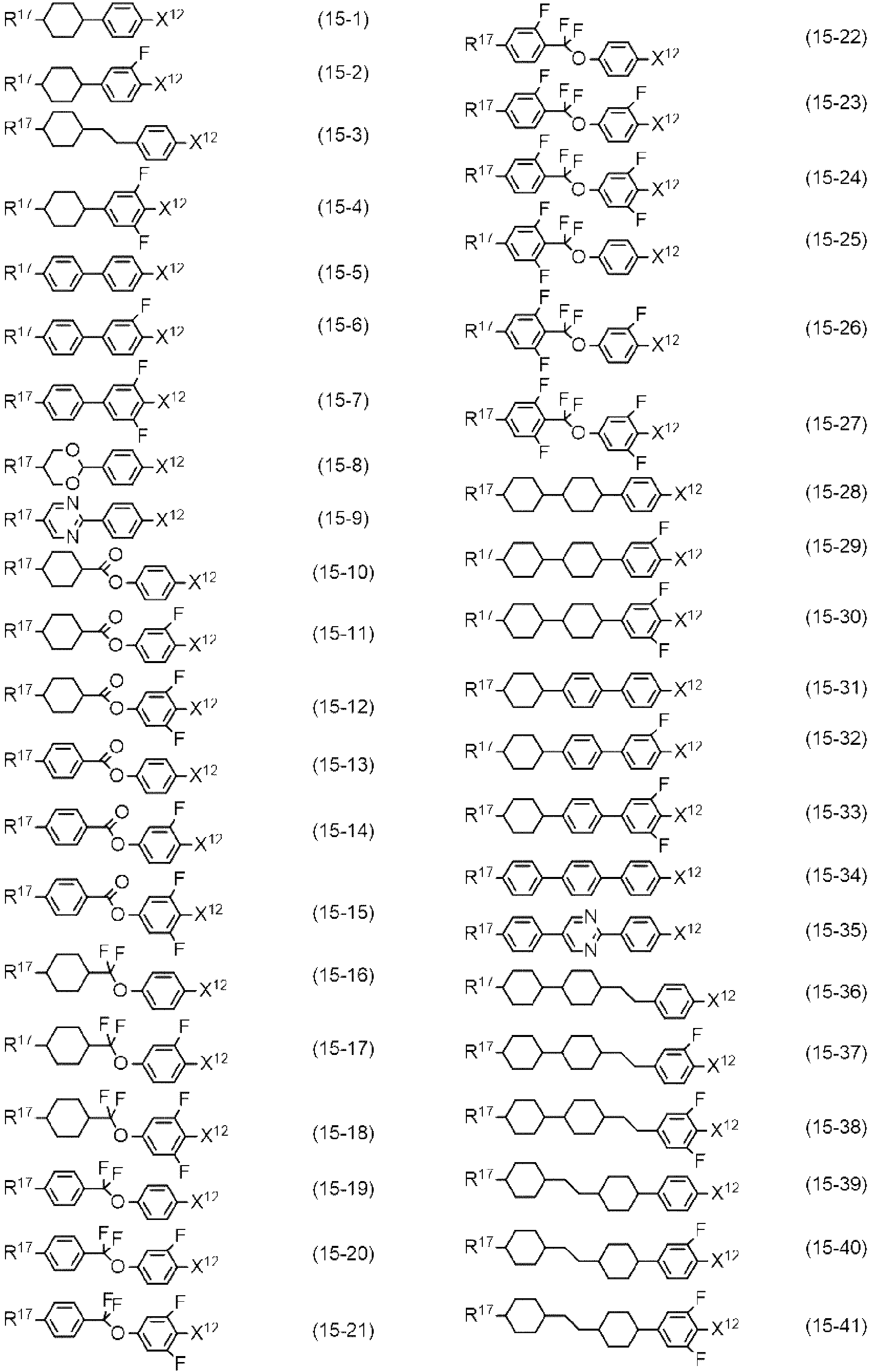

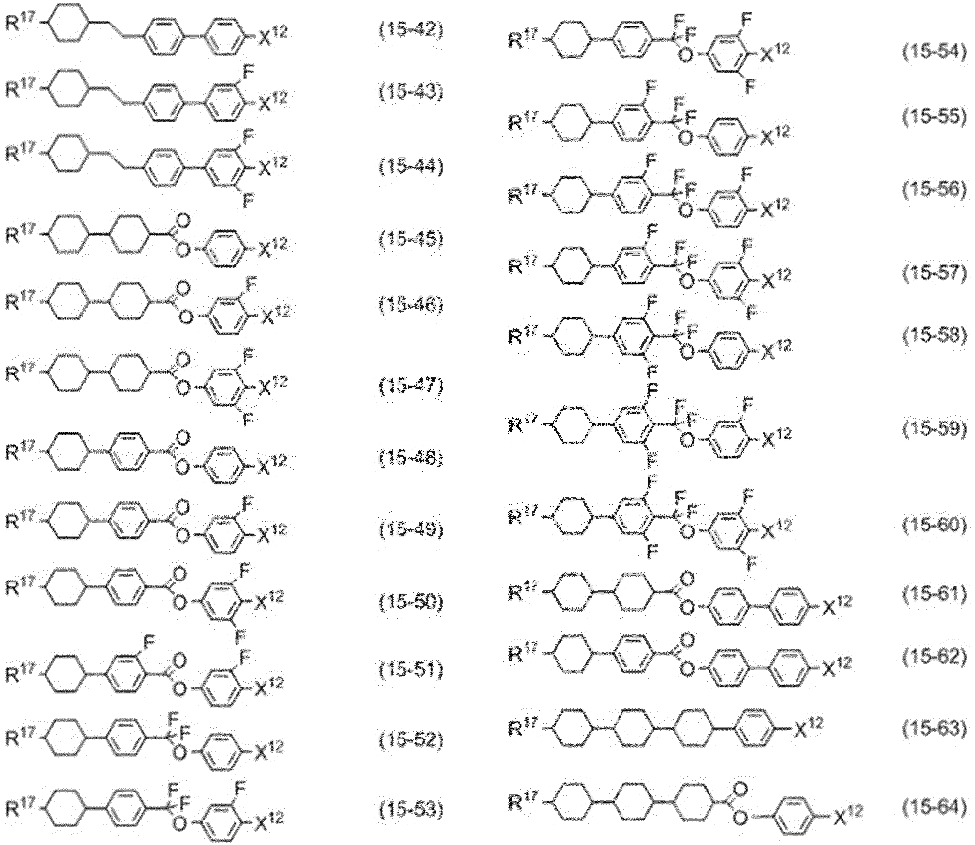

Component (e) has positive dielectric anisotropy and a value thereof is large, and therefore component (e) is used when a composition for the TN mode or the like is prepared. Addition of component (e) can increase the dielectric anisotropy of the composition. Component (e) is effective in extending the temperature range of the liquid crystal phase, adjusting the viscosity or adjusting the optical anisotropy. Component (e) is also useful for adjustment of the voltage-transmittance curve of the device.

When the composition for the TN mode or the like is prepared, a content of component (e) is suitably in the range of 1% by weight to 99% by weight, preferably in the range of 10% by weight to 97% by weight, and further preferably in the range of 40% by weight to 95% by weight, based on the weight of the liquid crystal composition. When component (e) is added to a composition having negative dielectric anisotropy, the content of component (e) is preferably 30% by weight or less. Addition of component (e) allows adjustment of the elastic constant of the composition and adjustment of the voltage-transmittance curve of the device.

A liquid crystal composition satisfying at least one of physical properties such as high stability to heat or light, high maximum temperature, low minimum temperature, small viscosity, suitable optical anisotropy (more specifically, large optical anisotropy or small optical anisotropy), large positive or negative dielectric anisotropy, large specific resistance and a suitable elastic constant (more specifically, a large elastic constant or a small elastic constant) can be prepared by combining a compound suitably selected from components (b) to (e) described above with compound (1). A device including such a composition has a wide temperature range in which the device can be used, a short response time, a large voltage holding ratio, low threshold voltage, a large contrast ratio, a small flicker rate and a long service life.

If the device is used for a long period of time, a flicker may be occasionally generated on a display screen. A flicker rate (%) can be represented by a formula: (| luminance upon applying positive voltage - luminance upon applying negative voltage | / average luminance) × 100. In a device having the flicker rate in the range of 0% to 1%, the flicker is hard to be generated on the display screen even if the device is used for a long period of time. The flicker is associated with image persistence, and is presumed to be generated according to a difference in electric potential between a positive frame and a negative frame in driving the device at an alternating current. The composition containing compound (1) is also useful for reducing generation of the flicker.

3-2. Additive

A liquid crystal composition is prepared according to a publicly known method. For example, the component compounds are mixed and dissolved in each other by heating. According to an application, an additive may be added to the composition. Specific examples of the additives include the polymerizable compound, the polymerization initiator, the polymerization inhibitor, the optically active compound, the antioxidant, the ultraviolet light absorber, the light stabilizer, the heat stabilizer, the dye and the antifoaming agent. Such additives are well known to those skilled in the art, and described in literature.

In a liquid crystal display device having the polymer sustained alignment (PSA) mode, the composition contains a polymer. The polymerizable compound is added for the purpose of forming the polymer in the composition. The polymerizable compound is polymerized by irradiation with ultraviolet light while voltage is applied between electrodes, and thus the polymer is formed in the composition. A suitable pretilt is achieved by the method, and therefore the device in which a response time is shortened and the image persistence is improved is prepared.

Preferred examples of the polymerizable compound include acrylate, methacrylate, a vinyl compound, a vinyloxy compound, propenyl ether, an epoxy compound (oxirane, oxetane) and vinyl ketone. Further preferred examples include a compound having at least one acryloyloxy, and a compound having at least one methacryloyloxy. Still further preferred examples also include a compound having both acryloyloxy and methacryloyloxy.

Still further preferred examples include compounds (M-1) to (M-18) . In the compounds, R²⁵ to R³¹ are independently hydrogen or methyl; R³², R³³ and R³⁴ are independently hydrogen or alkyl having 1 to 5 carbons, and at least one of R³², R³³ and R³⁴ is alkyl having 1 to 5 carbons; v, w and x are independently 0 or 1; and u and y are independently an integer from 1 to 10. L²¹ to L²⁶ are independently hydrogen or fluorine; and L²⁷ and L²⁸ are independently hydrogen, fluorine or methyl.

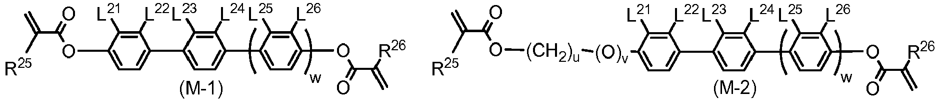

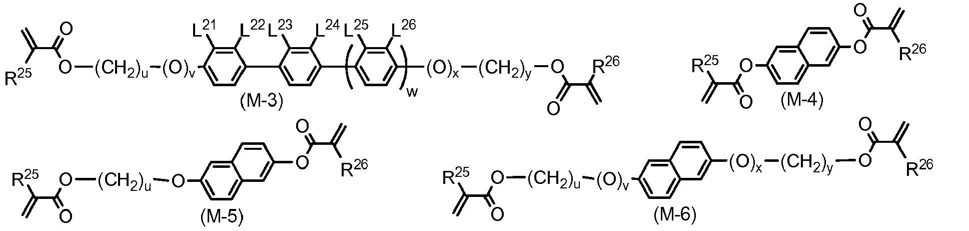

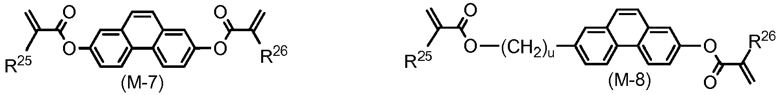

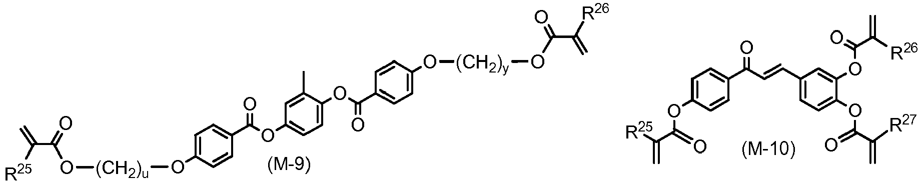

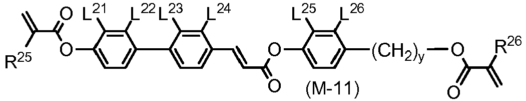

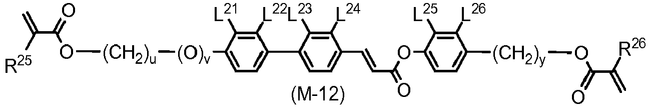

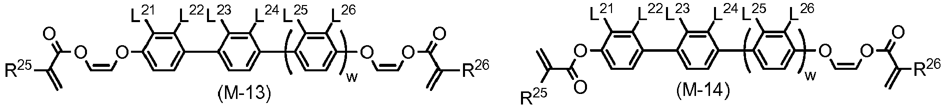

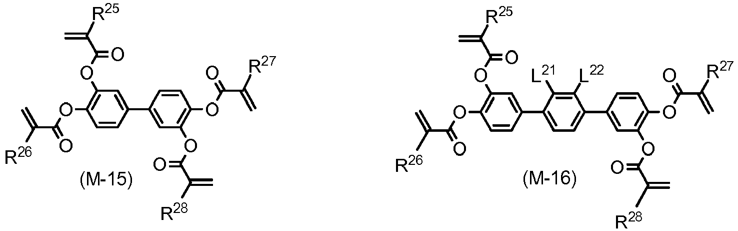

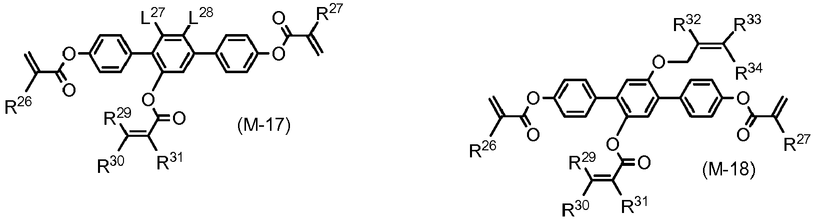

The polymerizable compound can be rapidly polymerized by adding the polymerization initiator. An amount of a remaining polymerizable compound can be reduced by optimizing reaction conditions. Examples of a photoradical polymerization initiator include TPO, 1173 and 4265 from Darocur series of BASF SE, and 184, 369, 500, 651, 784, 819, 907, 1300, 1700, 1800, 1850 and 2959 from Irgacure series thereof.

Additional examples of the photoradical polymerization initiator include 4-methoxyphenyl-2,4-bis(trichloromethyl)triazine, 2-(4-butoxystyryl)-5-trichloromethyl-1,3,4-oxadiazole, 9-phenylacridine, 9,10-benzphenazine, a benzophenone-Michler's ketone mixture, a hexaarylbiimidazole-mercaptobenzimidazole mixture, 1-(4-isopropylphenyl)-2-hydroxy-2-methylpropane-1-one, benzyl dimethyl ketal, 2-methyl-1-[4-(methylthio)phenyl]-2-morpholinopropane-1-one, a mixture of 2,4-diethylxanthone and methyl p-dimethylaminobenzoate, and a mixture of benzophenone and methyltriethanolamine.

After the photoradical polymerization initiator is added to the liquid crystal composition, polymerization can be performed by irradiation with ultraviolet light while an electric field is applied. However, an unreacted polymerization initiator or a decomposition product of the polymerization initiator may cause poor display such as image persistence in the device. In order to prevent such an event, photopolymerization may be performed with no addition of the polymerization initiator. A preferred wavelength of irradiation light is in the range of 150 nanometers to 500 nanometers . A further preferred wavelength is in the range of 250 nanometers to 450 nanometers, and a most preferred wavelength is in the range of 300 nanometers to 400 nanometers.

Upon storing the polymerizable compound, the polymerization inhibitor may be added thereto for preventing polymerization. The polymerizable compound is ordinarily added to the composition without removing the polymerization inhibitor. Examples of the polymerization inhibitor include hydroquinone, a hydroquinone derivative such as methylhydroquinone, 4-t-butylcatechol, 4-methoxyphenol and phenothiazine.

The optically active compound is effective in inducing helical structure in liquid crystal molecules to give a required twist angle, thereby preventing a reverse twist. A helical pitch can be adjusted by adding the optically active compound thereto. Two or more optically active compounds may be added for the purpose of adjusting temperature dependence of the helical pitch. Specific examples of a preferred optically active compound include compounds (Op-1) to (Op-18) described below. In compound (Op-18), ring J is 1, 4-cyclohexylene or 1,4-phenylene, and R28 is alkyl having 1 to 10 carbons. Asterisk mark (*) represents asymmetrical carbon.

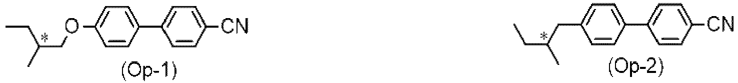

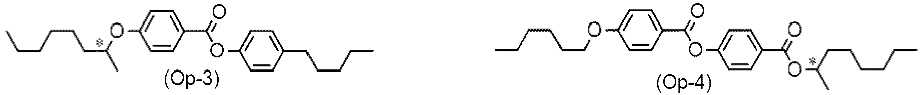

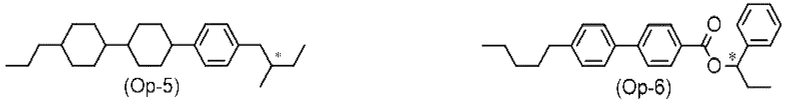

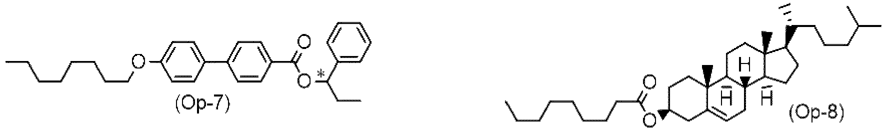

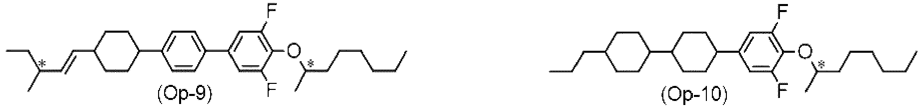

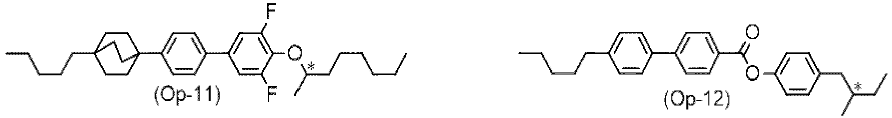

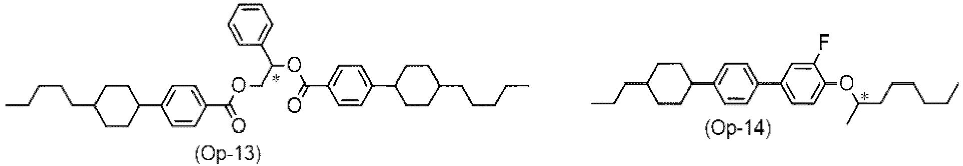

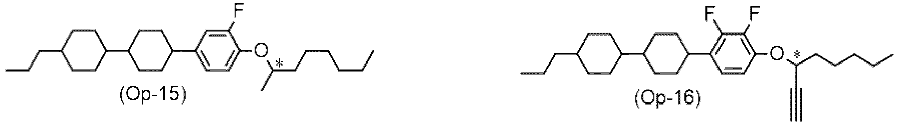

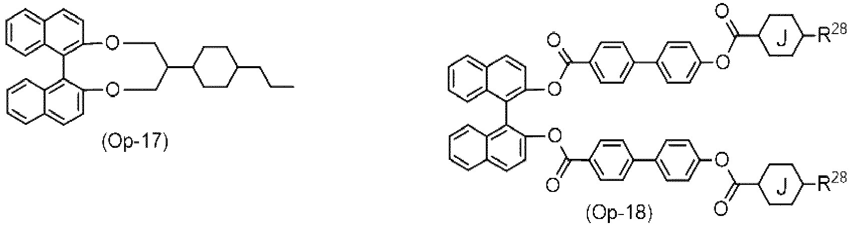

The antioxidant is effective for maintaining the large voltage holding ratio. Preferred examples of the antioxidant include compounds (AO-1) and (AO-2) described below; and Irganox 415, Irganox 565, Irganox 1010, Irganox 1035, Irganox 3114 and Irganox 1098 (trade names; BASF SE). The ultraviolet light absorber is effective for preventing a decrease of the maximum temperature. Preferred examples of the ultraviolet light absorber include a benzophenone derivative, a benzoate derivative and a triazole derivative, and specific examples include compounds (AO-3) and (AO-4) described below; Tinuvin 329, Tinuvin P, Tinuvin 326, Tinuvin 234, Tinuvin 213, Tinuvin 400, Tinuvin 328 and Tinuvin 99-2 (trade names; BASF SE); and 1,4-diazabicyclo[2.2.2]octane (DABCO).

The light stabilizer such as an amine having steric hindrance is preferred for maintaining the large voltage holding ratio. Preferred examples of the light stabilizer include compounds (AO-5), (AO-6) and (AO-7) described below; Tinuvin 144, Tinuvin 765 and Tinuvin 770DF (trade names; BASF SE) ; and LA-77Y and LA-77G (trade names; ADEKA Corporation). The heat stabilizer is also effective for maintaining the large voltage holding ratio, and specific preferred examples include Irgafos 168 (trade name; BASF SE). A dichroic dye such as an azo dye or an anthraquinone dye is added to the composition to be adapted for a device having a guest host (GH) mode. The antifoaming agent is effective for preventing foam formation. Preferred examples of the antifoaming agent include dimethyl silicone oil and methylphenyl silicone oil.

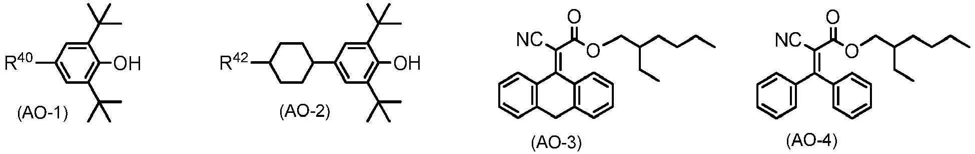

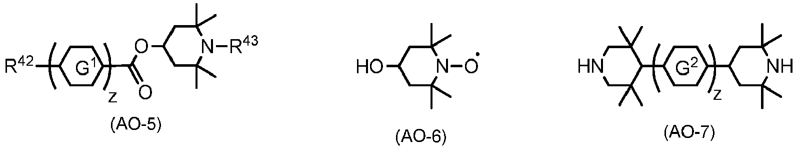

In compound (AO-1), R⁴⁰ is alkyl having 1 to 20 carbons, alkoxy having 1 to 20 carbons, -COOR⁴¹ or -CH₂CH₂COOR⁴¹, in which R⁴¹ is alkyl having 1 to 20 carbons. In compounds (AO-2) and (AO-5), R⁴² is alkyl having 1 to 20 carbons. In compound (AO-5), R⁴³ is hydrogen, methyl or O˙ (oxygen radical); and ring G¹ is 1,4-cyclohexylene or 1,4-phenylene; in compound (AO-7), ring G² is 1,4-cyclohexylene, 1,4-phenylene, or 1,4-phenylene in which at least one hydrogen is replaced by fluorine; and in compounds (AO-5) and (AO-7), z is 1, 2 or 3.

### 4. Liquid crystal display device

The liquid crystal composition can be used in a liquid crystal display device having an operating mode such as the PC mode, the TN mode, the STN mode, the OCB mode and the PSA mode, and driven by an active matrix mode. The composition can also be used in a liquid crystal display device having the operating mode such as the PC mode, the TN mode, the STN mode, the OCB mode, the VA mode and the IPS mode, and driven by a passive matrix mode. The devices can be applied to any of a reflective type, a transmissive type and a transflective type.

The composition is also suitable for a nematic curvilinear aligned phase (NCAP) device, and the composition is microencapsulated herein. The composition can also be used in a polymer dispersed liquid crystal display device (PDLCD) or a polymer network liquid crystal display device (PNLCD). In the compositions, a large amount of polymerizable compound is added. On the other hand, when a proportion of the polymerizable compound is 10% by weight or less based on the weight of the liquid crystal composition, the liquid crystal display device having the PSA mode is prepared. A preferred proportion is in the range of 0.1% by weight to 2% by weight based thereon. A further preferred proportion is in the range of 0.2% by weight to 1.0% by weight based thereon. The device having the PSA mode can be driven by the driving mode such as the active matrix mode and the passive matrix mode. Such devices can be applied to any of the reflective type, the transmissive type and the transflective type.

### Examples

### 1. Example of compound (1)

The invention will be described in greater detail by way of Examples . The Examples each is a typical example, and therefore the invention is not limited by the Examples. Compound (1) was prepared according to procedures described below. The thus prepared compound was identified by methods such as an NMR analysis. Physical properties of a compound and a composition, and characteristics of a device were measured by methods described below.

NMR analysis: For measurement, DRX-500 made by Bruker BioSpin Corporation was used. In ¹H-NMR measurement, a sample was dissolved in a deuterated solvent such as CDCl₃, and measurement was carried out under conditions of room temperature, 500 MHz and 16 times of accumulation. Tetramethylsilane was used as an internal standard. In ¹⁹F-NMR measurement, CFCl₃ was used as an internal standard, and measurement was carried out under conditions of 24 times of accumulation. In explaining nuclear magnetic resonance spectra obtained, s, d, t, q, quin, sex and m stand for a singlet, a doublet, a triplet, a quartet, a quintet, a sextet and a multiplet, and br being broad, respectively.

Gas chromatographic analysis: For measurement, GC-2010 Gas Chromatograph made by Shimadzu Corporation was used. As a column, a capillary column DB-1 (length 60 m, bore 0.25 mm, film thickness 0.25 µm) made by Agilent Technologies, Inc. was used. As a carrier gas, helium (1 mL/minute) was used. A temperature of a sample vaporizing chamber and a temperature of a detector (FID) were set to 300°C and 300°C, respectively. A sample was dissolved in acetone and prepared to be a 1 weight % solution, and then 1 microliter of the solution obtained was injected into the sample vaporizing chamber. As a recorder, GC Solution System made by Shimadzu Corporation or the like was used.

HPLC analysis: For measurement, Prominence (LC-20AD; SPD-20A) made by Shimadzu Corporation was used. As a column, YMC-Pack ODS-A (length 150 mm, bore 4.6 mm, particle diameter 5 µm) made by YMC Co., Ltd. was used. As an eluate, acetonitrile and water were appropriately mixed and used. As a detector, a UV detector, an RI detector, a CORONA detector or the like was appropriately used. When the UV detector was used, a detection wavelength was set to 254 nanometers. A sample was dissolved in acetonitrile and prepared to be a 0.1 weight % solution, and then 1 microliter of the solution was introduced into a sample chamber. As a recorder, C-R7Aplus made by Shimadzu Corporation was used.

Ultraviolet-Visible spectrophotometry: For measurement, PharmaSpec UV-1700 made by Shimadzu Corporation was used. A detection wavelength was adjusted in the range of 190 nanometers to 700 nanometers. A sample was dissolved in acetonitrile and prepared to be a 0.01 mmol/L solution, and measurement was carried out by putting the solution in a quartz cell (optical path length: 1 cm).

Sample for measurement: Upon measuring phase structure and a transition temperature (a clearing point, a melting point, a polymerization starting temperature or the like), the compound itself was used as a sample. Upon measuring physical properties such as maximum temperature of a nematic phase, viscosity, optical anisotropy and dielectric anisotropy, a mixture of the compound and a base liquid crystal was used as a sample.

Extrapolation method: When the sample prepared by mixing the compound with the base liquid crystal was used, measurement was carried out as described below. The sample was prepared by mixing 15% by weight of the compound and 85% by weight of the base liquid crystal. From a measured value of the sample, an extrapolated value was calculated according to the following equation, and the calculated value was described: [extrapolated value] = (100 × [measured value of a sample] - [% by weight of a base liquid crystal] × [measured value of the base liquid crystal]) / [% by weight of a compound].

When crystals (or a smectic phase) precipitated at 25°C at the ratio, a ratio of the compound to the base liquid crystal was changed in the order of (10% by weight : 90% by weight), (5% by weight : 95% by weight), and (1% by weight : 99% by weight), and the physical properties of the sample were measured at a ratio at which no crystal (or no smectic phase) precipitated at 25°C. In addition, unless otherwise noted, the ratio of the compound to the base liquid crystal was (15% by weight : 85% by weight).

When the dielectric anisotropy of the compound was zero or positive, base liquid crystal (A) described below was used. A proportion of each component was expressed in terms of weight percent (% by weight).

| | |
|---|---|
| 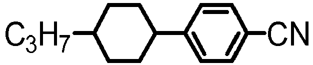 | 24% |
| 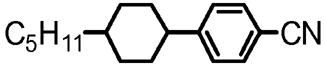 | 36% |
| 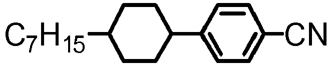 | 25% |
| 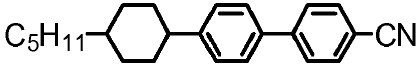 | 15% |

When the dielectric anisotropy of the compound was zero or negative, base liquid crystal (B) described below was used. A proportion of each component was expressed in terms of weight percent (% by weight).

| | |
|---|---|
| 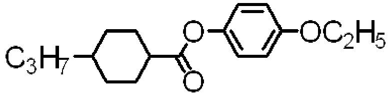 | 17.2% |
| 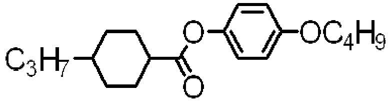 | 27.6% |
| 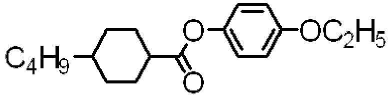 | 20.7% |
| 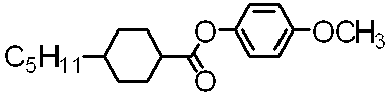 | 20.7% |
| 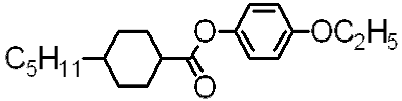 | 13.8% |

Base liquid crystal (C): Base liquid crystal (C) containing a fluorine-based compound as a component may be occasionally used.

| | |
|---|---|
| 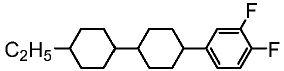 | 16.67% |
| 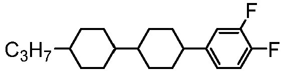 | 16.67% |
| 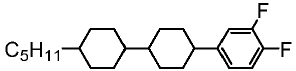 | 16.67% |
| 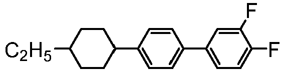 | 6.25% |
| 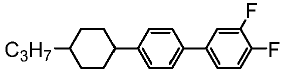 | 6.25% |
| 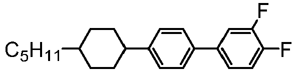 | 12.49% |
| 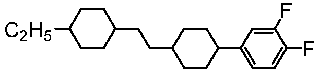 | 12.50% |
| 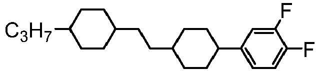 | 6.25% |
| 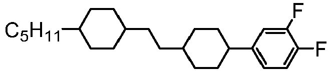 | 6.25% |

Measuring method: Physical properties were measured according to methods described below. Most of the methods are described in the Standard of Japan Electronics and Information Technology Industries Association (JEITA) discussed and established in JEITA (JEITA ED-2521B) . A modification of the methods were also used. No thin film transistor (TFT) was attached to a TN device used for measurement.
(1) Phase structure: A sample was placed on a hot plate in a melting point apparatus (FP-52 Hot Stage made by Mettler-Toledo International Inc.) equipped with a polarizing microscope. A state of phase and a change thereof were observed with the polarizing microscope while the sample was heated at a rate of 3°C per minute, and a kind of the phase was specified.
(2) Transition temperature (°C): For measurement, a differential scanning calorimeter, Diamond DSC System, made by PerkinElmer, Inc., or a high sensitivity differential scanning calorimeter, X-DSC7000, made by SII NanoTechnology Inc. was used. A sample was heated and then cooled at a rate of 3°C per minute, and a starting point of an endothermic peak or an exothermic peak caused by a phase change of the sample was determined by extrapolation, and thus a transition temperature was determined. A melting point and a polymerization starting temperature of a compound were also measured using the apparatus. Temperature at which a compound undergoes transition from a solid to a liquid crystal phase such as the smectic phase and the nematic phase may be occasionally abbreviated as "minimum temperature of the liquid crystal phase." Temperature at which the compound undergoes transition from the liquid crystal phase to liquid may be occasionally abbreviated as "clearing point."
   A crystal was expressed as C. When the crystals were distinguishable into two kinds, each of the crystals was expressed as C₁ or C₂. The smectic phase or the nematic phase was expressed as S or N. When a phase was distinguishable such as smectic A phase, smectic B phase, smectic C phase and smectic F, the phase was expressed as S_{A}, S_{B}, S_{C} and S_{F}, respectively. A liquid (isotropic) was expressed as I. A transition temperature was expressed as "C 50.0 N 100.0 I, " for example. The expression indicates that a transition temperature from the crystals to the nematic phase is 50.0°C, and a transition temperature from the nematic phase to the liquid is 100.0°C.
(3) Compatibility of compound: Samples in which the base liquid crystal and the compound were mixed for proportions of the compounds to be 20% by weight, 15% by weight, 10% by weight, 5% by weight, 3% by weight or 1% by weight were prepared. The samples were put in a glass vials, and kept in freezers at -10°C or -20°C for a predetermined period of time. Whether a nematic phase of the samples was maintained or crystals (or a smectic phase) precipitated was observed. Conditions on which the nematic phase was maintained were used as a measure of the compatibility. Proportions of the compounds and each temperature in the freezers may be occasionally changed when necessary.
(4) Maximum temperature of nematic phase (T_{NI} or NI; °C) : A sample was placed on a hot plate in a melting point apparatus equipped with a polarizing microscope, and heated at a rate of 1°C per minute. Temperature when part of the sample began to change from a nematic phase to an isotropic liquid was measured. When the sample was a mixture of compound (1) and the base liquid crystal, the maximum temperature was expressed in terms of a symbol T_{NI}. The value was calculated using the extrapolation method described above. When the sample was a mixture of compound (1) and a compound selected from compounds (2) to (15), the measured value was expressed in terms of a symbol NI. A maximum temperature of the nematic phase may be occasionally abbreviated as "maximum temperature."
(5) Minimum temperature of nematic phase (T_{C}; °C) : Samples each having a nematic phase were put in glass vials and kept in freezers at temperatures of 0°C, -10°C, -20°C, -30°C and -40°C for 10 days, and then liquid crystal phases were observed. For example, when the sample was maintained in the nematic phase at -20°C and changed to crystals or a smectic phase at -30°C, T_{C} was expressed as T_{C} < -20°C. A minimum temperature of the nematic phase may be occasionally abbreviated as "minimum temperature."
(6) Viscosity (bulk viscosity; η; measured at 20°C; mPa·s) : For measurement, a cone-plate (E type) rotational viscometer made by Tokyo Keiki Inc. was used.
(7) Optical anisotropy (refractive index anisotropy; measured at 25°C; Δn): Measurement was carried out by an Abbe refractometer with a polarizing plate mounted on an ocular, using light at a wavelength of 589 nanometers. A surface of a main prism was rubbed in one direction, and then a sample was added dropwise onto the main prism. A refractive index (n∥) was measured when a direction of polarized light was parallel to a direction of rubbing. A refractive index (n⊥) was measured when the direction of polarized light was perpendicular to the direction of rubbing. A value of optical anisotropy (Δn) was calculated from an equation: Δn = n∥- n⊥.
(8) Specific resistance (ρ; measured at 25°C; Ωcm) : Into a vessel equipped with electrodes, 1.0 milliliter of sample was injected. A direct current voltage (10 V) was applied to the vessel, and a direct current after 10 seconds was measured. Specific resistance was calculated from the following equation: (specific resistance) = {(voltage) × (electric capacity of a vessel)} / {(direct current) × (dielectric constant of vacuum)}.
(9) Voltage holding ratio (VHR-1; measured at 25°C; %) : A TN device used for measurement had a polyimide alignment film, and a distance (cell gap) between two glass substrates was 5 micrometers. A sample was put in the device, and then the device was sealed with an ultraviolet-curable adhesive. The device was charged by applying a pulse voltage (60 microseconds at 5 V). A decaying voltage was measured for 16.7 milliseconds with a high-speed voltmeter, and area A between a voltage curve and a horizontal axis in a unit cycle was determined. Area B is an area without decay. A voltage holding ratio is expressed in terms of a percentage of area A to area B.
(10) Voltage holding ratio (VHR-2; measured at 80°C; %) : A voltage holding ratio was measured according to the method described above except that the voltage holding ratio was measured at 80°C in place of 25°C. The results obtained were expressed in terms of a symbol VHR-2.
(11) Flicker rate (measured at 25°C; %): For measurement, 3298F Multimedia Display Tester made by Yokogawa Electric Corporation was used. A light source was LED. A sample was put in a normally black mode FFS device in which a distance (cell gap) between two glass substrates was 3.5 micrometers, and a rubbing direction was anti-parallel. The device was sealed with an ultraviolet-curable adhesive. Voltage was applied to the device, and a voltage having a maximum amount of light transmitted through the device was measured. A sensor part was brought close to the device while the voltage was applied, and a flicker rate displayed thereon was read.
(12) Viscosity (rotational viscosity; γ1; measured at 25°C; mPa·s) : Measurement was carried out according to a method described in M. Imai et al., Molecular Crystals and Liquid Crystals, Vol. 259, p. 37 (1995) . A sample was put in a VA device in which a distance (cell gap) between two glass substrates was 20 micrometers. Voltage was applied stepwise to the device from 39 V to 50 V at an increment of 1 V. After a period of 0.2 second with no voltage application, voltage was repeatedly applied under conditions of only one rectangular wave (rectangular pulse; 0.2 second) and no voltage application (2 seconds) . A peak current and a peak time of transient current generated by the applied voltage were measured. A value of rotational viscosity was obtained from the measured values and equation (8) on page 40 of the paper presented by M. Imai et al. Dielectric anisotropy required for the calculation was measured in a section of dielectric anisotropy described below.
(13) Dielectric anisotropy (Δε; measured at 25°C): A value of dielectric anisotropy was calculated from an equation: Δε =ε∥ - ε⊥. A dielectric constant (ε∥ and ε⊥) was measured as described below.
   (1) Measurement of dielectric constant (ε∥): An ethanol (20 mL) solution of octadecyltriethoxysilane (0.16 mL) was applied to a well-cleaned glass substrate. After rotating the glass substrate with a spinner, the glass substrate was heated at 150°C for 1 hour. A sample was put in a VA device in which a distance (cell gap) between two glass substrates was 4 micrometers, and the device was sealed with an ultraviolet-curable adhesive. Sine waves (0.5 V, 1 kHz) were applied to the device, and after 2 seconds, a dielectric constant (ε∥) of liquid crystal molecules in a major axis direction was measured.
   (2) Measurement of dielectric constant (ε⊥) : A polyimide solution was applied to a well-cleaned glass substrate. After calcining the glass substrate, rubbing treatment was applied to the alignment film obtained. A sample was put in a TN device in which a distance (cell gap) between two glass substrates was 9 micrometers and a twist angle was 80 degrees. Sine waves (0.5 V, 1 kHz) were applied to the device, and after 2 seconds, a dielectric constant (ε⊥) of liquid crystal molecules in a minor axis direction was measured.
(14) Elastic constant (K₁₁ and K₃₃; measured at 25°C; pN): For measurement, Elastic Constant Measurement System Model EC-1 made by TOYO Corporation was used. A sample was put in a vertical alignment device in which a distance (cell gap) between two glass substrates was 20 micrometers. An electric charge from 20 V to 0 V was applied to the device, and electrostatic capacity (C) and applied voltage (V) were measured. The measured values were fitted to equation (2.98) and equation (2.101) on page 75 of "Liquid Crystal Device Handbook (Ekisho Debaisu Handobukku in Japanese; Nikkan Kogyo Shimbun, Ltd.)," and values of elastic constants were obtained from equation (2.100).
(15) Threshold voltage (Vth; measured at 25°C; V) : For measurement, an LCD-5100 luminance meter made by Otsuka Electronics Co., Ltd. was used. A light source was a halogen lamp. A sample was put in a normally black mode VA device in which a distance (cell gap) between two glass substrates was 4 micrometers and a rubbing direction was anti-parallel, and the device was sealed with an ultraviolet-curable adhesive. A voltage (60 Hz, rectangular waves) to be applied to the device was stepwise increased from 0 V to 20 V at an increment of 0.02 V. On the occasion, the device was irradiated with light from a direction perpendicular to the device, and an amount of light transmitted through the device was measured. A voltage-transmittance curve was prepared, in which the maximum amount of light corresponds to 100% transmittance and the minimum amount of light corresponds to 0% transmittance. A threshold voltage is expressed in terms of voltage at 10% transmittance.
(16) Response time (τ; measured at 25°C; ms) : For measurement, an LCD-5100 luminance meter made by Otsuka Electronics Co., Ltd. was used. A light source was a halogen lamp. A low-pass filter was set to 5 kHz. A sample was put in a normally black mode PVA device in which a distance (cell gap) between two glass substrates was 3.2 micrometers, and a rubbing direction was anti-parallel. The device was sealed with an ultraviolet-curable adhesive. The device was applied with a voltage of a little exceeding a threshold voltage for 1 minute, and then was irradiated with ultraviolet light of 23.5 mW/cm² for 8 minutes, while applying a voltage of 5.6 V. A voltage (rectangular waves; 60 Hz, 10 V, 0.5 second) was applied to the device. On the occasion, the device was irradiated with light from a direction perpendicular to the device, and an amount of light transmitted through the device was measured. The maximum amount of light corresponds to 100% transmittance, and the minimum amount of light corresponds to 0% transmittance. A response time was expressed in terms of time required for a change from 90% transmittance to 10% transmittance (fall time; millisecond).

### Synthesis Example 1

### Synthesis of compound (No. 121)

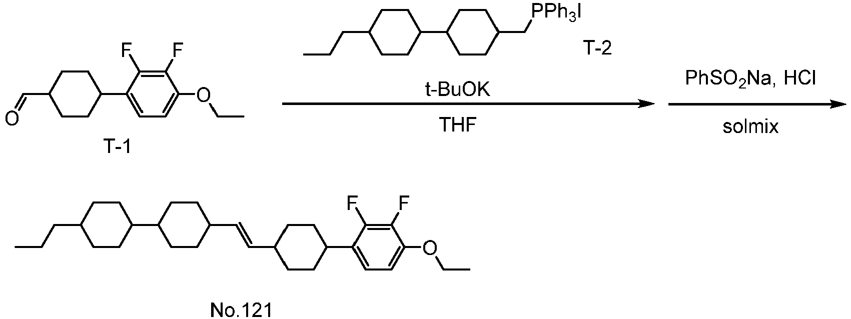

### First step:

A THF (100 mL) solution of compound (T-2) (15.5 g, 27.5 mmol) prepared by a publicly known method was cooled to -60 °C, and potassium t-butoxide (3.08 g, 27.5 mmol) was added dropwise thereto, and the resulting mixture was stirred for 1 hour. Thereto, a THF (100 mL) solution of compound (T-1) (6.7 g, 25 mmol) prepared by a publicly known method was added dropwise, and the resulting mixture was returned to room temperature while stirring. The resulting reaction mixture was poured into water, and an ordinary post-treatment was applied thereto, and the resulting solution was purified by silica gel chromatography. To the resulting purified material (8.3 g, 17.6 mmol; 70%), solmix A-11 (100 mL), toluene (50 mL and 6 N hydrochloric acid (20 mL) were added, and the resulting mixture was refluxed under heating for 4 days. An ordinary post-treatment was applied thereto, and the resulting material was purified by column chromatography and recrystallization to obtain compound (No. 121) (4.6 g, 9.75 mmol; 55%).

¹H-NMR (CDCl₃; δ ppm): 6.84 (1H, dt, 7.5 Hz, 2 Hz), 6.66 (1H, t, 7.3 Hz), 5.34 - 5.33 (2H, m), 4.09 (2H, q, 7.1 Hz), 2.77 - 2.70 (1H, m), 1.87 - 1.68 (14H, m), 1.48 - 1.42 (5H, m), 1.32 - 1.22 (4H,m), 1.14 - 1.12 (3H, m), 1.00 - 0.95 (8H, m), 0.88 - 0.82(5H, m).

Phase transition temperature: C 33.7 S_{C} 221.1 N 346.9 I. Maximum temperature (T_{NI}) = 269.3°C; dielectric anisotropy (Δε) =-4.76; optical anisotropy (Δn) = 0.131; viscosity (η) = 40.8.

### Synthesis Example 2

### Synthesis of compound (No. 122)

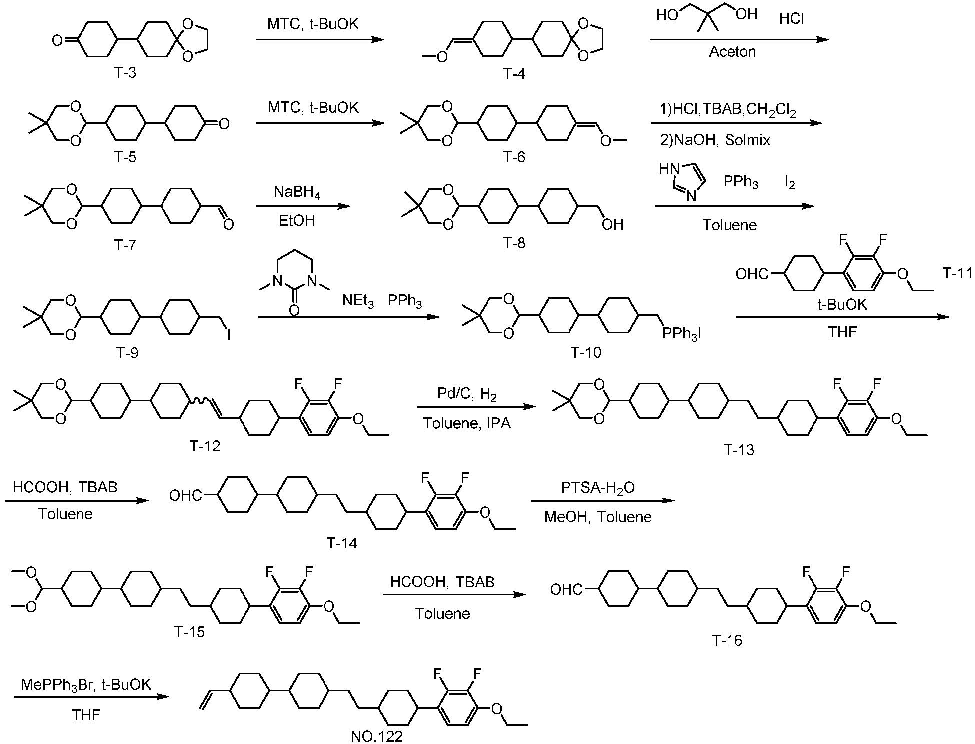

### First step:

A THF (2 L) solution of (methoxymethyl)triphenyl phosphonium chloride (482.08 g, 1.41 mol) was cooled to -60°C, and potassium t-butoxide (215.66 g, 1.92 mol) was added dropwise thereto, and the resulting mixture was stirred for 1 hour. Thereto, a THF (900 mL) solution of compound (T-3) (300.67 g, 1.26 mol) prepared by a publicly known method was added dropwise, and the resulting mixture was returned to room temperature while stirring. The resulting reaction mixture was poured into water, and an ordinary post-treatment was applied thereto, and the resulting material was purified by silica gel chromatography to obtain compound (T-4) (293.17 g, 1.10 mol; 87%).

### Second step:

Then, 6 N hydrochloric acid (180mL, 1.08 mol) was added dropwise to an acetone solution of compound (T-4) (293.17 g, 1.10 mol) and 2,2-dimethyl-1,3-propanediol (125.71 g, 1.21 mol), and the resulting mixture was stirred at room temperature for several days. An ordinary post-treatment was applied thereto, and the resulting material was purified by silica gel chromatography and recrystallization to obtain compound (T-5) (140.51 g, 0.48 mol; 43%).

### Third step:

A THF (700 mL) solution of (methoxymethyl)triphenyl phosphonium chloride (197.39 g, 0.58 mol) was cooled to -60°C, and potassium t-butoxide (59.29 g, 0.53 mol) was added dropwise thereto, and the resulting mixture was stirred for 1 hour. Thereto, a THF (700 mL) solution of compound (T-5) (140 g, 0.48 mol) prepared by a publicly known method was added dropwise, and the resulting mixture was returned to room temperature while stirring. The resulting reaction mixture was poured into water, and an ordinary post-treatment was applied thereto, and the resulting material was purified by silica gel chromatography to obtain compound (T-6) (153.51 g, 0.48 mol; quantitative).

### Fourth step:

Then, 6 N hydrochloric acid (380 mL, 1.14 mol) was added dropwise to a methylene chloride (600 mL) solution of compound (T-6) (153 g, 0.47 mol) and tetrabutylammonium bromide (TBAB; 9.28 g, 0.029 mol), and the resulting mixture was stirred for 22 hours. After an ordinary post-treatment was applied thereto, NaOH (1.13 g, 0.028 mol) and Solmix A-11 (1.1 L) were added to the post-treated solution, and the resulting mixture was stirred at room temperature for 18 hours. An ordinary post-treatment was applied thereto to obtain compound (T-7) (150.28 g, 0.49 mol, cis / trans = 9 / 91; quantitative). Solmix (registered trade name) A-11 is a mixture of ethanol (85.5%), methanol (13.4%) and isopropanol (1.1%), and was purchased from Japan Alcohol Trading Co., Ltd.

### Fifth step:

An ethanol (500 mL) solution of sodium borohydride (11.21 g, 0.30 mol) was ice-cooled, and an ethanol (600 mL) solution of compound (T-7) (146.21 g, 0.47 mol) was added dropwise thereto, and the resulting mixture was stirred at room temperature for 18 hours. An ordinary post-treatment was applied thereto, and the resulting material was purified by silica gel chromatography and recrystallization to obtain compound (T-8) (81.97 g, 0.26 mol; 56%).

### Sixth step:

A toluene (200 mL) solution of compound (T-8) (30.54 g, 0.098 mol), imidazole (8.82 g, 0.13 mol) and triphenyl phosphine (34.06 g, 0.13 mol) was ice-cooled, and a toluene (300 mL) solution of iodine (32.9 g, 0.13 mol) was added dropwise thereto, and the resulting mixture was stirred at room temperature for several days. An ordinary post-treatment was applied thereto, and the resulting material was purified by silica gel chromatography and recrystallization to obtain compound (T-9) (31.59 g, 0.075 mol; 77%).

### Seventh step:

To compound (T-9) (31.59 g, 0.075 mol), triethylamine (0.8 g, 7.91 mmol), triphenyl phosphine (20.89 g, 0.080 mol) and 1,3-dimethyl-3,4,5,6-tetrahydro-2(1H)-pyrimidinone (11 mL) were added, and the resulting mixture was stirred under heating for seven days. An ordinary treatment was applied thereto to obtain compound (T-10) (47.56 g, 0.07 mol; 93%).

### Eighth step:

A THF (100 mL) solution of compound (T-10) (15.61 g, 0.023 mol) was cooled to -60°C, and potassium t-butoxide (2.80 g, 0.025 mol) was added dropwise thereto, and the resulting mixture was stirred for 1 hour. Thereto, a THF (40 mL) solution of compound (T-11) (5.37 g, 0.02 mol) prepared by a publicly known method was added dropwise, and the resulting mixture was returned to room temperature while stirring. The resulting reaction mixture was poured into water, and an ordinary post-treatment was applied thereto, and the resulting material was purified by silica gel chromatography to obtain compound (T-12) (10.10 g, 0.019 mol; 93%).

### Ninth step:

Compound (T-12) (10.10 g, 0.019 mol) was dissolved in toluene (100 mL) and 2-propanol (IPA; 100 mL), and Pd/C (0.24 g) was further added thereto, and the resulting mixture was stirred under a hydrogen atmosphere at room temperature until hydrogen was not absorbed. After Pd/C was removed therefrom, the resulting material was purified by silica gel chromatography and recrystallization to obtain compound (T-13) (6.21 g, 0.011 mol; 61%).

### Tenth step:

To a toluene (300 mL) solution of compound (T-13) (6.21 g, 0.011 mol) and tetrabutylammonium bromide (TBAB; 0.84 g, 2.61 mmol), 87% formic acid (40 mL) was added, and the resulting mixture was stirred under heating for 11 hours. An ordinary post-treatment was applied thereto to obtain compound (T-14) (6.47 g, 0.014 mol, cis / trans = 24 / 76; quantitative).

### Eleventh step:

To compound (T-14) (5.23 g, 0.011 mol), toluene (75 mL), methanol (150 mL) and p-toluenesulfonic acid (PTSA; 0.71 g, 3.73 mmol) were added, and the resulting mixture was stirred under heating for 3 hours. An ordinary post-treatment was applied thereto to obtain compound (T-15) (5.40 g, 0.011 mol; quantitative).

### Twelfth step:

To a toluene (100 mL) solution of compound (T-15) (5.40 g, 0.011 mol) and tetrabutylammonium bromide (TBAB; 0.76 g, 2.36 mmol), 87% formic acid (20 mL) was added, and the resulting mixture was stirred at room temperature for several hours. An ordinary post-treatment was applied thereto to obtain compound (T-16) (3.72 g, 8.08 mmol; 77%).

### Thirteenth step:

A THF (50 mL) solution of methyltriphenylphosphonium bromide (3.47 g, 10.47 mol) was cooled to -70°C, and potassium t-butoxide (1.21 g, 10.78 mmol) was added dropwise, and the resulting mixture was stirred for 1 hour. Thereto, a THF (50 mL) solution of compound (T-16) (3.72 g, 8.08 mmol) was added dropwise, and the resulting mixture was returned to room temperature while stirring. The resulting reaction mixture was poured into water, and an ordinary post-treatment was applied thereto, and the resulting material was purified by silica gel chromatography and recrystallization to obtain compound (No. 122) (2.86 g, 6.24 mmol; 77%).

¹H-NMR (CDCl_{3;} δ ppm): 6.86 - 6.80 (1H, m), 6.66 (1H, dt, 7.5 Hz, 1.5 Hz), 5.81 - 5.73 (1H, m), 4.95 (1H, d, 16 Hz), 4.87 (1H, d, 10.5 Hz), 4.08 (2H, q, 7.5 Hz), 2.74 (1H, m), 1.85 - 1.69 (12H, m), 1.44 - 1.41 (6H, m), 1.21 - 1.19 (5H, m), 1.09 - 1.07 (10H, m), 1.04 - 1.02 (3H, m).

Phase transition temperature: C 4.8 S_{B} 165.8 N 277.6 I. Maximum temperature (T_{NI}) = 234.6°C; dielectric anisotropy (Δε) =-4.87; optical anisotropy (Δn) = 0.127; viscosity (η) = 56.5.

### Synthesis Example 3

### Synthesis of compound (No. 159)

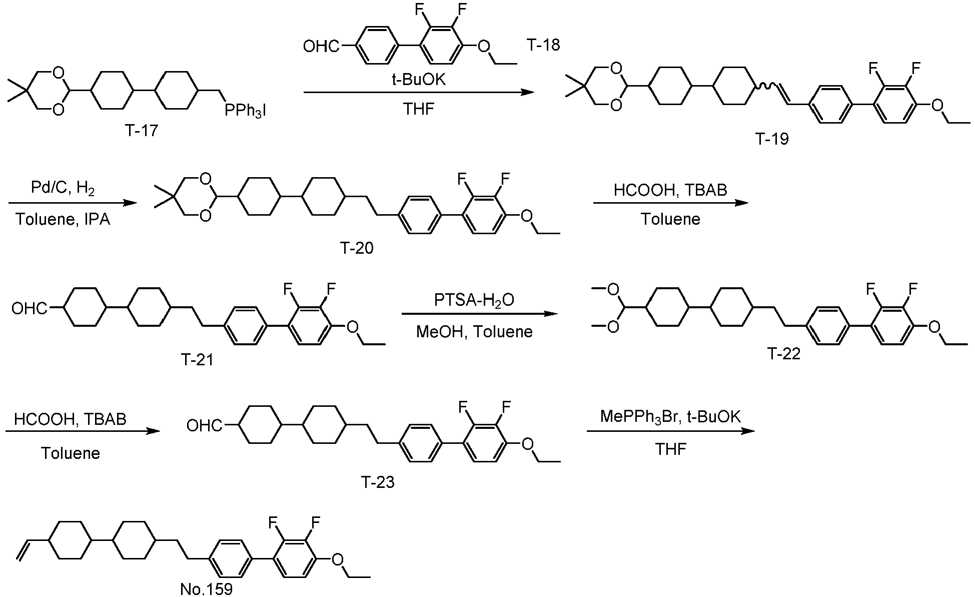

### First step:

A THF (200 mL) solution of compound (T-17) (15.86 g, 0.023 mol) was cooled to -40°C, and potassium t-butoxide (2.72 g, 0.024 mol) was added dropwise thereto, and the resulting mixture was stirred for 1 hour. Thereto, a THF (40 mL) solution of compound (T-18) (5.30 g, 0.02 mol) prepared by a publicly known method was added dropwise, and the resulting mixture was returned to room temperature while stirring. The resulting reaction mixture was poured into water, and an ordinary post-treatment was applied thereto, and the resulting material was purified by silica gel chromatography to obtain compound (T-19) (10.01 g, 0.018 mol; 92%).

### Second step:

Compound (T-19) (10.01 g, 0.019 mol) was dissolved in a mixture of toluene (500 mL) and 2-propanol (IPA; 100 mL), and Pd/C (0.98 g) was further added thereto, and the resulting mixture was stirred under a hydrogen atmosphere at room temperature until hydrogen was not absorbed. After Pd/C was removed therefrom, the resulting material was purified by silica gel chromatography and recrystallization to obtain compound (T-20) (8.55 g, 0.016 mol; 85%).

### Third step:

To a toluene (400 mL) solution of compound (T-20) (8.55 g, 0.016 mol) and tetrabutylammonium bromide (TBAB; 1.10 g, 3.41 mmol), 87% formic acid (40 mL) was added, and the resulting mixture was stirred for 17 hours under heating. An ordinary post-treatment was applied thereto to obtain compound (T-21) (7.02 g, 0.015 mol, cis / trans = 28 / 72; quantitative).

### Fourth step:

To compound (T-21) (7.02 g, 0.015 mol), toluene (200 mL), methanol (500 mL) and p-toluenesulfonic acid (PTSA; 0.93 g, 4.89 mmol) were added, and the resulting mixture was stirred under heating for 7 hours. An ordinary post-treatment was applied thereto, and the resulting material was purified by recrystallization to obtain compound (T-22) (7.04 g, 0.014 mol; 91%).

### Fifth step:

To a toluene (150 mL) solution of compound (T-22) (7.04 g, 0.014 mol) and tetrabutylammonium bromide (TBAB; 0.91 g, 2.82mmol), 87% formic acid (15 mL) was added, and the resulting mixture was stirred at room temperature for several hours. An ordinary post-treatment was applied thereto to obtain compound (T-23) (6.10 g, 13 mmol; 95%).

### Sixth step:

A THF (100 mL) solution of methyltriphenylphosphonium bromide (6.01 g, 16.82 mmol) was cooled to -70°C, and potassium t-butoxide (1.82 g, 16.22 mmol) was added dropwise thereto, and the resulting mixture was stirred for 1 hour. Thereto, a THF (50 mL) solution of compound (T-23) (6.10 g, 13.42 mmol) was added dropwise, and the resulting mixture was returned to room temperature while stirring. The resulting reaction mixture was poured into water, and an ordinary post-treatment was applied thereto, and the resulting material was purified by silica gel chromatography and recrystallization to obtain compound (No. 159) (4.72 g, 42 mmol: 78%).

¹H-NMR (CDCl_{3;} δ ppm): 7.42 - 7.40 (2 H, m), 7.26 - 7.23 (2H, m), 7.09 (1H, dt, 8.4 Hz, 2.0 Hz), 6.78 (1H, dt, 8.9 Hz, 1.9 Hz), 5.81 - 5.74 (1H, m), 4.95 (1H, d, 17.5 Hz), 4.87 (1H, d, 10.8 Hz), 4.15 (2H, q, 7.0 Hz), 2.67 - 2.63 (2H, m), 1.88 - 1.83 (3H, m), 1.78 - 1.72 (6H, m), 1.55 - 1.46 (5H, m), 1.28 - 1.22 (11H, m).

Phase transition temperature: C 104.2 S_{B} 112.6 S_{A} 135.1 N 262.1 I. Maximum temperature (T_{NI}) = 236.3°C; dielectric anisotropy (Δε) = -4.85; optical anisotropy (Δn) = 0.197.

### Comparative Example 1

### Comparison of physical properties

As a comparative compound, compound (S-1) described in the paragraph 0103 in JP 2002-193853 A was selected. The reason is that all bonding groups in compound (S-1) are a single bond, and compound (S-1) is different from a compound of the invention in the point. Compound (S-1) was prepared according to a publicly known method.

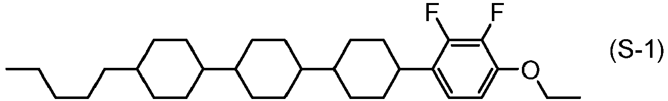

¹H-NMR (CDCl_{3;} δ ppm): 6.83 (1H, dt, J = 2.3 Hz, 8.9 Hz), 6.66 (1H, dt, J = 1.8 Hz, 9.1 Hz), 4.08 (2H, q, J = 7.0 Hz), 2.72 (1H, tt, J = 2.9 Hz, 12.1 Hz), 1.87 - 1.68 (12H, m), 1.44 - 1.37 (5H, m), 1.33 - 1.20 (6H, m), 1.17 - 1.07 (6H, m), 0.98 - 0.92 (9H, m), 0.89 - 0.80 (5H, m).

Physical properties of compound (S-1) were as described below. Phase transition temperature: S_{B} 164.8 N 169.1 I. Maximum temperature (T_{NI}) = 257.9°C; dielectric anisotropy (Δε) = -4.4; optical anisotropy (Δn) = 0.127; viscosity (η) = 52.9.

The results obtained by measuring compatibility between compound (No. 121), compound (No. 122) and compound (S-1) are summarized in Table 2. Samples for measurement were prepared according to the "extrapolation method" described above. Then, 15% by weight of compound (No. 121) and compound (No. 122) each was mixed with 85% by weight of liquid crystal (B), and dissolved therein by heating the mixture. The mixture was returned to room temperature, but no crystals precipitated. In contrast, when compound (S-1) was dissolved in liquid crystal (B) in an identical proportion and the resulting solution was returned to room temperature, crystals precipitated. A proportion of compound (S-1) was changed to 10% by weight or 5% by weight, but crystals precipitated. When a proportion was 1% by weight, no crystals precipitated. The results show that compound (No. 121) and compound (No. 122) are excellent in the compatibility. When dielectric anisotropy (Δε) of the sample was measured, compound (No. 121) and compound (No. 122) had a larger value. The results show that compound (No. 121) and compound (No. 122) are superior to comparative compound (S-1).

**Table 2. Characteristics of compounds**

| Compound | Structural formula | Proportion of compound | Dielectric anisotropy (Δε) |
|---|---|---|---|
| Compound (No. 121) | 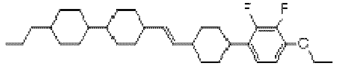 | 15% by weight / 85% by weight | -4.8 |
| Compound (No. 122) | 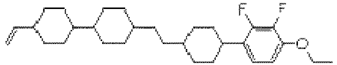 | 15% by weight / 85% by weight | -4.9 |
| Compound (S-1) | 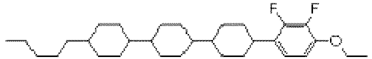 | 1% by weight / 99% by weight | -4.4 |

### Synthesis Example 4

### Synthesis of compound (No. 76)

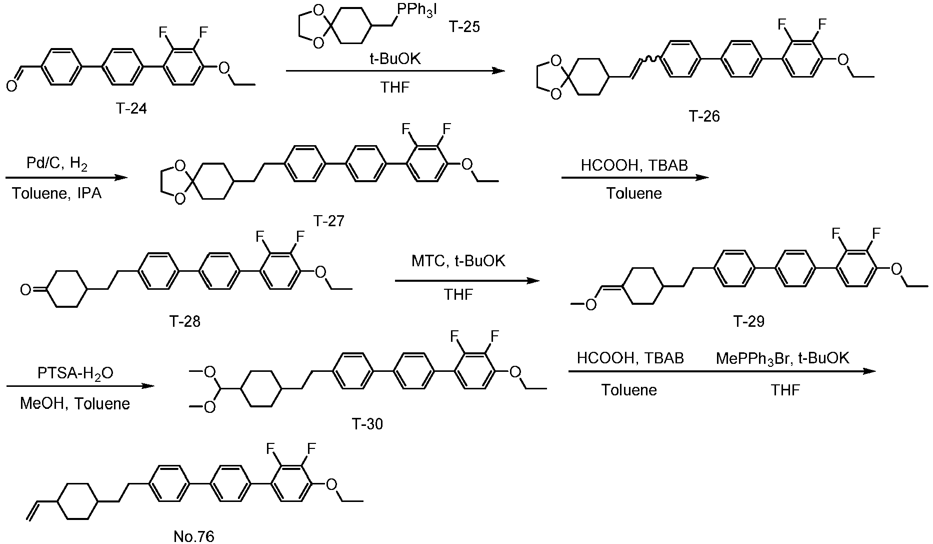

### First step:

A THF (150 mL) solution of compound (T-25) (23.27 g, 42.56 mmol) prepared by a publicly known method was cooled to -60°C, and potassium t-butoxide (4.78 g, 42.56mol) was added dropwise thereto, and the resulting mixture was stirred for 1 hour. Thereto, a THF (50 mL) solution of compound (T-24) (12 g, 35.47 mmol) prepared by a publicly known method is added dropwise, and the resulting mixture was returned to room temperature while stirring. The resulting reaction mixture was poured into water, and an ordinary post-treatment was applied thereto, and the resulting material was purified by silica gel chromatography to obtain compound (T-26) (16.84 g, 35.34 mmol; 100%) .

### Second step:

Compound (T-26) (16.9 g, 35.46 mmol) was dissolved in a mixture of toluene (150 mL) and 2-propanol (IPA; 150 mL), and Pd/C (0.507 g) was further added thereto, and the resulting mixture was stirred under a hydrogen atmosphere at room temperature until hydrogen was not absorbed. After Pd/C was removed, the resulting material was purified by silica gel chromatography and recrystallization to obtain compound (T-27) (16.84 g, 35.19 mmol; 99%).

### Third step:

To a toluene (160 mL) solution of compound (T-27) (16.84 g, 35.19 mmol), 87% formic acid (13.5 mL) was added, and the resulting mixture was stirred under heating for 8 hours. An ordinary post-treatment was applied thereto to obtain compound (T-28) (15.5 g, 35.67 mmol; quantitative).

### Fourth step:

A THF (150 mL) solution of (methoxymethyl)triphenyl phosphonium chloride (13.49 g, 39.49 mmol) was cooled to -60°C, and potassium t-butoxide (4.42 g, 39.36 mol) was added dropwise thereto, and the resulting mixture was stirred for 1 hour. A THF (50 mL) solution of compound (T-28) (15.55 g) was added dropwise thereto, and the resulting mixture was returned to room temperature while stirring. The resulting reaction mixture was poured into water, and an ordinary post-treatment was applied thereto, and the resulting material was purified by silica gel chromatography to obtain compound (T-29) (12 g, 25.94 mmol; 72%) .

### Fifth step:

To compound (T-29) (11.45 g, 24.75 mmol), toluene (300 mL), methanol (200 mL) and p-toluenesulfonic acid (PTSA; 1.41 g, 7.43 mmol) were added, and the resulting mixture was stirred under heating for 3 hours. An ordinary post-treatment was applied thereto to obtain compound (T-30) (7.97 g, 16.11 mmol; 65%).

### Sixth step:

To a toluene (70 mL) solution of compound (T-30) (7.97 g, 16.11 mmol) and tetrabutylammonium bromide (TBAB; 1.56 g, 4.83 mmol), 87% formic acid (12.36 mL) was added, and the resulting mixture was stirred at room temperature for several hours. An ordinary post-treatment was applied thereto. On the other hand, a THF (50 mL) solution of methyltriphenylphosphonium bromide (3.47 g, 10.47 mol) was cooled to -70°C, and potassium t-butoxide (1.21 g, 10.78 mmol) was added dropwise thereto, and the resulting mixture was stirred for 1 hour. Thereto, the solution in which the post-treatment was applied earlier was added dropwise, and the resulting mixture was returned to room temperature while stirring. The resulting reaction mixture was poured into water, and an ordinary post-treatment was applied thereto, and the resulting material was purified by silica gel chromatography and recrystallization to obtain compound (No. 76) (2.8 g, 6.27 mmol; 39%) .

¹H-NMR (CDCl_{3;} δ ppm) : 7.66 (2H, d, 8.4 Hz), 7.58 - 7.54 (4H, m), 7.27 (2H, d, 9.7 Hz), 7.14 (1H, dd, 8.5 Hz, 2.2 Hz), 6.81 (1H, dd, 7.4 Hz, 1.7 Hz), 5.82 - 5.75 (1H, m), 4.97 (1H, dt, 4.9 Hz, 1.4 Hz), 4.90 - 4.88 (1H, m), 4.17 (2H, q, 7Hz), 2.69 - 2.66 (2H, m), 1.95 - 1.77 (5H, m),1.59 - 1.48 (7H, m), 1.30 - 1.1.24 (1H, m), 1.15 - 0.97 (4H, m) .

Phase transition temperature: C 4.8 S_{C} 119.1 S_{A} 187.1 N 277.9 I. Maximum temperature (T_{NI}) = 250.3°C; dielectric anisotropy (Δε) =-5.16; optical anisotropy (Δn) = 0.287; viscosity (η) = 63.3.

### Comparative Example 2

### Comparison of physical properties

As a comparative compound, compound (S-2) described below was selected. The reason is that all bonding groups in compound (S-2) are a single bond, and compound (S-2) is different from a compound of the invention in the point. Compound (S-2) is included in the compound described in formula I in JP 2002-193853 A. Compound (S-2) was prepared by a publicly known method.

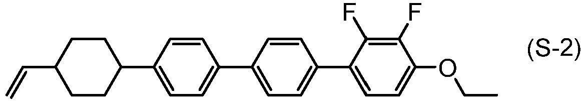

¹H-NMR (CDCl_{3;} δ ppm): 7.65 (2H, d, 8.4 Hz), 7.57 (4H, d, 8.2 Hz), 7.31 (2H, d, 8.2 Hz), 7.15 (1H, dt, 8.4 Hz, 2.4 Hz), 6.81 (1H, dt, 7.4 Hz, 1.8 Hz), 5.88 - 5.81 (1H, m), 5.03 (1H, dt, 17.4 Hz, 1.1 Hz), 4.94 (1H, dt, 12.8 Hz, 1.4 Hz), 4.17 (2H, q, 7.0 Hz), 2.54 (1H, tt, 12 Hz, 3.3 Hz), 2.07 - 1.91 (5H, m), 1.60 - 1.48 (5H, m), 1.30 (2H, dq, 12.9 Hz, 3.1 Hz).

Physical properties of compound (S-2) were as described below. Phase transition temperature: C 130 N 343.4 I. Maximum temperature (T_{NI}) = 266.6°C; dielectric anisotropy (Δε) = -6.03; optical anisotropy (Δn) = 0.287; viscosity (η) = 69.9.

The results obtained by measuring compatibility between compound (No. 76) and compound (S-2) are summarized in Table 3. Samples for measurement were prepared according to the "extrapolation method" described above. Then, 10% by weight of compound (No. 76) was mixed with 90% by weight of liquid crystal (B), and dissolved therein by heating the mixture. The mixture was returned to room temperature, but no crystals precipitated. In contrast, when compound (S-2) was dissolved in liquid crystal (B) in an identical proportion and the resulting solution was returned to room temperature, crystals precipitated. When a proportion of compound (S-1) was changed to 5% by weight, no crystals precipitated. The results show that compound (No. 76) is excellent in the compatibility. When viscosity (η) of the samples was measured, compound (No. 76) showed a smaller value. The results show that compound (No. 76) is superior to comparative compound (S-2) .

**Table 3. Characteristics of compounds**

| Compound | Structural formula | Proportion of compound | Viscosity (η) |
|---|---|---|---|
| Compound (No. 76) | 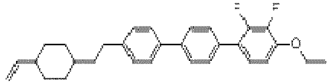 | 10% by weight / 90% by weight | 63.3 |
| Compound (S-2) | 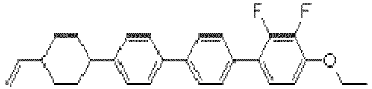 | 5% by weight / 95% by weight | 69.9 |

### Synthesis Example 5

### Synthesis of compound (No. 1)

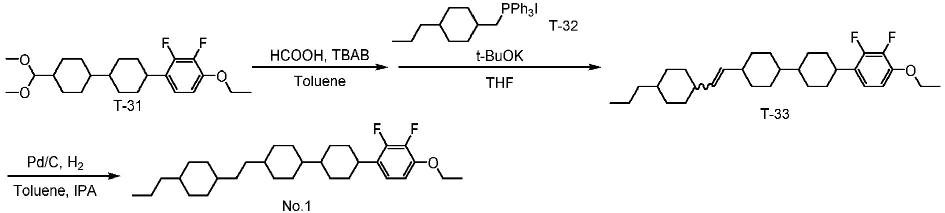

### First step:

To a toluene (50 mL) solution of compound (T-31) (5 g, 12.61 mmol) prepared by a publicly known method, 87% formic acid (9.7 mL) was added, and the resulting mixture was stirred at room temperature for several hours. An ordinary post-treatment was applied thereto. On the other hand, a THF (50 mL) solution of compound (T-32) (6.7 g, 12.61 mmol) prepared by a publicly known method was cooled to -30°C, and potassium t-butoxide (1.56 g, 13.87 mmol) was added dropwise thereto, and the resulting mixture was stirred for 1 hour. Thereto, the solution in which the post-treatment was applied earlier was added dropwise, and the resulting mixture was returned to room temperature while stirring. The resulting reaction mixture was poured into water, and an ordinary post-treatment was applied thereto, and the resulting material was purified by silica gel chromatography and recrystallization to obtain compound (T-33) (5 g, 10.58 mmol; 84%).

### Second step:

Compound (T-33) (5 g, 10.58 mmol) was dissolved in a mixture of toluene (50 mL) and 2-propanol (IPA; 50 mL), and Pd/C (0.15 g) was further added thereto, and the resulting mixture was stirred under a hydrogen atmosphere at room temperature until hydrogen was not absorbed. After Pd/C was removed, the resulting material was purified by silica gel chromatography and recrystallization to obtain compound (No. 1) (2.78 g, 5.86 mmol; 55%).

¹H-NMR (CDCl_{3;} δ ppm): 6.83 (1H, dt, 8.4 Hz, 2.3 Hz), 6.66 (1H, dt, 9.2 Hz, 1.9 Hz), 4.08 (2H, q, 7.0 Hz), 2.72 (1H, tt, 12.2 Hz, 3.2 Hz), 1.88 - 1.70 (12H, m), 1.44 - 1.26 (7H, m), 1.20 - 0.95 (15H, m), 0.91 - 0.82 (9H, m).

Phase transition temperature: C 21.8 S_{B} 283.81 N 292.4 I. Maximum temperature (T_{NI}) = 256.3°C; dielectric anisotropy (Δε) = -4.2; optical anisotropy (Δn) = 0.147; viscosity (η) = 49.5.

### Synthesis Example 6

### Synthesis of compound (No. 161) (not within the scope of the invention)

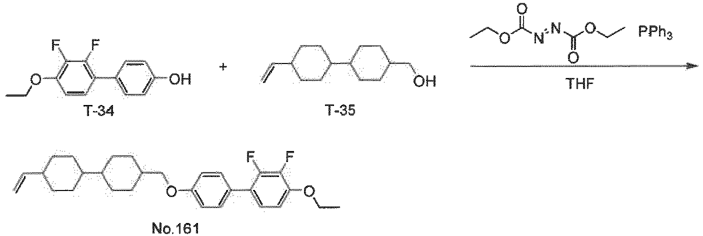

### First step:

To a THF (85 mL) solution of compound (T-34) (6.5 g, 25.97 mmol) and compound (T-35) (5.69 g, 25.59 mmol), triphenyl phosphine (7.38 g, 28.15 mmol) was added, and the resulting mixture was cooled to 0°C. Diethyl azodicarboxylate (12.79 mL, 28.15 mmol) was added dropwise thereto, and the resulting mixture was stirred for 2 hours. The resulting reaction mixture was poured into water, and an ordinary post-treatment was applied thereto, and the resulting material was purified by silica gel chromatography and recrystallization to obtain compound (No. 161) (3 g, 6.59 mmol; 26%).

¹H-NMR (CDCl_{3;} δ ppm): 7.41 (2H, dd, 8.7 Hz, 1.5 Hz), 7.05 (1H, dt, 8.4 Hz, 2.1 Hz), 6.96 - 6.94 (2H, m), 6.77 (1H, dt, 9.0 Hz, 1.6 Hz), 5.81 - 5.74 (1H, m), 4.98 - 4.87 (2H, m), 4.15 (2H, q, 7.0 Hz), 3.79 (2H, d, 6.4 Hz), 1.95 - 1.78 (10H, m), 1.47 (3H, t, 7.0 Hz), 1.12 - 1.05 (10H, m).

Phase transition temperature: C 141.1 N 260.4 I. Maximum temperature (T_{NI}) = 237.6°C; dielectric anisotropy (Δε) = -5.03; optical anisotropy (Δn) = 0.2203; viscosity (η) = 76.

### Synthesis Example 7

### Synthesis of compound (No. 452)

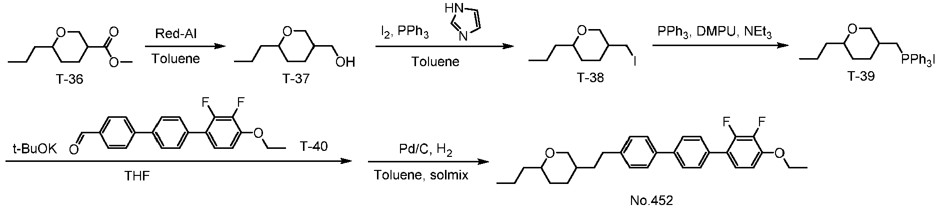

### First step:

A toluene (100 mL) solution of compound (T-36) (14.6 g, 78.7 mmol) was cooled to 0°C, and a 70% toluene solution of sodium bis(2-methoxyethoxy)aluminum hydride (Red-Al; 25 g, 86.56 mmol) was added dropwise thereto, and the resulting mixture was stirred for 1 hour. The reaction mixture was poured into 1 N hydrochloric acid solution, and an ordinary post-treatment was applied thereto, and the resulting material was purified by silica gel chromatography and recrystallization to obtain compound (T-37) (10 g, 63.13 mmol; 80%).

### Second step:

A toluene (30 mL) solution of compound (T-37) (3 g, 18.96 mmol), imidazole (1.68 g, 24.65 mmol) and triphenyl phosphine (6.46 g, 24.65 mmol) was ice-cooled, and a toluene (30 mL) solution of iodine (6.26 g, 24.65 mmol) was added dropwise thereto, and the resulting mixture was stirred at room temperature for several days. An ordinary post-treatment was applied thereto, and the resulting material was purified by silica gel chromatography and recrystallization to obtain compound (T-38) (3.85 g, 14.36 mmol; 70%).

### Third step:

To compound (T-38) (3.85 g, 14.36 mmol), triethylamine (0.15 g, 1.44 mmol), triphenylphosphine (3.95 g, 15.08 mol) and 1,3-dimethyl-3,4,5,6-tetrahydro-2(1H)-pyrimidinone (1.93 mL) were added, and the resulting mixture was stirred under heating for 7 days. An ordinary treatment was applied thereto to obtain compound (T-39) (3.15 g, 5.94 mmol; 41%).

### Fourth step:

A THF (20 mL) solution of compound (T-39) (3.15 g, 5.94 mmol) was cooled to -30°C, and potassium t-butoxide (0.67 g, 5.94 mmol) was added dropwise thereto, and the resulting mixture was stirred for 1 hour. Thereto, a THF (10 mL) solution of compound (T-40) (1.67 g, 4.95 mmol) prepared by a publicly known method was added dropwise, and the resulting mixture was returned to room temperature while stirring. The resulting reaction mixture was poured into water, and an ordinary post-treatment was applied thereto, and the resulting material was purified by silica gel chromatography and recrystallization. Thereto, toluene (7 mL) and THF (15 mL) were added, and Pd/C (0.13 g) was further added thereto, and the resulting mixture was stirred under a hydrogen atmosphere at room temperature until hydrogen was not absorbed. After Pd/C was removed therefrom, the resulting material was purified by silica gel chromatography and recrystallization to obtain compound (No. 452) (0.95 g, 2.04 mmol; 72%).

¹H-NMR (CDCl_{3;} δ ppm): 7.66 (2H, dd, 8.3 Hz, 1.8 Hz), 7.58 - 7.54 (4H, m), 7.26 - 7.25 (2H, m), 7.14 (1H, dt, 8.4 Hz, 2.2 Hz), 6.81 (1H, dt, 7.3 Hz, 1.7 Hz), 4.98 - 4.87 (2H, m), 4.01 - 3.98 (1H, m), 3.23 - 3.18 (1H, m), 3.08 (1H, t, 11.2 Hz), 2.71 - 2.60 (2H, m), 1.97 - 1.94 (1H, m), 1.66 - 1.59 (2H, m), 1.50 - 1.14 (1H, m), 0.91 (3H, t, 7.2 Hz) .

Phase transition temperature: C 113.5 N 221.3 I. Maximum temperature (T_{NI}) = 238.3°C; dielectric anisotropy (Δε) = -5.00; optical anisotropy (Δn) = 0.267; viscosity (η) = 83.3.

Compound (No. 45) was obtained in a manner similar to the synthesis method described in Synthesis Examples.

¹H-NMR (CDCl_{3;} δ ppm): 7.44 - 7.42 (2H, m), 7.28 - 7.27 (2H, m), 7.08 (1H, dt, 8.4 Hz, 2.3 Hz), 6.78 (1H, dt, 7.3 Hz, 1.8 Hz), 5.82 - 5.75 (1H, m), 4.98 - 4.94 (1H, m), 4.89 - 4.87 (1H, m), 4.15 (2H, q, 7.0 Hz), 2.50 (1H, tt, 12.1 Hz, 3.2 Hz), 1.94 - 1.87 (5H, m), 1.80 - 1.75 (4H, m), 1.52 - 1.44 (5H, m), 1.24 - 0.89 (12H, m).

Phase transition temperature: C 82 S_{B} 134.6 S_{A} 138.2 N 276.8 I. Maximum temperature (T_{NI}) = 241.6°C; dielectric anisotropy (Δε) = -4.9; optical anisotropy (Δn) = 0.187; viscosity (η) = 59.4.

Compound (No. 71) was obtained in a manner similar to the synthesis method described in Synthesis Examples.

¹H-NMR (CDCl_{3;} δ ppm): 7.66 - 7.53 (6H, m), 7.27 - 7.25 (2H, m), 7.14 (1H, dt, 8.4 Hz, 2.1 Hz), 6.80 (1H, dt, 7.5 Hz, 1.6 Hz), 4.16 (2H, q, 7.0 Hz), 2.68 - 2.65 (2H, m), 1.83 - 1.73 (4H, m), 1.56 - 1.47 (5H, m), 1.34 - 1.13 (6H, m), 0.99 - 0.94 (7H, m).

Phase transition temperature: C 100.2 S_{BA} 116.3 N 282.7 I. Maximum temperature (T_{NI}) = 257.3°C; dielectric anisotropy (Δε) = -4.9; optical anisotropy (Δn) = 0.277; viscosity (η) = 64.4.

Compound (No. 157) was obtained in a manner similar to the synthesis method described in Synthesis Examples.

¹H-NMR (CDCl_{3;} δ ppm): 7.40 (2H, d, 6.7 Hz), 7.24 (2H, d, 8.1 Hz), 7.08 (1H, dt, 8.4 Hz, 2.3 Hz), 6.78 (1H, dt, 7.3 Hz, 1.7 Hz), 4.16 (2H, q, 7.0 Hz), 2.67 - 2.63 (2H, m),1.85 - 1.69 (8H, m), 1.53 - 1.46 (5H, m), 1.24 - 1.16 (3H, m), 1.05 - 0.91 (9H, m), 0.89 - 0.80 (5H, m).

Phase transition temperature: C 85.8 Sc 135 S_{A} 156.2 N 245 I. Maximum temperature (T_{NI}) = 229°C; dielectric anisotropy (Δε) = -4.84; optical anisotropy (Δn) = 0.180; viscosity (η) = 66.6.

Compound (No. 160) was obtained in a manner similar to the synthesis method described in Synthesis Examples.

¹H-NMR (CDCl_{3;} δ ppm): 7.41 (2H, d, 7.8 Hz), 7.24 (2H, d, 8.1 Hz), 7.08 (1H, dt, 8.7 Hz, 2.1 Hz), 6.78 (1H, dt, 9.1 Hz, 1.6 Hz), 5.81 - 5.74 (1H, m), 4.97 - 4.93 (1H, m), 4.88 - 4.86 (1H, m), 4.08 (2H, t, 6.5 Hz), 2.67 - 2.64 (2H, m), 1.85 - 1.72 (10H, m), 1.56 - 1.49 (6H, m), 1.25 - 1.20 (1H, m), 1.10 - 0.90 (12H, m).

Phase transition temperature: C 75.6 Sc 112.6 S_{A} 181.9 N 250.7 I. Maximum temperature (T_{NI}) = 229.6°C; dielectric anisotropy (Δε) = -4.69; optical anisotropy (Δn) = 0.180; viscosity (η) = 51.4.

Compound (No. 163) was obtained in a manner similar to the synthesis method described in Synthesis Examples.

¹H-NMR (CDCl_{3;} δ ppm): 7.41 (2H, dd, 8.1 Hz), 7.26 - 7.23 (2H, m), 7.08 (1H, dt, 8.5 Hz, 2.4 Hz), 6.78 (1H, dt, 9.0 Hz, 1.7 Hz), 5.85 - 5.77 (1H, m), 5.01 - 4.91 (2H, m) and 4.15 (2H, q, 7.0 Hz), 2.66 - 2.63 (2H, m), 2.08 - 2.03 (2H, m), 1.85 - 1.69 (8H, m), 1.55 - 1.46 (5H, m), 1.28 - 1.13 (4H, m), 1.01 - 0.82 (10H, m).

Phase transition temperature: C 85.1 S_{A} 170.8 N 268.7 I. Maximum temperature (T_{NI}) = 250.3°C; dielectric anisotropy (Δε) = -4.43; optical anisotropy (Δn) = 0.187; viscosity (η) = 41.5.

Compound (No. 181) was obtained in a manner similar to the synthesis method described in Synthesis Examples.

¹H-NMR (CDCl_{3;} δ ppm): 7.43 (2H, dd, 7.1 Hz, 1.3 Hz), 7.30 - 7.26 (4H, m), 7.17 - 7.14 (2H, m), 7.09 (1H, dt, 8.5 Hz, 2.2 Hz), 6.79 (1H, dt, 7.4 Hz, 1.7 Hz), 4.16 (2H, q, 7.0 Hz), 2.97 - 2.90 (4H, m), 2.48 - 2.42 (1H, m), 1.91 - 1.84 (4H, m), 1.54 - 1.40 (5H, m), 1.37 - 1.19 (5H, m), 1.09 - 1.00 (2H, m), 0.90 (3H, t, 7.1 Hz).

Phase transition temperature: C 68.1 S_{B} 76.6 S_{A} 103 N 228 I. Maximum temperature (T_{NI}) = 220.3°C; dielectric anisotropy (Δε) = -4.9; optical anisotropy (Δn) = 0.217; viscosity (η) = 53.6.

Compound (No. 350) was obtained in a manner similar to the synthesis method described in Synthesis Examples.

¹H-NMR (CDCl_{3;} δ ppm): 7.51 - 7.49 (4H, m), 7.28 - 7.21 (4H, m), 6.75 (1H, dt, 8.0 Hz, 2.0 HZ), 6.62 (1H, dt, 8.8 Hz, 1.7 Hz), 4.08 (2H, q, 7.0 Hz), 2.91 (4H, s), 2.50 (1H, tt, 12.2 Hz, 3.2 Hz), 1.94 - 1.87 (4H, m), 1.51 - 1.42 (5H, m), 1.40 - 1.20 (5H, m), 1.10 - 1.02 (2H, m), 0.91 (3H, t, 7.4 Hz).

Phase transition temperature: C 72.1 S_{B} 119.3 N 247.3 I. Maximum temperature (T_{NI}) = 231.3°C; dielectric anisotropy (Δε) = -5.1; optical anisotropy (Δn) = 0.237; viscosity (η) = 51.5.

Compound (No. 453) was obtained in a manner similar to the synthesis method described in Synthesis Examples.

¹H-NMR (CDCl_{3;} δ ppm): 7.53 (2H, d, 8.1 Hz), 7.43 - 7.38 (3H, m), 7.27 (2H, d, 8.1 Hz), 7.09 - 7.01 (2H, m), 2.71 - 2.65 (4H, m), 1.83 - 1.68 (6H, m), 1.57 - 1.52 (3H, m), 1.34 - 1.29 (6H, m), 1.02 - 0.86 (9H, m).

Phase transition temperature: C 84.9.1 S_{A} 150.8 N 220.7 I. Maximum temperature (T_{NI}) = 193.6°C; dielectric anisotropy (Δε) = -2.2; optical anisotropy (Δn) = 0.227; viscosity (η) = 80.0.

Compounds (No. 1) to (No. 454) described below are synthesized in a manner similar to the method described in Synthesis Examples. Compounds (No. 2), (No. 3), (No. 7), (No. 8), (No. 12), (No. 13), (No. 17), (No. 18), (No. 21), (No. 22), (No. 25), (No. 28), (No. 31), (No. 34), (No. 37), (No. 38), (No. 42), (No. 46) to (No. 48), (No. 52), (No. 53), (No. 56), (No. 57), (No. 60), (No. 63), (No. 69), (No. 73), (No. 75), (No. 78), (No. 79), (No. 81), (No. 84), (No. 85), (No. 88), (No. 92), (No. 93), (No. 95), (No. 98), (No. 99), (No. 103), (No. 105) to (No. 107), (No. 110), (No. 114), (No. 116), (No. 118), (No. 119), (No. 124) to (No. 126), (No. 130) to (No. 132), (No. 137) to (No. 139), (No. 143), (No. 145), (No. 149), (No. 151), (No. 155), (No. 156), (No. 161), (No. 162), (No. 165), (No. 166), (No. 170), (No. 171), (No. 173), (No. 177), (No. 179), (No. 180), (No. 184), (No. 185), (No. 188), (No. 189), (No. 194), (No. 198), (No. 210), (No. 211), (No. 214), (No. 215), (No. 220), (No. 224), (No. 237) to (No. 240), (No. 242), (No. 245), (No. 249), (No. 250), (No. 253), (No. 254), (No. 257), (No. 263), (No. 264), (No. 267), (No. 268), (No. 272), (No. 281), (No. 282), (No. 285), (No. 289), (No. 291), (No. 294), (No. 299), (No. 304), (No. 309), (No. 314), (No. 319), (No. 323), (No. 327), (No. 331), (No. 335), (No. 340), (No. 344), (No. 352), (No. 356), (No. 361), (No. 364), (No. 369), (No. 374), (No. 379), (No. 384), (No. 388), (No. 391), (No. 394), (No. 398), (No. 403), (No. 408), (No. 413), (No. 416), (No. 421), (No. 422), (No. 426), (No. 431), (No. 433), (No. 436), (No. 441), (No. 442), (No. 446), (No. 448) and (No. 451) do not fall within the scope of the invention.

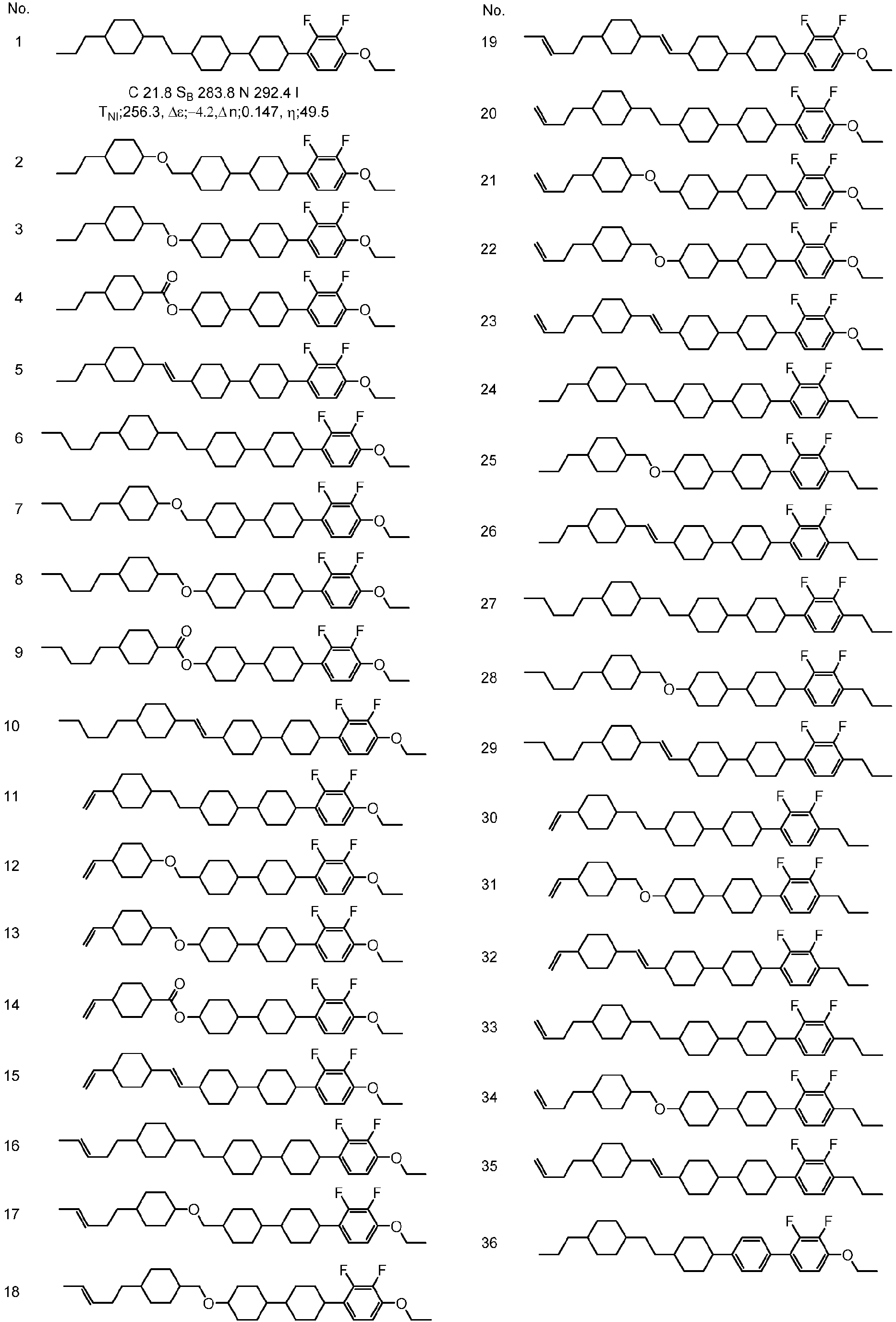

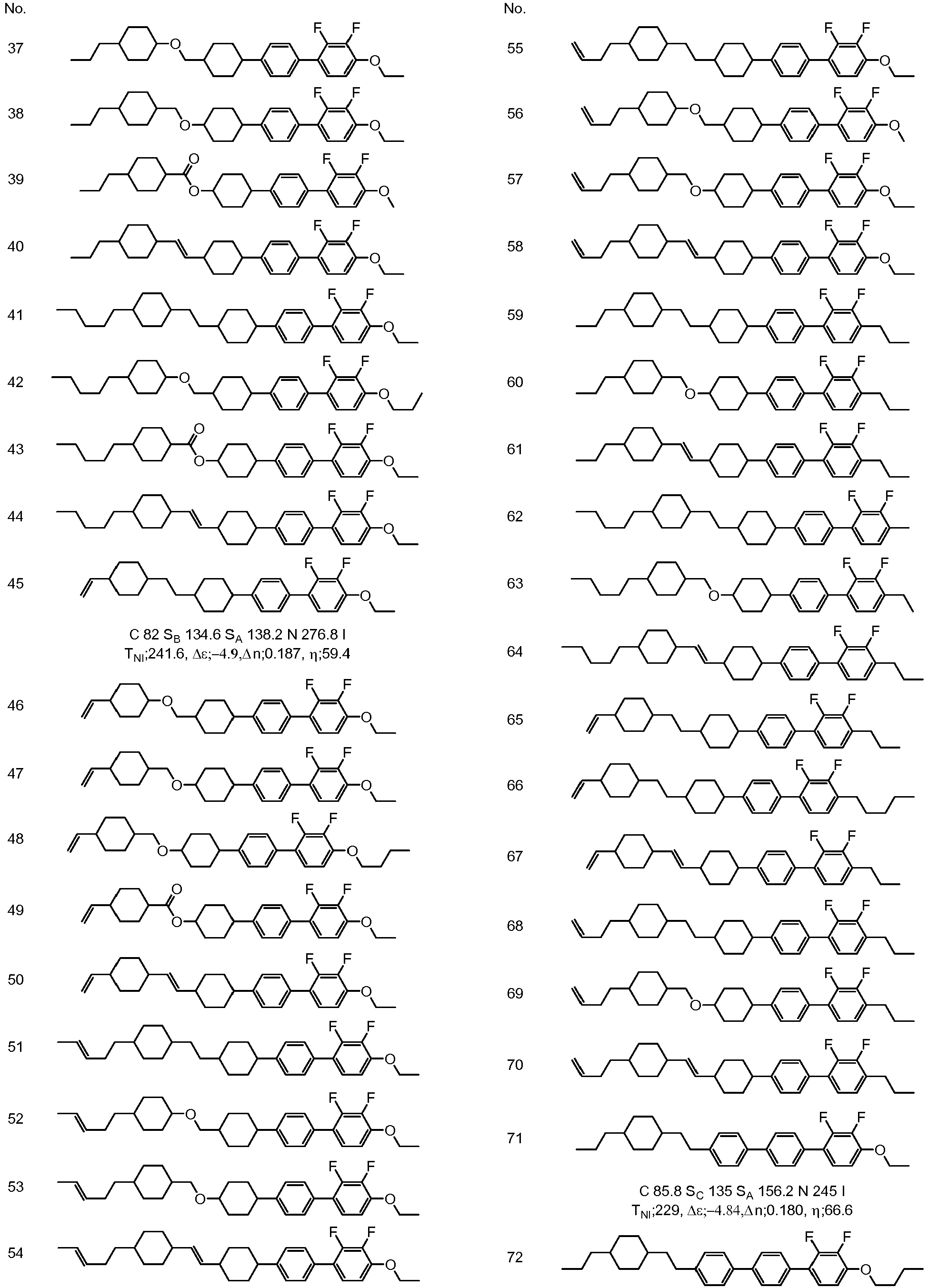

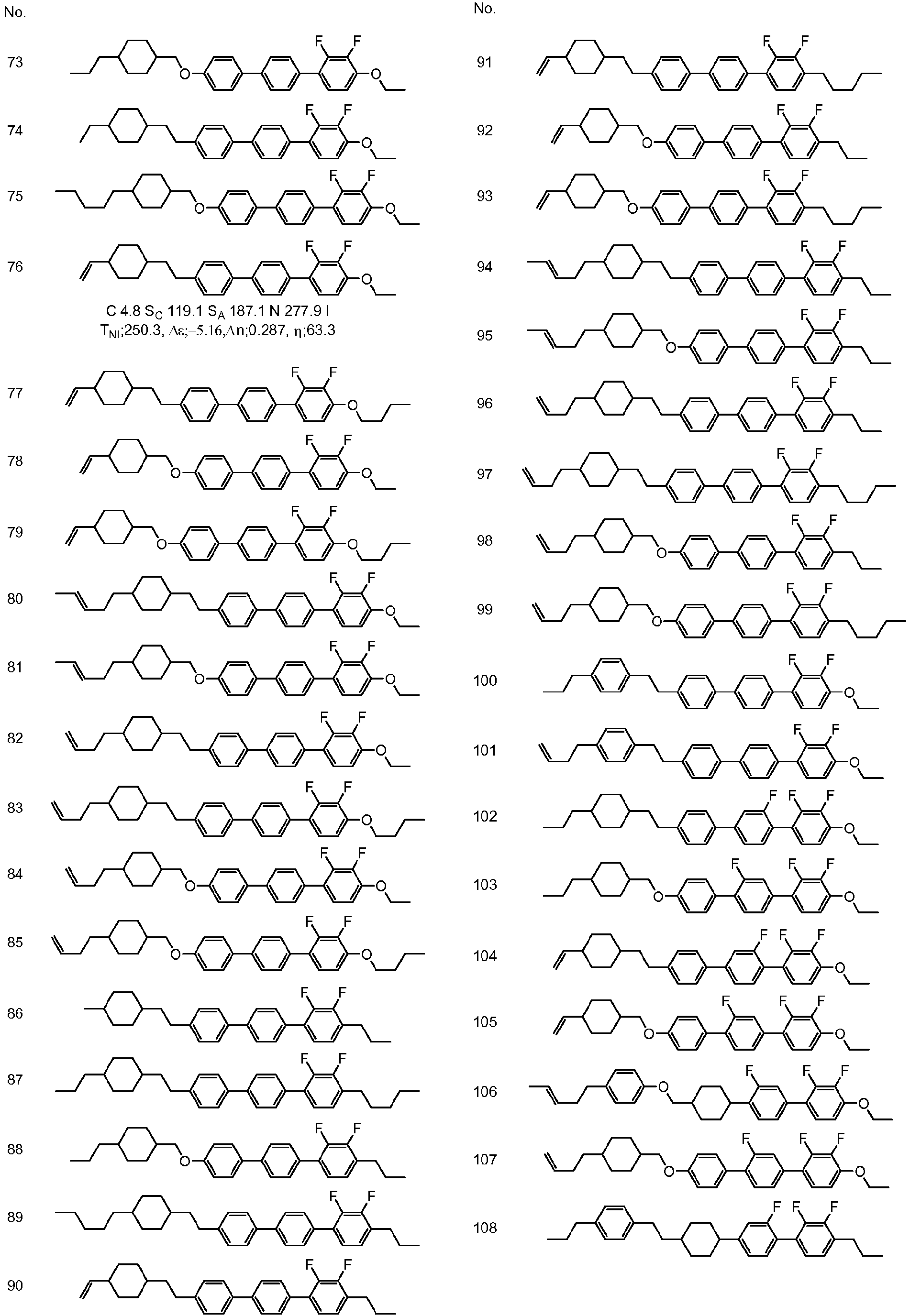

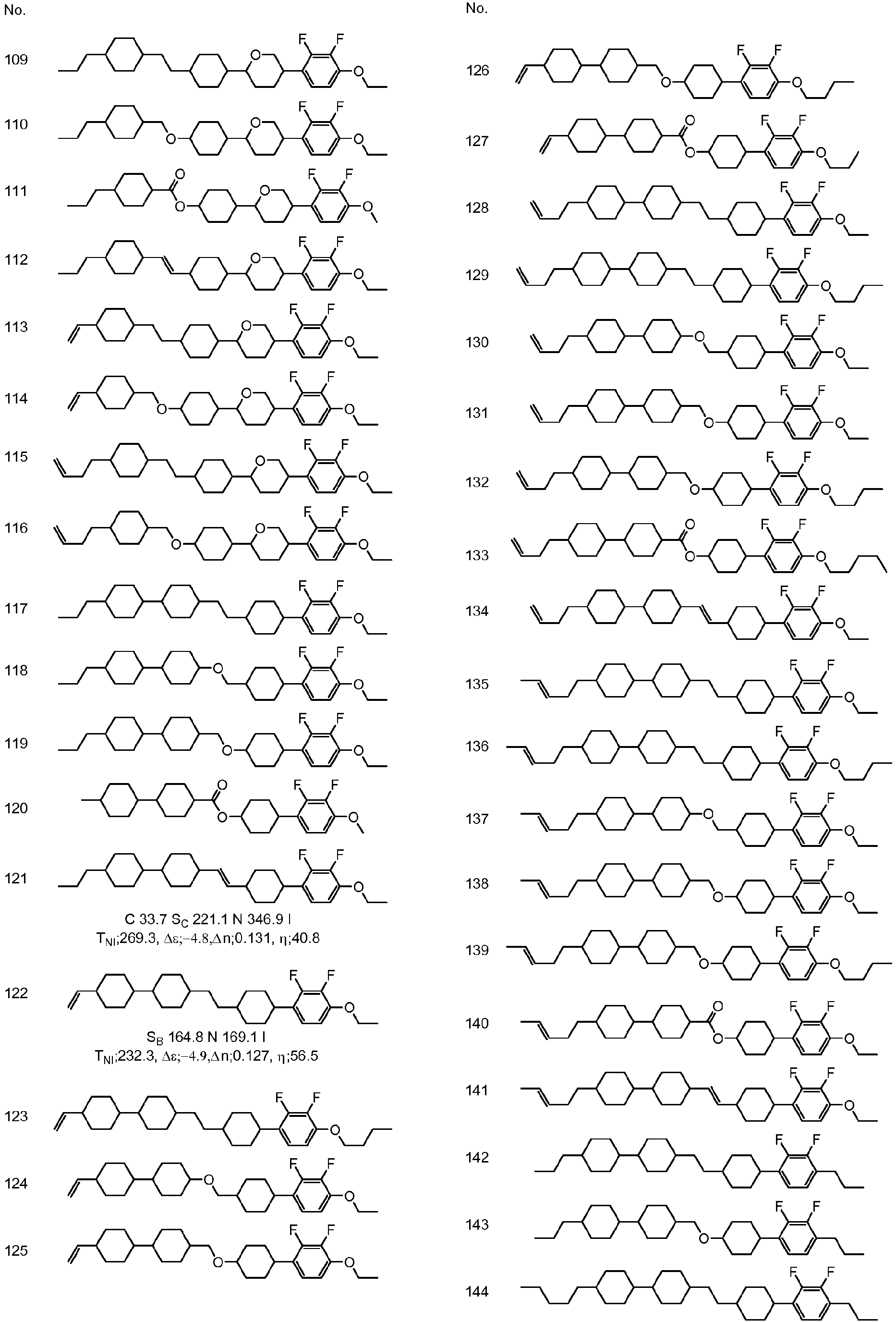

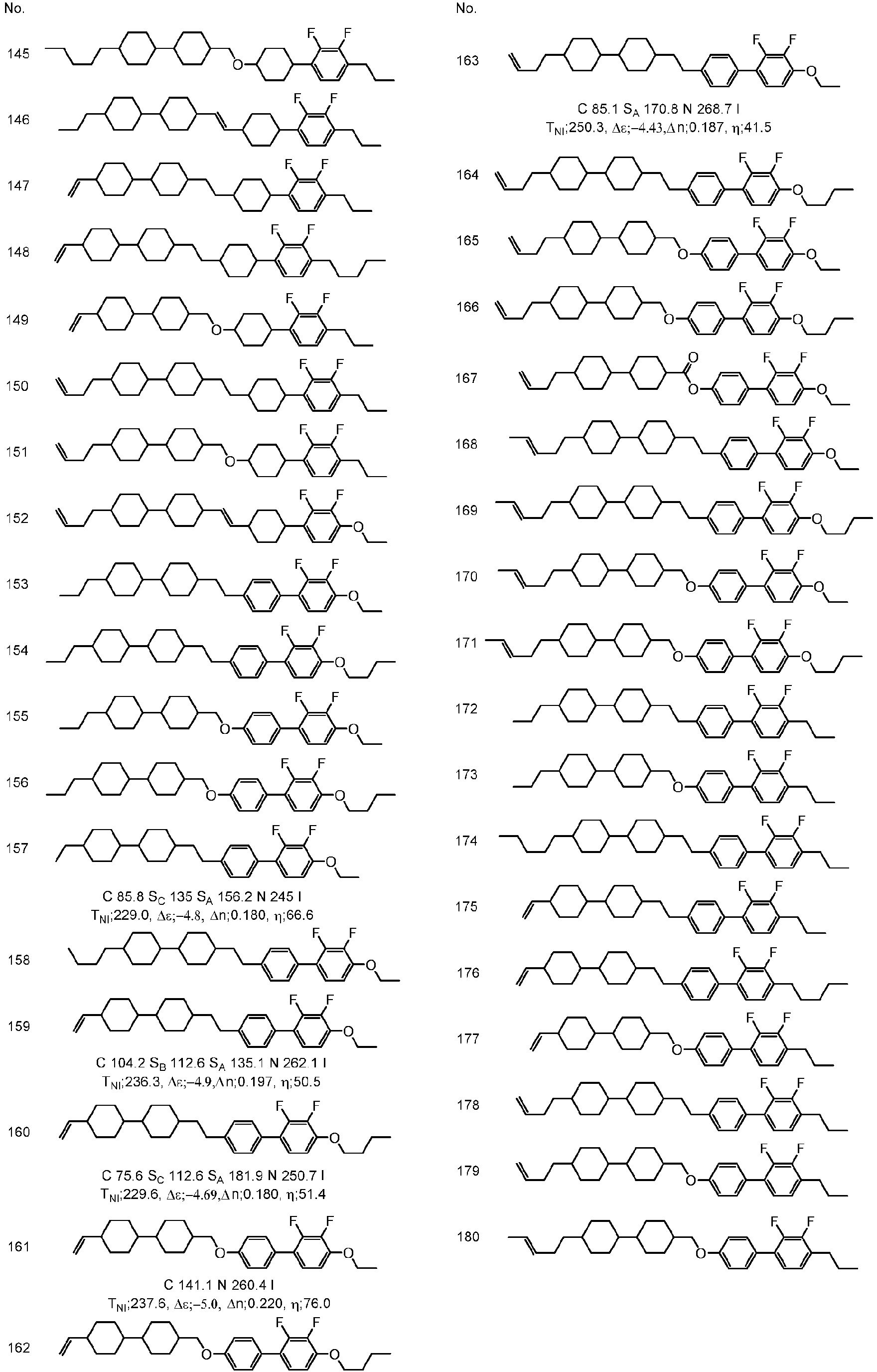

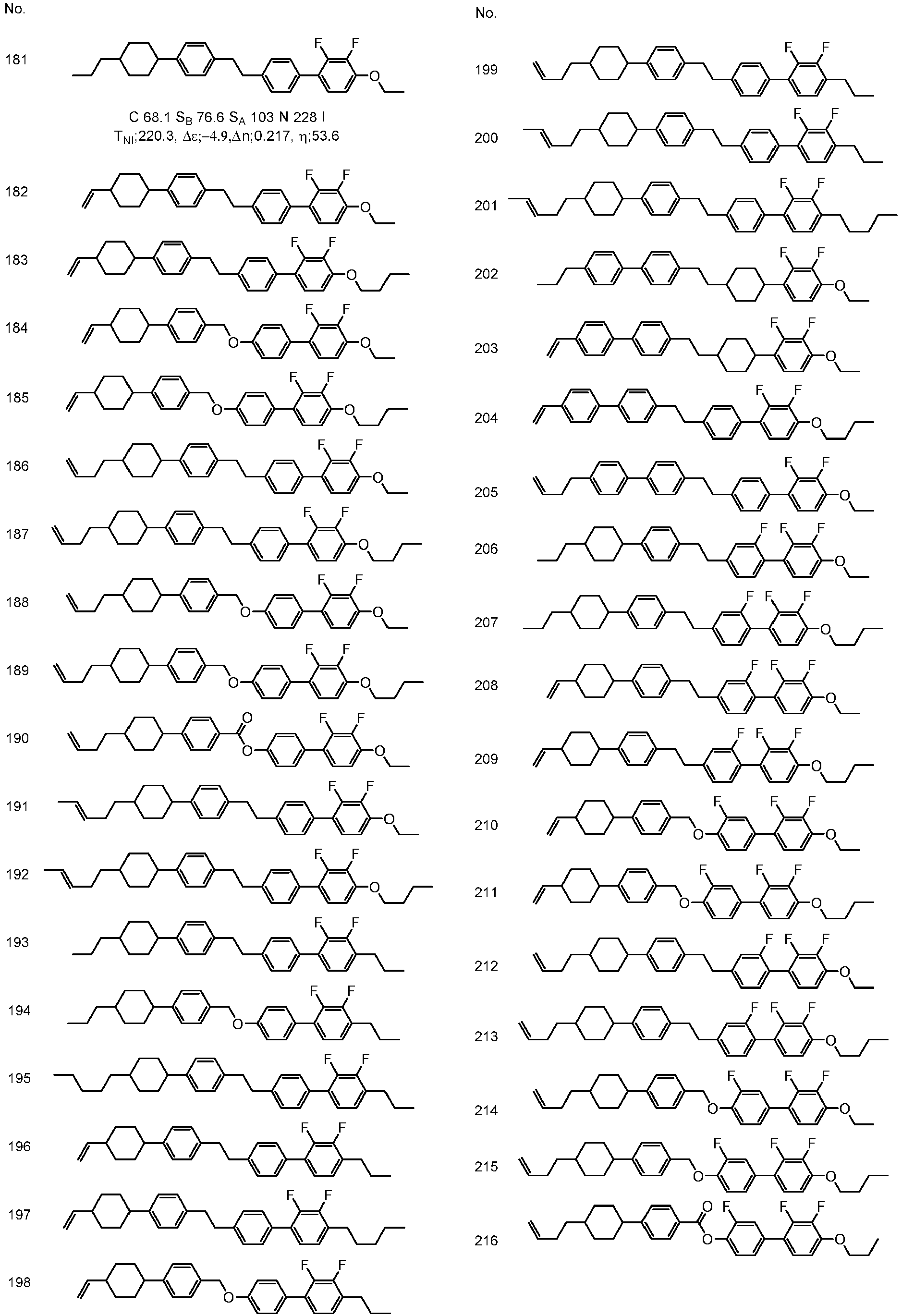

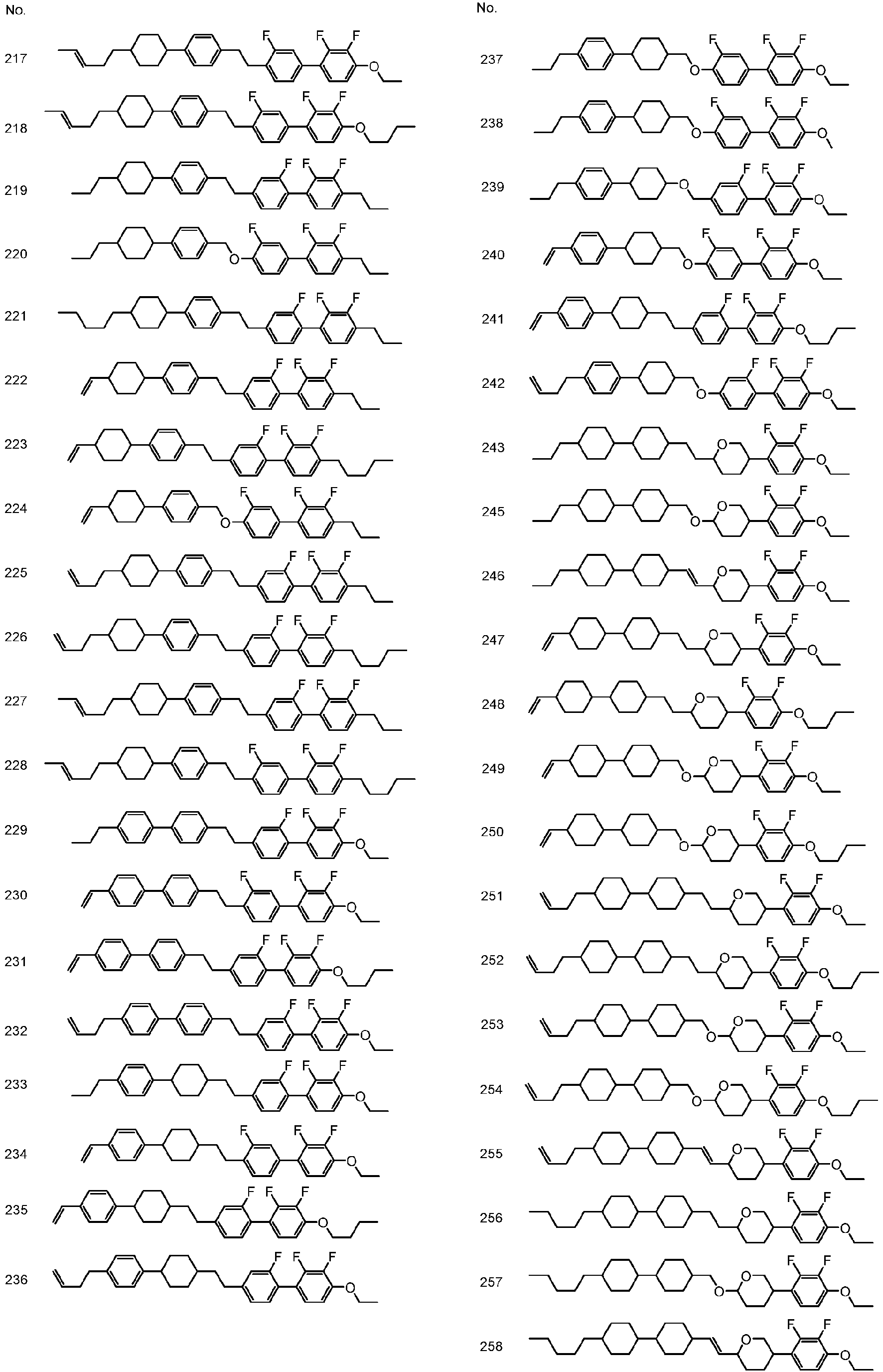

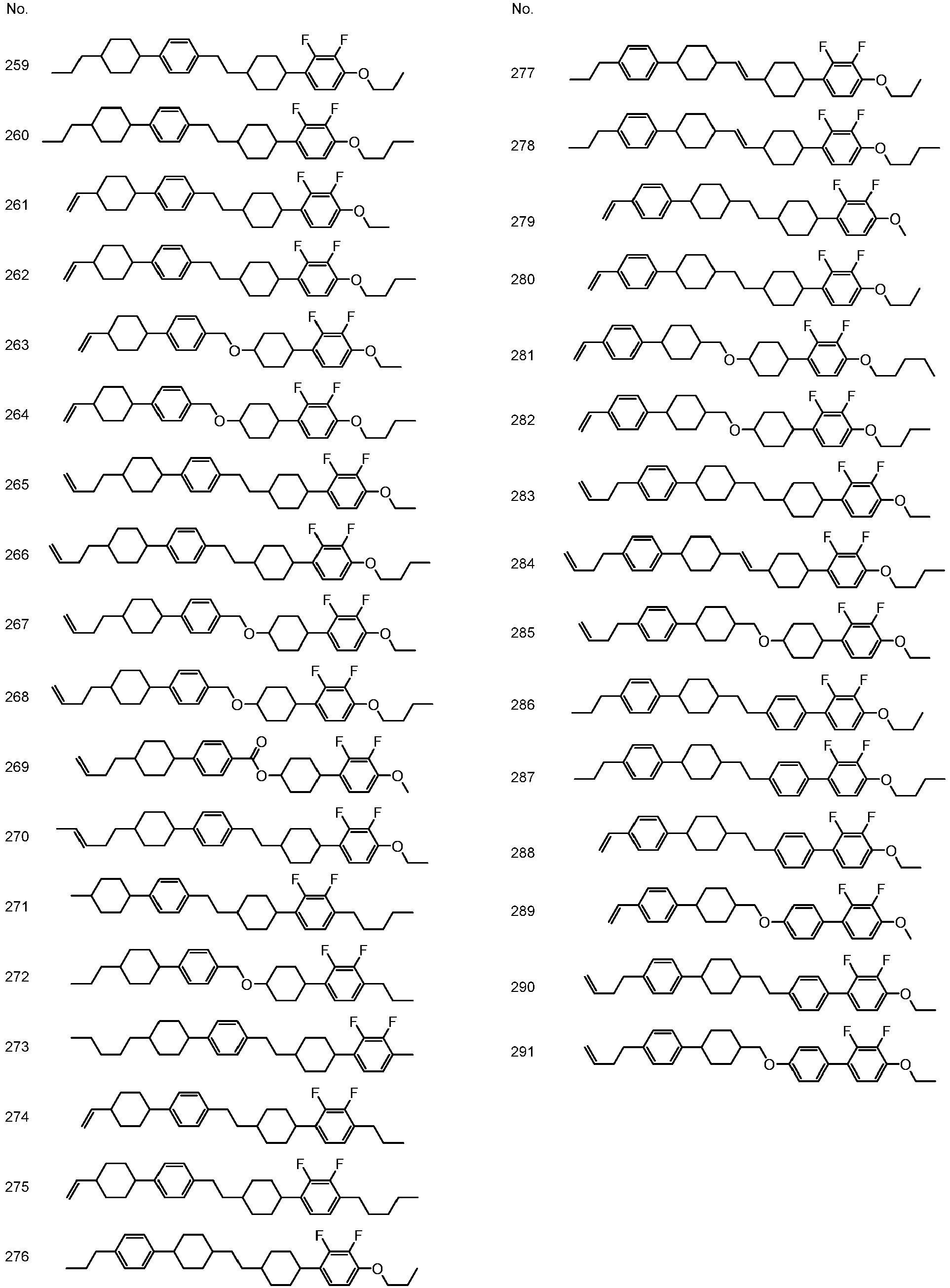

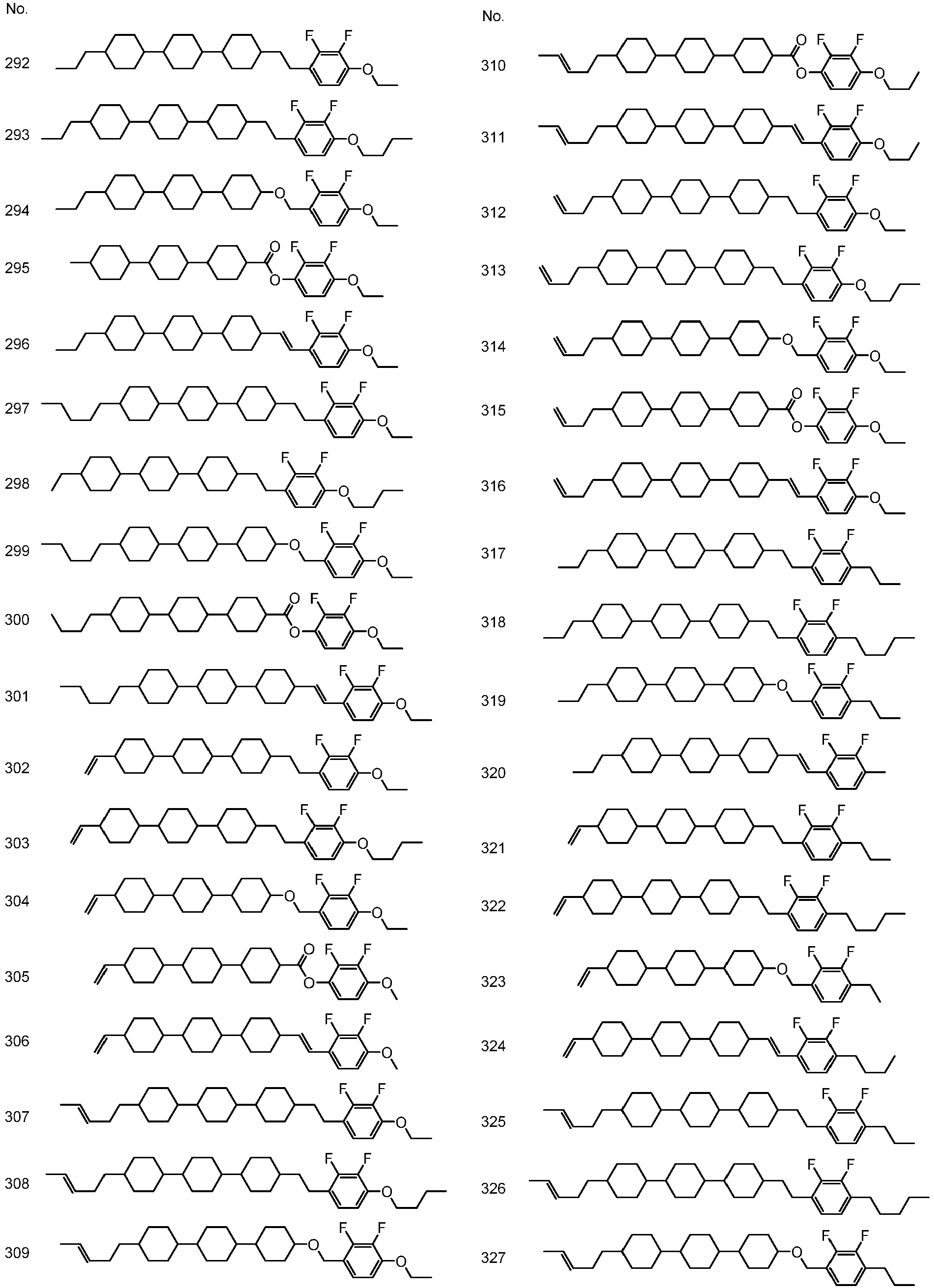

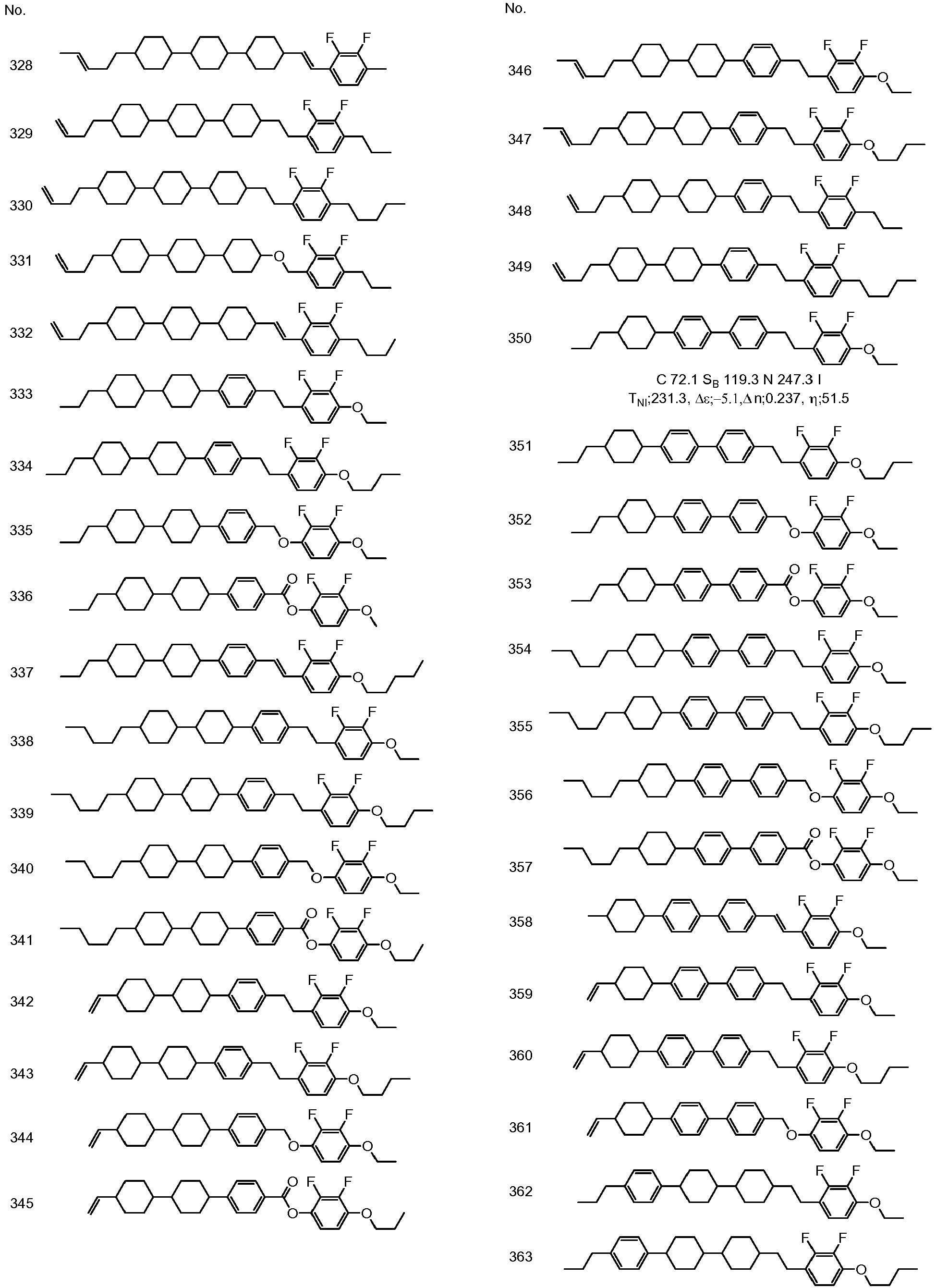

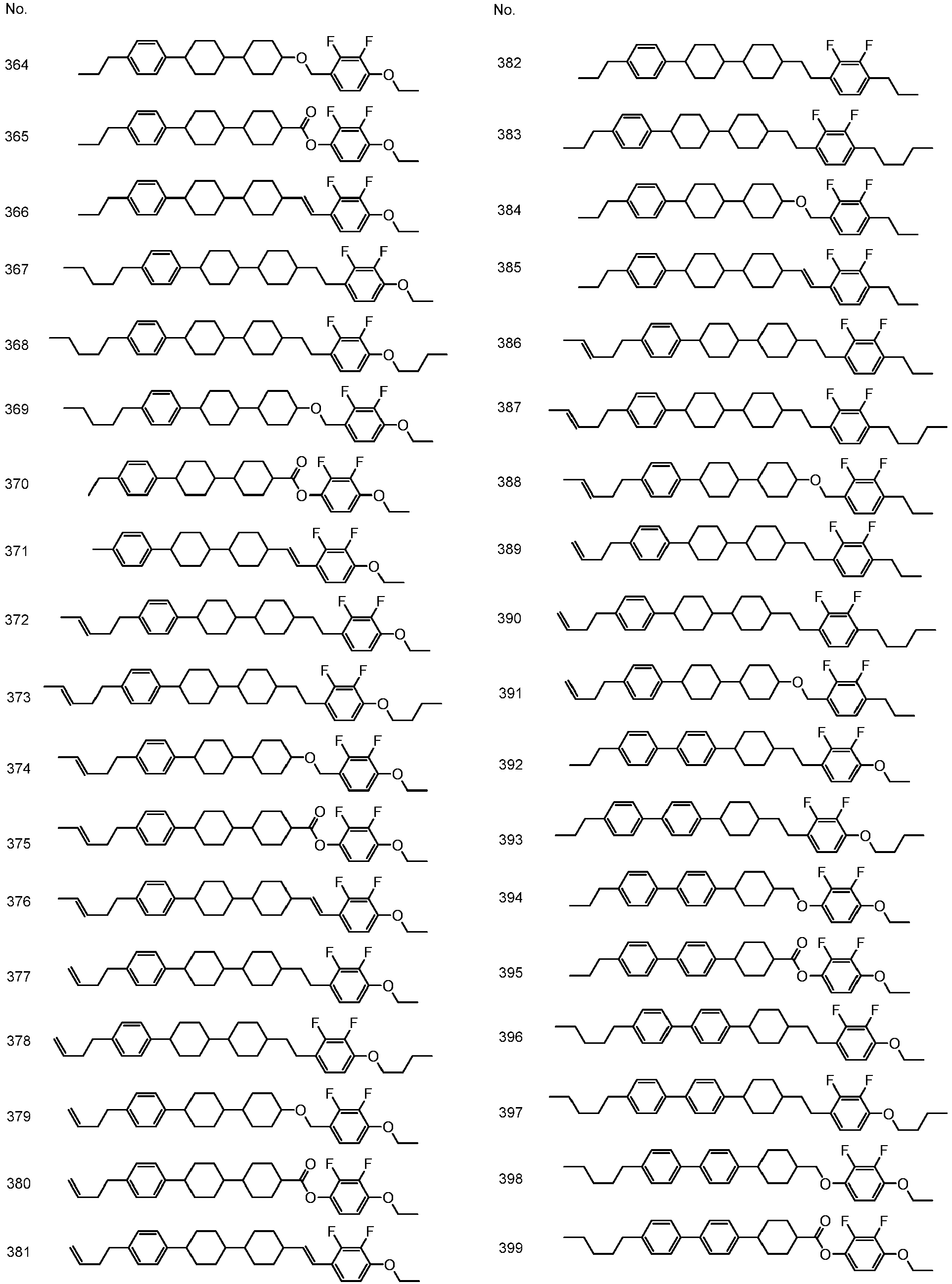

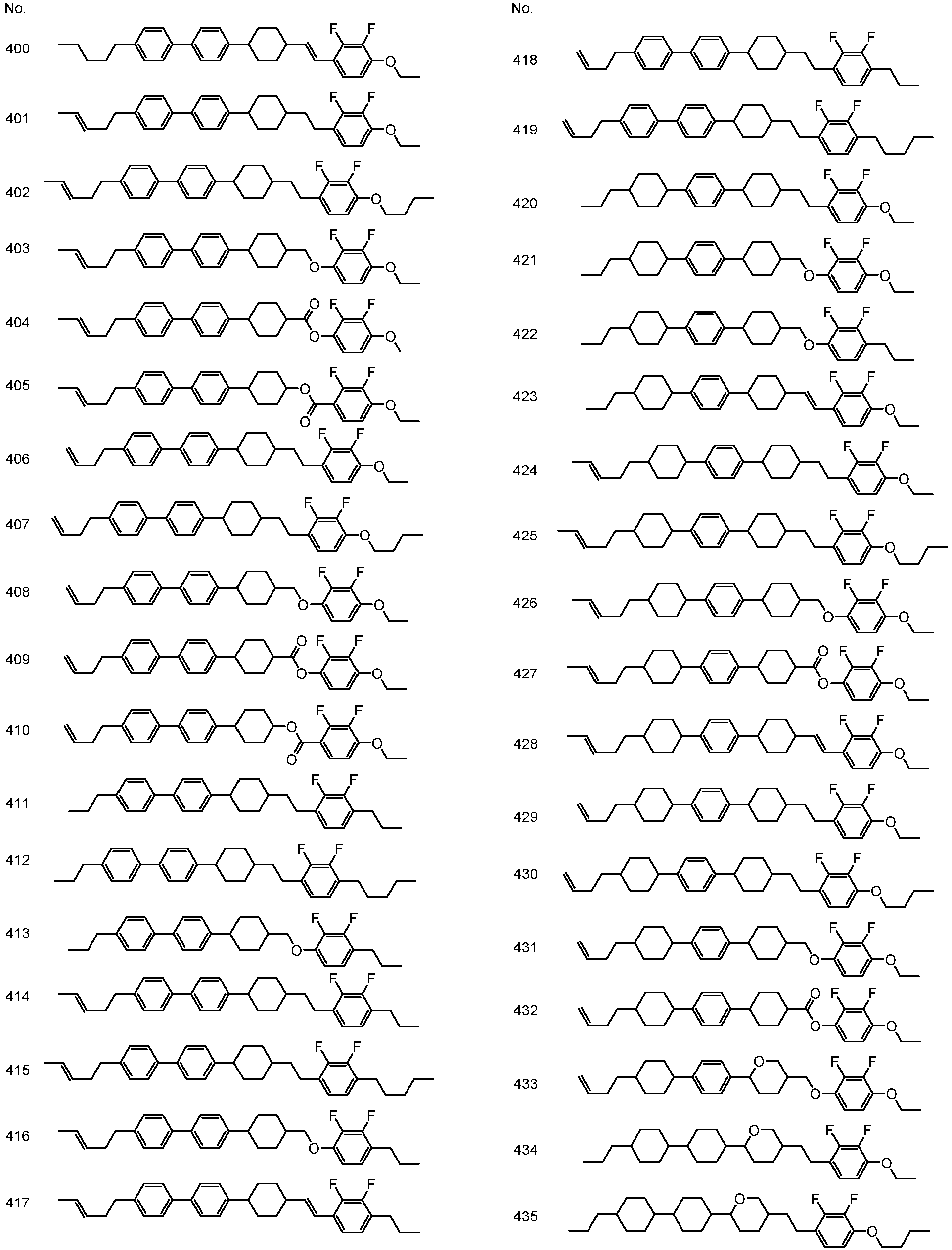

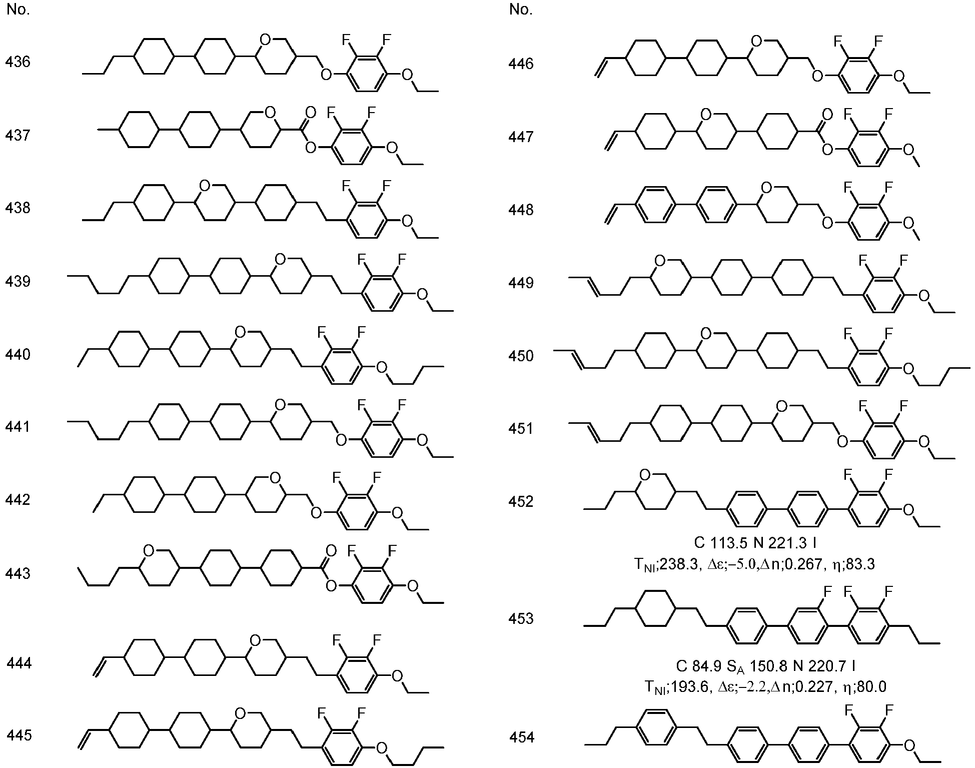

The invention will be described in greater detail by way of Examples . The Examples each is a typical example, and therefore the invention is not limited by the Examples. For example, in addition to compositions in Use Examples, the invention includes a mixture of a composition in Use Example 1 and a composition in Use Example 2. The invention also includes a mixture prepared by mixing at least two of the compositions in Use Examples. Compounds in Use Examples were represented using symbols according to definitions in Table 2 described below. In Table 2, the configuration of 1,4-cyclohexylene is trans. A parenthesized number next to a symbolized compound represents a chemical formula to which the compound belongs. A symbol (-) means a liquid crystal compound different from compounds (1) to (15). A proportion (percentage) of the liquid crystal compound is expressed in terms of weight percent (% by weight) based on the weight of the liquid crystal composition containing no additives. Values of physical properties of the composition are summarized in a last part. The physical properties were measured according to the methods described above, and measured values are directly described (without extrapolation).

**Table 1 Method for description of compounds using symbols**

| R-(A₁)-Z₁-·····-Zₙ-(Aₙ)-R' | | | |
|---|---|---|---|
| 1) Left-terminal group R- | Symbol | 4) Ring structure -Aₙ- | Symbol |
| FCₙH₂ₙ- | Fn- | 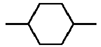 | H |
| CₙH₂ₙ₊₁- | n- | | |
| CₙH₂ₙ₊₁O- | nO- | 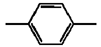 | B |
| CₘH₂ₘ₊₁OCₙH₂ₙ- | mOn- | | |
| CH₂=CH- | V- | | |
| CₙH₂ₙ₊₁-CH-CH- | nV- | 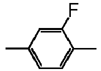 | B(F) |
| CH₂=CH-CₙH₂ₙ- | Vn- | | |
| CₘH₂ₘ₊₁-CH=CH-CₙH₂ₙ- | mVn- | | |
| CF₂=CH- | VFF- | 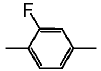 | B(2F) |
| CF₂=CH-CₙH₂ₙ- | VFFn- | | |
| 2) Right-terminal group -R' | Symbol | | |
| -CₙH₂ₙ₊₁ | -n | 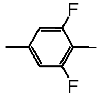 | B(F,F) |
| -OCₙH₂ₙ₊₁ | -On | | |
| -COOCH₃ | -EMe | | |
| -CH=CH₂ | -V | 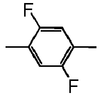 | B(2F,5F) |
| -CH=CH-CₙH₂ₙ₊₁ | -Vn | | |
| -CₙH₂ₙ-CH=CH₂ | -nV | | |
| ⁻CₘH₂ₘ-CH=CH-CₙH₂ₙ₊₁ | -mVn | 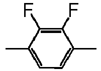 | B(2F,3F) |
| -CH=CF₂ | -VFF | | |
| -F | -F | | |
| -Cl | -CL | 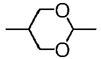 | G |
| -OCF₃ | -OCF3 | | |
| -OCF₂H | -OCF2H | | |
| -CF₃ | -CF3 | 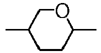 | dh |
| -C≡N | -C | | |
| 3) Bonding group -Zₙ- | Symbol | 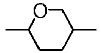 | Dh |
| -CₙH₂ₙ- | n | | |
| -COO- | E | 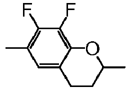 | Cro(7F,8F) |
| -CH=CH- | V | | |
| -CH₂O- | 1O | | |
| -OCH₂- | O1 | 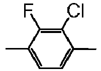 | B(2F,3CL) |
| -CF₂O- | X | | |
| -C≡C- | T | | |
| | | | |

| 5) Examples of description | | | |
|---|---|---|---|
| Example 1 V-HH2HB(2F,3F)-O2 | | Example 2 3-HBB(F,F)-F | |
| 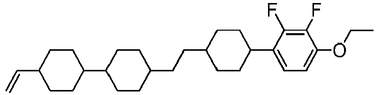 | | 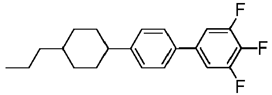 | |

### Use Example 1

| | | |
|---|---|---|
| V-HH2HB (2F,3F)-O2 | (No.122) | 2 % |
| V-HH2BB (2F,3F)-O2 | (No.159) | 3 % |
| 2-HB-C | (15-1) | 5 % |
| 3-HB-C | (15-1) | 10% |
| 3-HB-O2 | (2-5) | 15% |
| 2-BTB-1 | (2-10) | 3 % |
| 3-HHB-F | (13-1) | 4 % |
| 3-HHB-1 | (3-1) | 7 % |
| 3-HHB-O1 | (3-1) | 5 % |
| 3-HHB-3 | (3-1) | 12% |
| 3-HHEB-F | (13-10) | 4% |
| 5-HHEB-F | (13-10) | 4% |
| 2-HHB(F)-F | (13-2) | 7% |
| 3-HHB(F)-F | (13-2) | 7% |
| 5-HHB(F)-F | (13-2) | 7% |
| 3-HHB(F,F)-F | (13-3) | 5% |

NI = 106.9°C; η = 19.6 mPa·s; Δn = 0.103; Δε = 4.1.

### Use Example 2

| | | |
|---|---|---|
| V-HH2BB(2F,3F)-O4 | (No.160) | 3% |
| V2-HH2BB(2F,3F)-O2 | (No.163) | 3% |
| 3-HB-CL | (12-2) | 10% |
| 3-HH-4 | (2-1) | 12% |
| 3-HB-O2 | (2-5) | 8 % |
| 3-HHB(F,F)-F | (13-3) | 3% |
| 3-HBB(F,F)-F | (13-24) | 28% |
| 5-HBB(F,F)-F | (13-24) | 24% |
| 5-HBB(F)B-2 | (4-5) | 4% |
| 5-HBB(F)B-3 | (4-5) | 5 % |

### Use Example 3

| | | |
|---|---|---|
| V-H2BBB(2F,3F)-O2 | (No.76) | 2 % |
| V-H2HBB(2F,3F)-O2 | (No.45) | 3 % |
| 7-HB(F,F)-F | (12-4) | 3 % |
| 3-HB-O2 | (2-5) | 7 % |
| 2-HHB(F)-F | (13-2) | 8 % |
| 3-HHB(F)-F | (13-2) | 9 % |
| 5-HHB(F)-F | (13-2) | 10% |
| 2-HBB(F)-F | (13-23) | 9 % |
| 3-HBB(F)-F | (13-23) | 9 % |
| 5-HBB(F)-F | (13-23) | 14% |
| 2-HBB-F | (13-22) | 4 % |
| 3-HBB-F | (13-22) | 4 % |
| 5-HBB-F | (13-22) | 3 % |
| 3-HBB(F,F)-F | (13-24) | 5 % |
| 5-HBB(F,F)-F | (13-24) | 10% |

### Use Example 4

| | | |
|---|---|---|
| 4-HH2BB(2F,3F)-O2 | (No.158) | 3 % |
| V-HH1OBB(2F,3F)-O2 | (No.161) | 5 % |
| 5-HB-CL | (15-2) | 14% |
| 3-HH-4 | (2-1) | 10% |
| 3-HH-5 | (2-1) | 4% |
| 3-HHB-F | (13-1) | 3% |
| 3-HHB-CL | (13-1) | 3% |
| 4-HHB-CL | (13-1) | 3% |
| 3-HHB(F)-F | (13-2) | 10% |
| 4-HHB(F)-F | (13-2) | 9% |
| 5-HHB(F)-F | (13-2) | 8% |
| 7-HHB(F)-F | (13-2) | 8% |
| 5-HBB(F)-F | (13-23) | 3% |
| 1O1-HBBH-5 | (4-1) | 3% |
| 3-HHBB(F,F)-F | (14-6) | 2% |
| 4-HHBB(F,F)-F | (14-6) | 3 % |
| 5-HHBB(F,F)-F | (14-6) | 3% |
| 3-HH2BB(F,F)-F | (14-15) | 3% |
| 4-HH2BB(F,F)-F | (14-15) | 3% |

### Use Example 5

| | | |
|---|---|---|
| V-HH1OBB(2F,3F)-3 | (No.161) | 3% |
| 3-HHVHB(2F,3F)-O2 | (No.121) | 3% |
| 3-HHB(F,F)-F | (13-3) | 8% |
| 3-H2HB(F,F)-F | (13-15) | 8% |
| 4-H2HB(F,F)-F | (13-15) | 8% |
| 5-H2HB(F,F)-F | (13-15) | 7% |
| 3-HBB(F,F)-F | (13-24) | 20% |
| 5-HBB(F,F)-F | (13-24) | 18% |
| 3-H2BB(F,F)-F | (13-27) | 10% |
| 5-HHBB(F,F)-F | (14-6) | 3% |
| 5-HHEBB-F | (14-17) | 2% |
| 3-HH2BB(F,F)-F | (14-15) | 3% |
| 1O1-HBBH-4 | (4-1) | 4% |
| 1O1-HBBH-5 | (4-1) | 3% |

### Use Example 6

| | | |
|---|---|---|
| 3-BB2HB(2F,3F)-O2 | (No.202) | 3 % |
| V-HVHBB(2F,3F)-3 | (No.67) | 3% |
| 5-HB-F | (12-2) | 10% |
| 6-HB-F | (12-2) | 9 % |
| 7-HB-F | (12-2) | 6% |
| 2-HHB-OCF3 | (13-1) | 6% |
| 3-HHB-OCF3 | (13-1) | 7% |
| 4-HHB-OCF3 | (13-1) | 6% |
| 5-HHB-OCF3 | (13-1) | 5% |
| 3-HH2B-OCF3 | (13-4) | 4% |
| 5-HH2B-OCF3 | (13-4) | 4% |
| 3-HHB(F,F)-OCF2H | (13-3) | 4% |
| 3-HHB(F,F)-OCF3 | (13-3) | 5% |
| 3-HH2B(F)-F | (13-5) | 3% |
| 3-HBB(F)-F | (13-23) | 9% |
| 5-HBB(F)-F | (13-23) | 10% |
| 5-HBBH-3 | (4-1) | 3% |
| 3-HB(F)BH-3 | (4-2) | 3% |

### Use Example 7

| | | |
|---|---|---|
| 3-HBH10B(2F,3F)-3 | (No.422) | 3% |
| V-HHH2B(2F,3F)-O2 | (No.302) | 5% |
| 5-HB-CL | (12-2) | 9 % |
| 3-HH-4 | (2-1) | 8% |
| 3-HHB-1 | (3-1) | 5% |
| 3-HHB(F,F)-F | (13-3) | 7% |
| 3-HBB(F,F)-F | (13-24) | 17% |
| 5-HBB(F,F)-F | (13-24) | 15% |
| 3-HHEB(F,F)-F | (13-12) | 9 % |
| 4-HHEB(F,F)-F | (13-12) | 3% |
| 5-HHEB(F,F)-F | (13-12) | 3% |
| 2-HBEB(F,F)-F | (13-39) | 3% |
| 3-HBEB(F,F)-F | (13-39) | 5% |
| 5-HBEB(F,F)-F | (13-39) | 3% |
| 3-HHBB(F,F)-F | (14-6) | 5% |

### Use Example 8

| | | |
|---|---|---|
| 3-BH1OB(2F)B(2F,3F)-O2 | (No.237) | 3% |
| 3-B2HB(F)B(2F,3F)-3 | (No.108) | 4% |
| 3-HB-CL | (12-2) | 6% |
| 5-HB-CL | (12-2) | 4% |
| 3-HHB-OCF3 | (13-1) | 5% |
| 3-H2HB-OCF3 | (13-13) | 4% |
| 5-H4HB-OCF3 | (13-19) | 14% |
| V-HHB(F)-F | (13-2) | 4% |
| 3-HHB(F)-F | (13-2) | 5% |
| 5-HHB(F)-F | (13-2) | 5% |
| 3-H4HB(F,F)-CF3 | (13-21) | 7% |
| 5-H4HB(F,F)-CF3 | (13-21) | 9% |
| 5-H2HB(F,F)-F | (13-15) | 5% |
| 5-H4HB(F,F)-F | (13-21) | 6% |
| 2-H2BB(F)-F | (13-26) | 4% |
| 3-H2BB(F)-F | (13-26) | 10% |
| 3-HBEB(F,F)-F | (13-39) | 5% |

### Use Example 9 (not within the scope of the invention)

| | | |
|---|---|---|
| V-HHDh1OB(2F,3F)-O2 | (No.446) | 5% |
| 5-HB-CL | (12-2) | 17% |
| 7-HB(F,F)-F | (12-4) | 3% |
| 3-HH-4 | (2-1) | 8% |
| 3-HH-5 | (2-1) | 5% |
| 3-HB-O2 | (2-5) | 15% |
| 3-HHB-1 | (3-1) | 7% |
| 3-HHB-O1 | (3-1) | 5% |
| 2-HHB(F)-F | (13-2) | 6% |
| 3-HHB(F)-F | (13-2) | 6% |
| 5-HHB(F)-F | (13-2) | 7% |
| 3-HHB(F,F)-F | (13-3) | 6% |
| 3-H2HB(F,F)-F | (13-15) | 5% |
| 4-H2HB(F,F)-F | (13-15) | 5% |

### Use Example 10

| | | |
|---|---|---|
| V-HH2HB (2F,3F)-O2 | (No.122) | 3 % |
| 5-HB-CL | (12-2) | 3 % |
| 7-HB(F)-F | (12-3) | 7 % |
| 3-HH-4 | (2-1) | 9% |
| 3-HH-5 | (2-1) | 10% |
| 3-HB-O2 | (2-5) | 13% |
| 3-HHEB-F | (13-10) | 7% |
| 5-HHEB-F | (13-10) | 7% |
| 3-HHEB(F,F)-F | (13-12) | 10% |
| 4-HHEB(F,F)-F | (13-12) | 5% |
| 3-GHB(F,F)-F | (13-109) | 5 % |
| 4-GHB(F,F)-F | (13-109) | 5% |
| 5-GHB(F,F)-F | (13-109) | 7% |
| 2-HHB(F,F)-F | (13-3) | 4% |
| 3-HHB(F,F)-F | (13-3) | 5% |

NI = 74.5°C; η = 19.1 mPa·s; Δn = 0.069; Δε = 5.4.

### Use Example 11

| | | |
|---|---|---|
| V-HH2BB(2F,3F)-O2 | (No.159) | 3 % |
| 1V2-BEB(F,F)-C | (15-13) | 6 % |
| 3-HB-C | (15-1) | 18 % |
| 2-BTB-1 | (2-10) | 10% |
| 5-HH-VFF | (2-1) | 27% |
| 3-HHB-1 | (3-1) | 4% |
| VFF-HHB-1 | (3-1) | 7% |
| VFF2-HHB-1 | (3-1) | 11% |
| 3-H2BTB-2 | (3-17) | 6% |
| 3-H2BTB-3 | (3-17) | 4% |
| 3-H2BTB-4 | (3-17) | 4% |

NI = 87.3°C; η = 13.6 mPa·s; Δn = 0.136; Δε = 6.3.

### Use Example 12

| | | |
|---|---|---|
| V-HH2BB(2F,3F)-O4 | (No.160) | 5% |
| 5-HB(F)B(F,F)XB(F,F)-F | (14-41) | 5% |
| 3-BB(F)B(F,F)XB(F,F)-F | (14-47) | 3% |
| 4-BB(F)B(F,F)XB(F,F)-F | (14-47) | 5% |
| 5-BB(F)B(F,F)XB(F,F)-F | (14-47) | 3% |
| 3-HH-V | (2-1) | 41% |
| 3-HH-V1 | (2-1) | 6% |
| 3-HHEH-5 | (3-13) | 3% |
| 3-HHB-1 | (3-1) | 4% |
| V-HHB-1 | (3-1) | 5% |
| V2-BB(F)B-1 | (3-6) | 4% |
| 1V2-BB-F | (12-1) | 3% |
| 3-BB(F,F)XB(F,F)-F | (13-97) | 10% |
| 3-HHBB(F,F)-F | (14-6) | 3% |

### Use Example 13

| | | |
|---|---|---|
| V2-HH2BB(2F,3F)-O2 | (No.163) | 3% |
| V-H2BBB(2F,3F)-O2 | (No.76) | 3% |
| 3-GB(F)B(F,F)XB(F,F)-F | (14-57) | 5% |
| 3-BB(F)B(F,F)XB(F,F)-F | (14-47) | 3% |
| 4-BB(F)B(F,F)XB(F,F)-F | (14-47) | 7% |
| 5-BB(F)B(F,F)XB(F,F)-F | (14-47) | 3% |
| 3-HH-V | (2-1) | 38% |
| 3-HH-V1 | (2-1) | 7% |
| 3-HHEH-5 | (3-13) | 3% |
| 3-HHB-1 | (3-1) | 3% |
| V-HHB-1 | (3-1) | 3% |
| V2-BB(F)B-1 | (3-6) | 5% |
| 1V2-BB-F | (12-1) | 3% |
| 3-BB(F,F)XB(F,F)-F | (13-97) | 6% |
| 3-GB(F,F)XB(F,F)-F | (13-113) | 5% |
| 3-HHBB(F,F)-F | (14-6) | 3% |

A liquid crystal compound of the invention has good physical properties. A liquid crystal composition containing the compound can be widely applied to a liquid crystal display device used in a personal computer, a television and so forth.

## Claims

1. A compound, represented by formula (1): 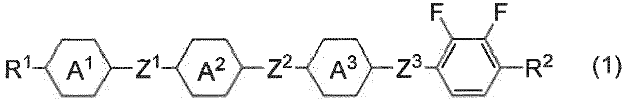 wherein, in formula (1),
R¹ is alkyl having 1 to 15 carbons, and in the alkyl, at least one piece of -CH₂- may be replaced by -O-, and one piece of -CH₂CH₂- may be replaced by -CH=CH-, and R² is alkyl having 1 to 15 carbons, alkoxy having 1 to 15 carbons or alkenyl having 2 to 15 carbons;
ring A¹ and ring A² are independently 1,4-cyclohexylene, 1,4-phenylene, tetrahydropyran-2,5-diyl, 1,3-dioxane-2,5-diyl, pyridine-2,5-diyl or pyrimidine-2,5-diyl, and ring A³ is 1,4-cyclohexylene, 1,4-phenylene, 2-fluoro-1,4-phenylene, tetrahydropyran-2,5-diyl, 1,3-dioxane-2,5-diyl, pyridine-2,5-diyl or pyrimidine-2,5-diyl; and
Z¹, Z² and Z³ are independently a single bond, -COO-, -OCO-, -CH₂CH₂- or -CH=CH-, and at least one of Z¹, Z² and Z³ is -COO-, -OCO-, -CH₂CH₂- or -CH=CH-,
in which, when ring A³ is 2-fluoro-1,4-phenylene, at least one of ring A¹ and ring A² is 1,4-phenylene, tetrahydropyran-2,5-diyl, 1,3-dioxane-2,5-diyl, pyridine-2,5-diyl or pyrimidine-2,5-diyl, and Z³ is a single bond,
in which, when R¹ is CH₂=CH- or alkenyloxy, Z¹ and Z³ each are a single bond and Z² is -CH=CH-, at least one of ring A¹, ring A² and ring A³ is 1,4-phenylene, tetrahydropyran-2,5-diyl, 1,3-dioxane-2,5-diyl, pyridine-2,5-diyl or pyrimidine-2,5-diyl.

2. The compound according to claim 1, wherein, in formula (1), R¹ is alkenyl having 2 to 10 carbons, and R² is alkyl having 1 to 15 carbons, alkoxy having 1 to 15 carbons or alkenyl having 2 to 15 carbons; ring A¹ is 1,4-cyclohexylene, and ring A² and ring A³ are independently 1,4-cyclohexylene, 1,4-phenylene, tetrahydropyran-2,5-diyl, 1,3-dioxane-2,5-diyl, pyridine-2,5-diyl or pyrimidine-2,5-diyl; and Z¹, Z² and Z³ are independently a single bond, -COO-, -OCO- or -CH₂CH₂-, and at least one of Z¹, Z² and Z³ is -COO-, -OCO- or -CH₂CH₂-.

3. The compound according to claim 1, represented by formula (1-1), formula (1-2) or formula (1-3): 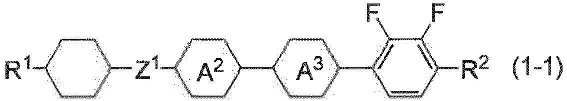 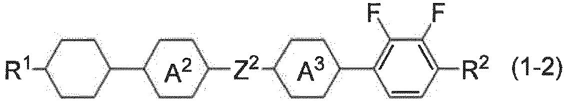 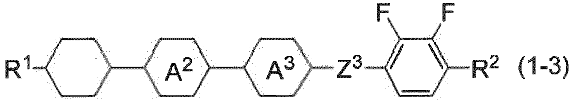 wherein, in formula (1-1) to formula (1-3),
R¹ is alkyl having 1 to 10 carbons, and in the alkyl, at least one piece of -CH₂- may be replaced by -O-, and one piece of -CH₂CH₂- may be replaced by -CH=CH-, and R² is alkyl having 1 to 10 carbons, alkoxy having 1 to 10 carbons or alkenyl having 2 to 10 carbons;
ring A² and ring A³ are independently 1,4-cyclohexylene, 1,4-phenylene, tetrahydropyran-2,5-diyl, 1,3-dioxane-2,5-diyl, pyridine-2,5-diyl or pyrimidine-2,5-diyl; and
Z¹, Z² and Z³ are independently -COO-, -OCO- or -CH₂CH₂-.

4. The compound according to claim 1, represented by any one of formula (1-4) to formula (1-7): 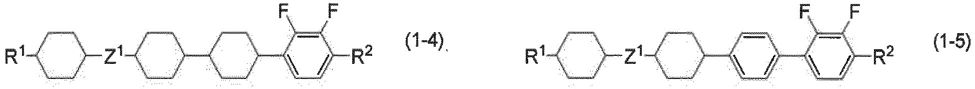 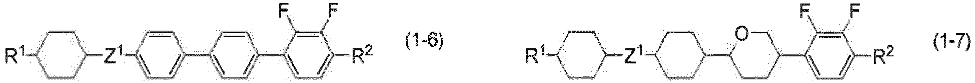 wherein, in formula (1-4) to formula (1-7), R¹ and R² are independently alkyl having 1 to 10 carbons, alkoxy having 1 to 10 carbons or alkenyl having 2 to 10 carbons; and Z¹ is -COO-, -OCO- or -CH₂CH₂-.

5. The compound according to claim 1, represented by any one of formula (1-8) to formula (1-10): 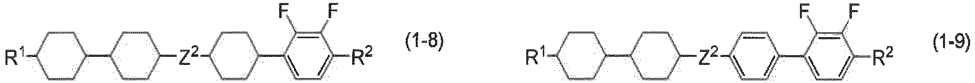 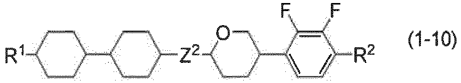 wherein, in formula (1-8) to formula (1-10), R¹ and R² are independently alkyl having 1 to 10 carbons, alkoxy having 1 to 10 carbons or alkenyl having 2 to 10 carbons; and Z² is -COO-, -OCO- or -CH₂CH₂-.

6. The compound according to claim 1, represented by any one of formula (1-11) or formula (1-12): 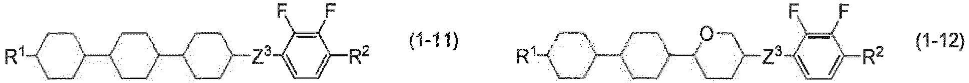 wherein, in formula (1-11) or formula (1-12), R¹ and R² are alkyl having 1 to 10 carbons, alkoxy having 1 to 10 carbons or alkenyl having 2 to 10 carbons; and Z³ is -COO-, -OCO-or -CH₂CH₂-.

7. The compound according to claim 1, represented by any one of formula (1-13) to formula (1-15), formula (1-19) to formula (1-22) and formula (1-24): 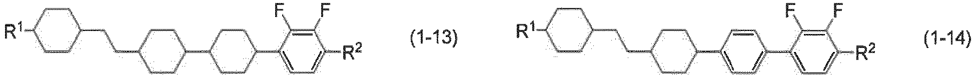 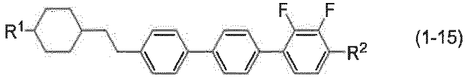 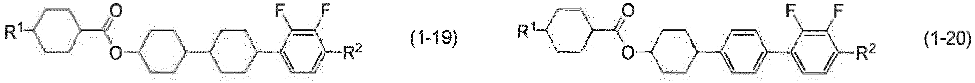 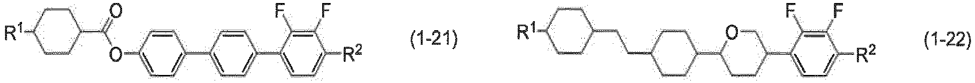 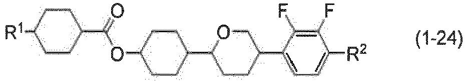 wherein, in formula (1-13) to formula (1-15), formula (1-19) to formula (1-22) and formula (1-24), R¹ is alkenyl having 2 to 5 carbons, and R² is alkyl having 1 to 5 carbons, alkoxy having 1 to 5 carbons or alkenyl having 2 to 5 carbons.

8. The compound according to claim 1, represented by any one of formula (1-25), formula (1-26) and formula (1-29) to formula (1-31): 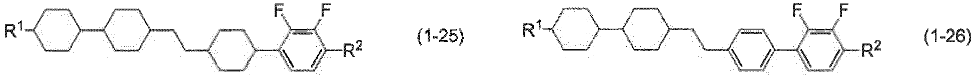 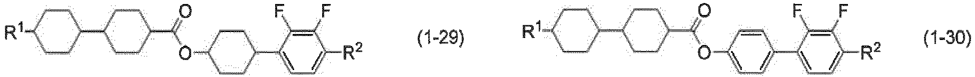 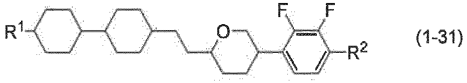 wherein, in formula (1-25), formula (1-26) and formula (1-29) to formula (1-31), R¹ is alkenyl having 2 to 5 carbons, and R² is alkyl having 1 to 5 carbons, alkoxy having 1 to 5 carbons or alkenyl having 2 to 5 carbons.

9. The compound according to claim 1, represented by any one of formula (1-32), formula (1-34) and formula (1-36) : 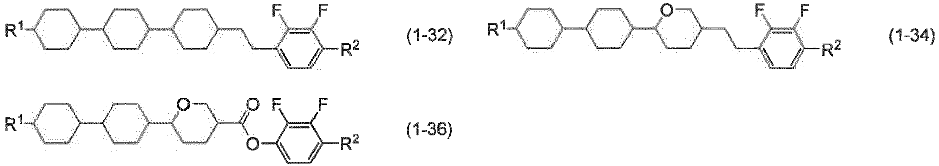 wherein, in formula (1-32), formula (1-34) and formula (1-36), R¹ is alkenyl having 2 to 5 carbons, and R² is alkyl having 1 to 5 carbons, alkoxy having 1 to 5 carbons or alkenyl having 2 to 5 carbons.

10. A liquid crystal composition, containing at least one compound according to any one of claims 1 to 9.

11. The liquid crystal composition according to claim 10, further containing at least one compound selected from the group of compounds represented by formulas (2) to (4) : 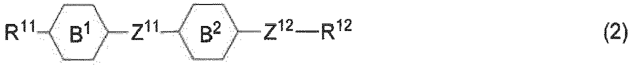 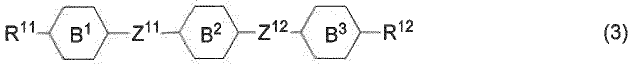 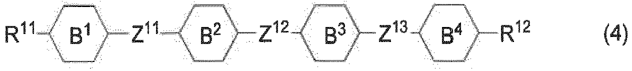 wherein, in formulas (2) to (4),
R¹¹ and R¹² are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
ring B¹, ring B², ring B³ and ring B⁴ are independently 1,4-cyclohexylene, 1,4-phenylene, 2-fluoro-1,4-phenylene, 2,5-difluoro-1,4-phenylene or pyrimidine-2,5-diyl; and
Z¹¹, Z¹² and Z¹³ are independently a single bond, -COO-, -CH₂CH₂-, -CH=CH- or -C=C-.

12. The liquid crystal composition according to claim 11, further containing at least one compound selected from the group of compounds represented by formulas (5) to (11) : 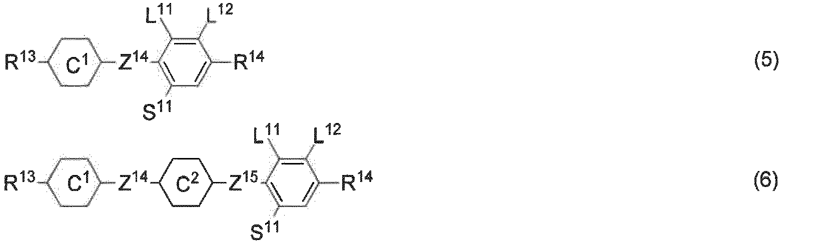 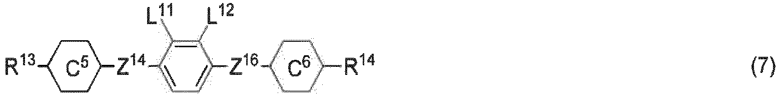 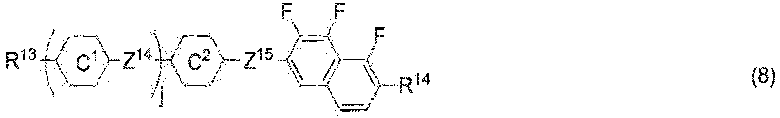 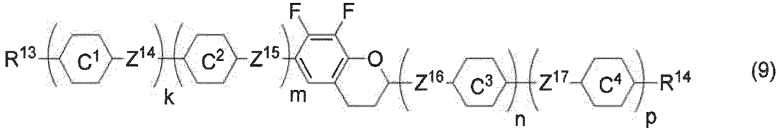 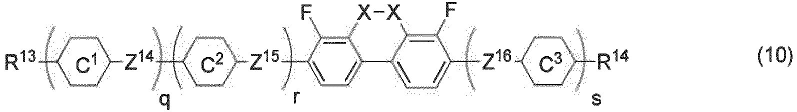 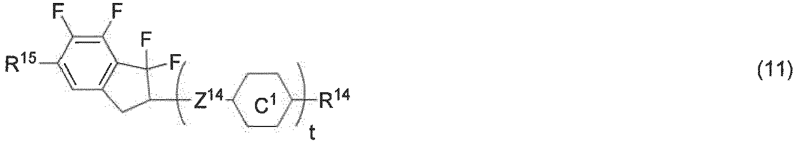 wherein, in formulas (5) to (11),
R¹³, R¹⁴ and R¹⁵ are independently alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine, and R¹⁵ may be hydrogen or fluorine;
ring C¹, ring C², ring C³ and ring C⁴ are independently 1,4-cyclohexylene, 1,4-cyclohexenylene, 1,4-phenylene, 1,4-phenylene in which at least one hydrogen is replaced by fluorine, tetrahydropyran-2,5-diyl or decahydronaphthalene-2,6-diyl;
ring C⁵ and ring C⁶ are independently 1,4-cyclohexylene, 1,4-cyclohexenylene, 1,4-phenylene, tetrahydropyran-2,5-diyl or decahydronaphthalene-2,6-diyl;
Z¹⁴, Z¹⁵, Z¹⁶ and Z¹⁷ are independently a single bond, -COO-, -CH₂O-, -OCF₂-, -CH₂CH₂- or -OCF₂CH₂CH₂-;
L¹¹ and L¹² are independently fluorine or chlorine;
S¹¹ is hydrogen or methyl;
X is -CHF- or -CF₂-; and
j, k, m, n, p, q, r and s are independently 0 or 1, and a sum of k, m, n and p is 1 or 2, and a sum of q, r and s is 0, 1, 2 or 3, and t is 1, 2, or 3.

13. The liquid crystal composition according to claim 11 or 12, further containing at least one compound selected from the group of compounds represented by formulas (12) to (14): 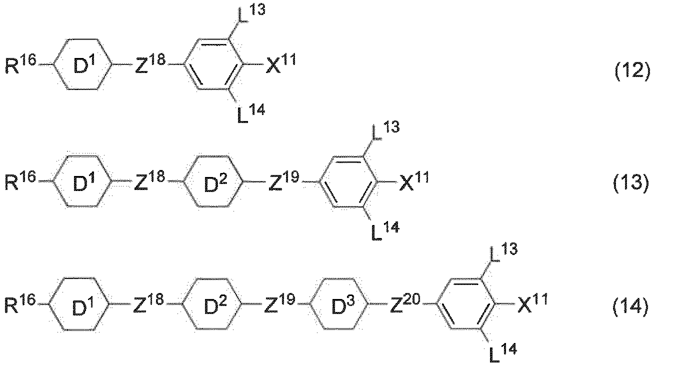 wherein, in formulas (12) to (14),
R¹⁶ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹¹ is fluorine, chlorine, -CF₃, -CHF₂, -CH₂F, -OCF₃, -OCHF₂, -OCF₂CHF₂ or -OCF₂CHFCF₃;
ring D¹, ring D² and ring D³ are independently 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z¹⁸, Z¹⁹ and Z²⁰ are independently a single bond, -COO-, -CH₂O-, -CF₂O-, -OCF₂-, -CH₂CH₂-, -CH=CH-, -C≡C- or -(CH₂)₄-; and
L¹³ and L¹⁴ are independently hydrogen or fluorine.

14. The liquid crystal composition according to any one of claims 11 to 13, further containing at least one compound selected from the group of compounds represented by formula (15): 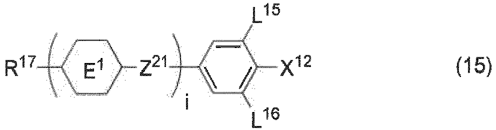 wherein, in formula (15),
R¹⁷ is alkyl having 1 to 10 carbons or alkenyl having 2 to 10 carbons, and in the alkyl and the alkenyl, at least one piece of -CH₂- may be replaced by -O-, and in the groups, at least one hydrogen may be replaced by fluorine;
X¹² is -C≡N or -C≡C-C≡N;
ring E¹ is 1,4-cyclohexylene, 1,4-phenylene, 1,4-phenylene in which at least one hydrogen is replaced by fluorine, tetrahydropyran-2,5-diyl, 1,3-dioxane-2,5-diyl or pyrimidine-2,5-diyl;
Z²¹ is a single bond, -COO-, -CH₂O-, -CF₂O-, -OCF₂-, -CH₂CH₂- or -C≡C-;
L¹⁵ and L¹⁶ are independently hydrogen or fluorine; and
i is 1, 2, 3 or 4.

15. A liquid crystal display device, including the liquid crystal composition according to any one of claims 10 to 14.

## Patentansprüche

1. Verbindung, dargestellt durch Formel (1): 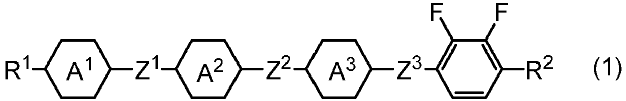 worin in Formel (1)
R¹ Alkyl mit 1 bis 15 Kohlenstoffen ist und in dem Alkyl mindestens ein -CH₂-Stück durch -O- ersetzt sein kann und ein -CH₂CH₂- Stück durch -CH=CH- ersetzt sein kann, und R² Alkyl mit 1 bis 15 Kohlenstoffen, Alkoxy mit 1 bis 15 Kohlenstoffen oder Alkenyl mit 2 bis 15 Kohlenstoffen ist;
Ring A¹ und Ring A² unabhängig voneinander 1,4-Cyclohexylen, 1,4-Phenylen, Tetrahydropyran-2,5-diyl, 1,3-Dioxan-2,5-diyl, Pyridin-2,5-diyl oder Pyrimidin-2,5-diyl sind, und Ring A³ 1,4-Cyclohexylen, 1,4-Phenylen, 2-Fluor-1,4-phenylen, Tetrahydropyran-2,5-diyl, 1,3-Dioxan-2,5-diyl, Pyridin-2,5-diyl oder Pyrimidin-2,5-diyl ist; und
Z¹, Z² und Z³ unabhängig voneinander eine Einfachbindung, -COO-, -OCO-, -CH₂CH₂- oder -CH=CH- sind, und mindestens eines von Z¹, Z² und Z³ -COO-, -OCO-, -CH₂CH₂- oder -CH=CH- ist,
worin, wenn Ring A³ 2-Fluor-1,4-phenylen ist, mindestens einer von Ring A¹ und Ring A² 1,4-Phenylen, Tetrahydropyron-2,5-diyl, 1,3-Dioxan-2,5-diyl, Pyridin-2,5-diyl oder Pyrimidin-2,5-diyl ist, und Z³ eine Einfachbindung ist, worin, wenn R¹ CH₂=CH- oder Alkenyloxy ist, Z¹ und Z³ jeweils eine Einfachbindung sind und Z₂-CH=CH- ist, mindestens einer von Ring A¹, Ring A² und Ring A³ 1,4-Phenylen, Tetrahydropyran-2,5-diyl, 1,3-Dioxan-2,5-diyl, Pyridin-2,5-diyl oder Pyrimidin-2,5-diyl ist.

2. Verbindung gemäß Anspruch 1, wobei in der Formel (1) R¹ Alkenyl mit 2 bis 10 Kohlenstoffen ist und R² Alkyl mit 1 bis 15 Kohlenstoffen, Alkoxy mit 1 bis 15 Kohlenstoffen oder Alkenyl mit 2 bis 15 Kohlenstoffen ist; Ring A¹ 1,4-Cyclohexylen ist, und Ring A² und Ring A³ unabhängig voneinander 1,4-Cyclohexylen, 1,4-Phenylen, Tetrahydropyran-2,5-diyl, 1,3-Dioxan-2,5-diyl, Pyridin-2,5-diyl und Pyrimidin-2,5-diyl sind; und
Z¹, Z² und Z³ unabhängig voneinander eine Einfachbindung, -COO-, -OCO-oder -CH₂CH₂- sind, und mindestens eines von Z¹, Z² und Z³ -COO-, -OCO-oder -CH₂CH₂- ist.

3. Verbindung gemäß Anspruch 1, dargestellt durch Formel (1-1), Formel (1-2) oder Formel (1-3): 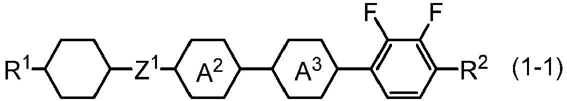 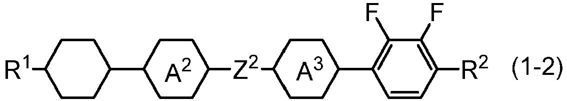 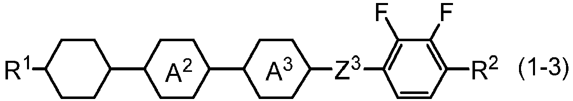 worin in Formel (1-1) bis Formel (1-3)
R¹ Alkyl mit 1 bis 10 Kohlenstoffen ist und in dem Alkyl mindestens ein -CH₂-Stück durch -O- ersetzt sein kann und -CH₂CH₂- Stück durch -CH=CH- ersetzt sein kann, und R² Alkyl mit 1 bis 10 Kohlenstoffen, Alkoxy mit 1 bis 10 Kohlenstoffen oder Alkenyl mit 2 bis 10 Kohlenstoffen ist;
Ring A² und Ring A³ unabhängig voneinander 1,4-Cyclohexylen, 1,4-Phenylen, Tetrahydropyran-2,5-diyl, 1,3-Dioxan-2,5-diyl, Pyridin-2,5-diyl oder Pyrimidin-2,5-diyl sind; und
Z¹ Z² und Z³ unabhängig voneinander -COO-, -OCO- oder -CH₂CH₂- sind.

4. Verbindung gemäß Anspruch 1, dargestellt durch eine der Formeln (1-4) bis Formel (1-7): 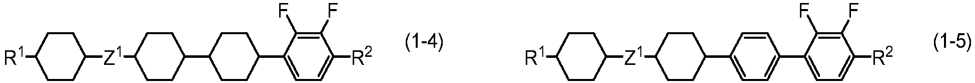 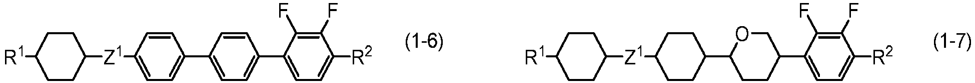 worin in Formel (1-4) bis Formel (1-7) R¹ und R² unabhängig voneinander Alkyl mit 1 bis 10 Kohlenstoffen, Alkoxy mit 1 bis 10 Kohlenstoffen oder Alkenyl mit 2 bis 10 Kohlenstoffen sind; und Z¹ -COO-, -OCO- oder -CH₂CH₂- ist.

5. Verbindung gemäß Anspruch 1, dargestellt durch eine der Formeln (1-8) bis Formel (1-10): 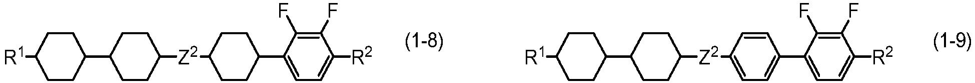 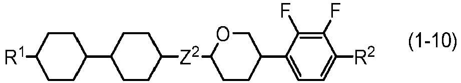 worin in Formel (1-8) bis Formel (1-10) R¹ und R² unabhängig voneinander Alkyl mit 1 bis 10 Kohlenstoffen, Alkoxy mit 1 bis 10 Kohlenstoffen oder Alkenyl mit 2 bis 10 Kohlenstoffen sind; und Z² -COO-, -OCO- oder -CH₂CH₂- ist.

6. Verbindung gemäß Anspruch 1, dargestellt durch eine der Formeln (1-11) bis Formel (1-12): 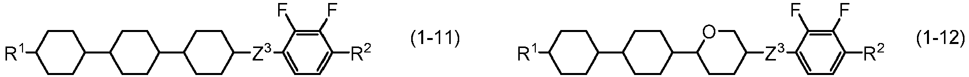 worin in Formel (1-11) bis Formel (1-12) R¹ und R² unabhängig voneinander Alkyl mit 1 bis 10 Kohlenstoffen, Alkoxy mit 1 bis 10 Kohlenstoffen oder Alkenyl mit 2 bis 10 Kohlenstoffen sind; und Z² -COO-, -OCO- oder -CH₂CH₂- ist.

7. Verbindung gemäß Anspruch 1, dargestellt durch eine der Formel (1-13) bis Formel (1-15), Formel (1-19) bis Formel (1-22) und Formel (1-24): 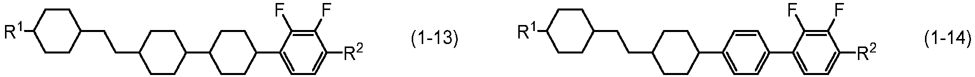 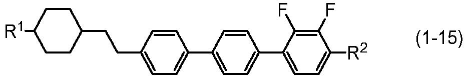 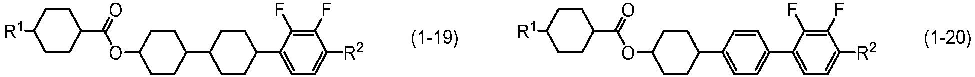 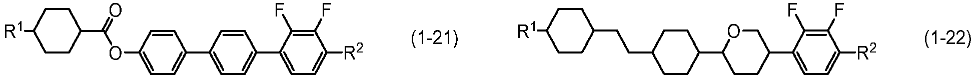 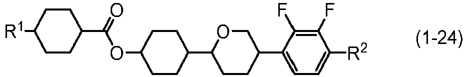 worin in Formel (1-13) bis Formel (1-15), Formel (1-19) bis Formel (1-22) und Formel (1-24) R¹ Alkenyl mit 2 bis 5 Kohlenstoffen ist und R² Alkyl mit 1 bis 5 Kohlenstoffen, Alkoxy mit 1 - 5 Kohlenstoffen oder Alkenyl mit 2 bis 5 Kohlenstoffen ist.

8. Verbindung gemäß Anspruch 1, dargestellt durch eine der Formel (1-25), Formel (1-26) und Formel (1-29) bis Formel (1-31): 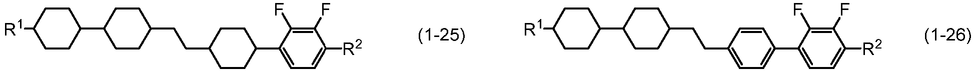 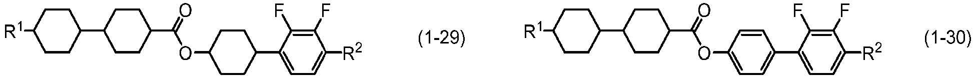 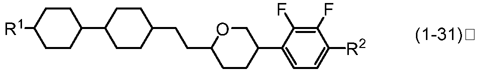 worin in Formel (1-25), Formel (1-26) und Formel (1-29) bis Formel (1-31) R¹ Alkenyl mit 2 bis 5 Kohlenstoffen ist und R² Alkyl mit 1 bis 5 Kohlenstoffen, Alkoxy mit 1 - 5 Kohlenstoffen oder Alkenyl mit 2 bis 5 Kohlenstoffen ist.

9. Verbindung gemäß Anspruch 1, dargestellt durch eine der Formel (1-32), Formel (1-34) und Formel (1-36): 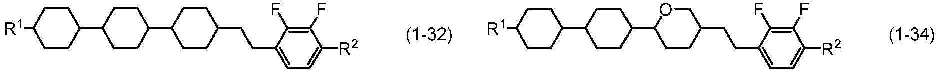 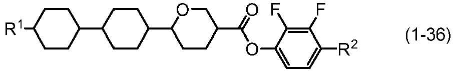 worin in Formel (1-32), Formel (1346) und Formel (1-36) R¹ Alkenyl mit 2 bis 5 Kohlenstoffen ist und R² Alkyl mit 1 bis 5 Kohlenstoffen, Alkoxy mit 1 - 5 Kohlenstoffen oder Alkenyl mit 2 bis 5 Kohlenstoffen ist.

10. Flüssigkristallzusammensetzung, enthaltend mindestens eine Verbindung gemäß einem der Ansprüche 1 bis 9.

11. Flüssigkristallzusammensetzung gemäß Anspruch 10, weiter enthaltend mindestens eine Verbindung, ausgewählt aus der Gruppe an Verbindungen, dargestellt durch Formeln (2) bis (4): 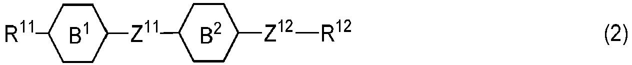 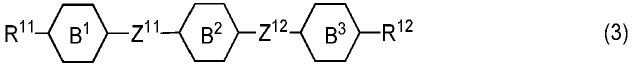 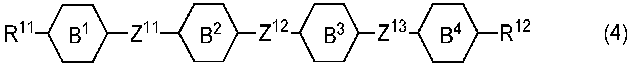 worin in den Formeln (2) bis (4)
R¹¹ und R¹² unabhängig voneinander Alkyl mit 1 bis 10 Kohlenstoffen oder Alkenyl mit 2 bis 10 Kohlenstoff sind, und in dem Alkyl und dem Alkenyl mindestens ein -CH₂- Stück durch -O- ersetzt sein kann und in den Gruppen mindestens ein Wasserstoff durch Fluor ersetzt sein kann;
Ring B¹, Ring B², Ring B³ und Ring B⁴ unabhängig voneinander 1,4-Cyclohexylen, 1,4-Phenylen, 2-Fluor-1,4-phenylen, 2,5-Difluor-1,4-phenylen oder Pyrimidin-2,5-diyl sind; und
Z¹¹, Z¹² und Z¹³ unabhängig voneinander eine Einfachbindung, -COO-, -CH₂CH₂-, -CH=CH- oder -C≡C- sind.

12. Flüssigkristallzusammensetzung gemäß Anspruch 11, weiter enthaltend mindestens eine Verbindung, ausgewählt aus der Gruppe von Verbindungen, dargestellt durch Formeln (5) bis (11): 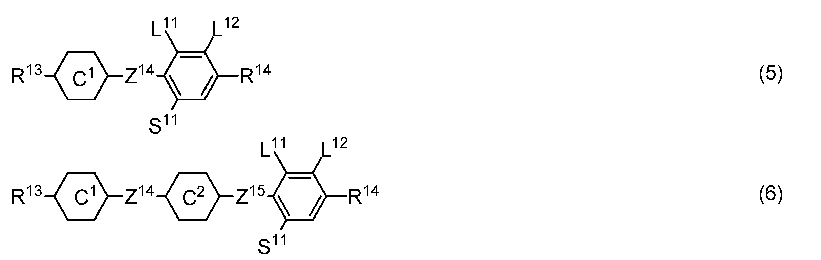 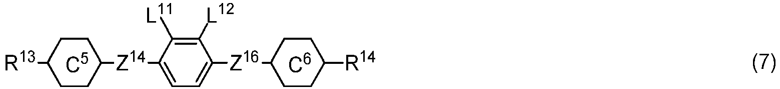 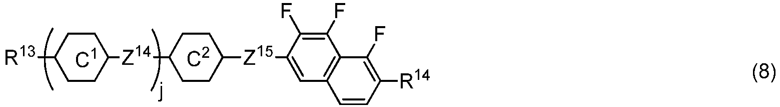 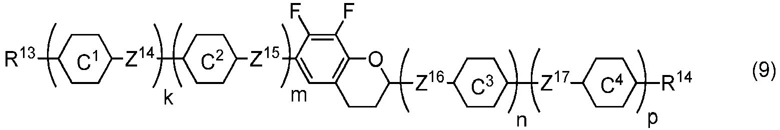 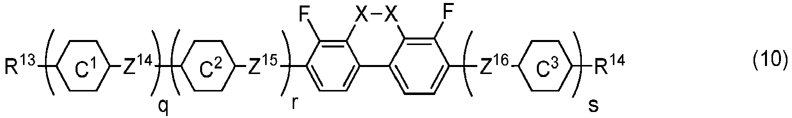 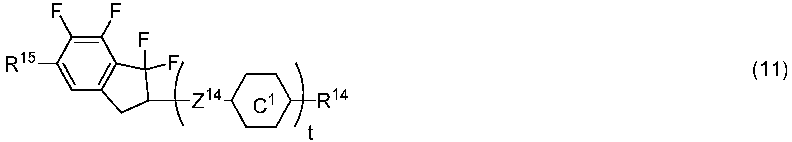 worin in Formeln (5) bis (11)
R¹³, R¹⁴ und R¹⁵ unabhängig voneinander Alkyl mit 1 bis 10 Kohlenstoffen oder Alkenyl mit 2 bis 10 Kohlenstoffen sind und in dem Alkyl und dem Alkenyl mindestens ein -CH₂- Stück durch -O- ersetzt sein kann und in den Gruppen mindestens ein Wasserstoff durch Fluor ersetzt sein kann, und R¹⁵ Wasserstoff oder Fluor sein kann;
Ring C¹, Ring C², Ring C³ und Ring C⁴ unabhängig voneinander 1,4-Cyclohexylen, 1,4-Cyclohexenylen, 1,4-Phenylen, 1,4-Phenylen worin mindestens ein Wasserstoff durch Fluor ersetzt sein kann, Tetrahydropyran-2,5-diyl oder Decahydronaphthalin-2-6-diyl sind;
Ring C⁵ und Ring C⁶ unabhängig voneinander 1,4-Cyclohexylen, 1,4-Cyclohexenylen, 1,4-Phenylen, Tetrahydropyran-2,5-diyl oder Decahydronaph-thalen-2,6-diyl sind;
Z¹⁴, Z¹⁵, Z¹⁶ und Z¹⁷ unabhängig voneinander eine Einfachbindung, -COO-, -CH₂O-, -OCF₂-, -CH₂CH₂- oder -OCF₂CH₂CH₂- sind;
L¹¹ und L¹² unabhängig voneinander Fluor oder Chlor sind;
S¹¹ Wasserstoff oder Methyl ist;
X -CHF- oder -CF₂- ist, und
j, k, m, n, p, q, r und s unabhängig voneinander 0 oder 1 sind, und eine Summe von k, m, n und p 1 oder 2 ist, und eine Summe von q, r und s 0, 1, 2 oder 3 ist, und t 1, 2 oder 3 ist.

13. Flüssigkristallzusammensetzung gemäß Anspruch 11 oder 12, weiter enthaltend mindestens eine Verbindung, ausgewählt aus der Gruppe von Verbindungen, dargestellt durch Formeln (12) bis (14): 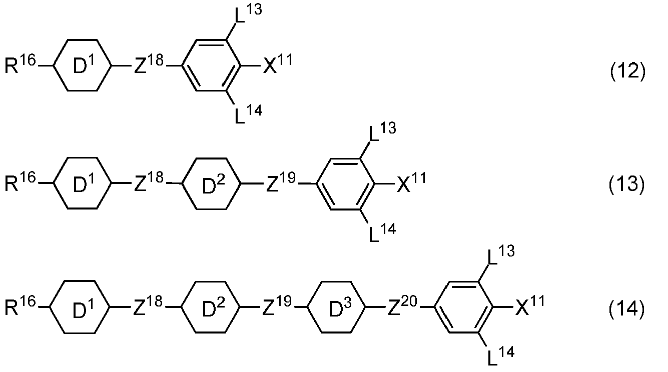 worin in Formeln (12) bis (14)
R¹⁶ Alkyl mit 1 bis 10 Kohlenstoffen oder Alkenyl mit 2 bis 10 Kohlenstoffen ist,
und in dem Alkyl und dem Alkenyl mindestens ein -CH₂- Stück durch -O- ersetzt sein kann, und in den Gruppen mindestens ein Wasserstoff durch Fluor ersetzt sein kann;
X¹¹ Fluor, Chlor, -CF₃, -CHF₂, -CH₂F, -OCF₃, -OCHF₂, -OCF₂CHF₂ oder -OCF₂CHFCF₃ ist;
Ring D¹, Ring D² und Ring D³ unabhängig voneinander 1,4-Cyclohexylen, 1,4-Phenylen, 1,4-Phenylen worin mindestens ein Wasserstoff durch Fluor ersetzt ist, Tetrahydropyran-2,5-diyl, 1,3-Dioxan-2,5-diyl oder Pyrimidin-2,5-diyl sind; Z¹⁸, Z¹⁹ und Z²⁰ unabhängig voneinander eine Einfachbindung, -COO-, -CH₂O-, -CF₂O-, -OCF₂-, -CH₂CH₂-, -CH=CH-, -C≡C- oder -(CH₂)₄- sind;
L¹³ und L¹⁴ unabhängig voneinander Wasserstoff oder Fluor sind.

14. Flüssigkristallzusammensetzung gemäß einem der Ansprüche 11 bis 13, weiter enthaltend mindestens eine Verbindung, dargestellt durch die Gruppe von Verbindungen, dargestellt durch Formel (15): 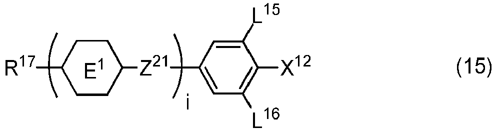 worin in Formel (15),
R¹⁷ Alkyl mit 1 bis 10 Kohlenstoffen oder Alkenyl mit 2 bis 10 Kohlenstoffen ist, und in dem Alkyl und dem Alkenyl mindestens ein -CH₂- Stück durch -O- ersetzt sein kann, und in den Gruppen mindestens ein Wasserstoff durch Fluor ersetzt sein kann,
X¹² -C≡N or -C≡C-C≡N ist;
Ring E¹ 1,4-Cyclohexylen, 1,4-Phenylen, 1,4-Phenylen worin mindestens ein Wasserstoff durch Fluor ersetzt ist, Tetrahydropyran-2,5-diyl, 1,3-Dioxan-2,5-diyl oder Pyrimidin-2,5-diyl ist;
Z²¹ eine Einfachbindung, -COO-, CH₂O-, -CF₂O-, -OCF₂-, -CH₂CH₂- oder -C≡Cist;
L¹⁵ und L¹⁶ unabhängig voneinander Wasserstoff oder Fluor sind; und
i 1, 2, 3 oder 4 ist.

15. Flüssigkristallanzeigevorrichtung, welche die Flüssigkristallzusammensetzung gemäß einem der Ansprüche 10 bis 14 einschließt.

## Revendications

1. Composé représenté par la formule (1) : 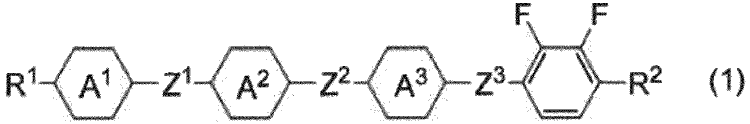 où dans la formule (1),
R¹ est un alkyle ayant 1 à 15 carbones, et dans l'alkyle, au moins un élément -CH₂-peut être remplacé par -O-, et un élément -CH₂CH₂- peut être remplacé par -CH=CH-, et R² est un alkyle ayant 1 à 15 carbones, un alcoxy ayant 1 à 15 carbones, ou un alcényle ayant 2 à 15 carbones ;
le cycle A¹ et le cycle A² sont indépendamment, 1,4-cyclohexylène, 1,4-phénylène, tétrahydropyran-2,5-diyle, 1,3-dioxan-2,5-diyle, pyridin-2,5-diyle ou pyrimidin-2,5-diyle, et le cycle A³ est 1,4-cyclohexylène, 1,4-phénylène, 2-fluoro-1,4-phénylène, tétrahydropyran-2,5-diyle, 1,3-dioxan-2,5-diyle, pyridin-2,5-diyle ou pyrimidin-2,5-diyle ; et
Z¹, Z² et Z³ sont indépendamment une simple liaison, -COO-, -OCO-, -CH₂CH₂- ou - CH=CH-, et au moins l'un parmi Z¹, Z² et Z³ est -COO-, -OCO-, -CH₂CH₂- ou - CH=CH-,
dans lequel, lorsque le cycle A³ est 2-fluoro-1,4-phénylène, au moins l'un du cycle A¹ et du cycle A² est 1,4-phénylène, tétrahydropyran-2,5-diyle, 1,3-dioxan-2,5-diyle, pyridin-2,5-diyle ou pyrimidin-2,5-diyle, et Z³ est une simple liaison,
dans lequel lorsque R¹ est CH₂=CH- ou alcényloxy, Z¹ et Z³ sont chacun, une simple liaison et Z² est -CH=CH-, au moins l'un du cycle A¹, du cycle A² et du cycle A³ est 1,4-phénylène, tétrahydropyran-2,5-diyle, 1,3-dioxan-2,5-diyle, pyridin-2,5-diyle ou pyrimidin-2,5-diyle.

2. Composé selon la revendication 1, dans lequel, dans la formule (1), R¹ est un alcényle ayant 2 à 10 carbones, et R² est un alkyle ayant 1 à 15 carbones, un alcoxy ayant 1 à 15 carbones, ou un alcényle ayant 2 à 15 carbones ; le cycle A¹ est 1,4-cyclohexylène, et le cycle A² et le cycle A³ sont indépendamment, 1,4-cyclohexylène, 1,4-phénylène, tétrahydropyran-2,5-diyle, 1,3-dioxan-2,5-diyle, pyridin-2,5-diyle ou pyrimidin-2,5-diyle ; et Z¹, Z² et Z³ sont indépendamment une simple liaison, -COO-, - OCO- ou -CH₂CH₂-, et au moins l'un parmi Z¹, Z² et Z³ est -COO-, -OCO- ou - CH₂CH₂-.

3. Composé selon la revendication 1, représenté par la formule (1-1), la formule (1-2) ou la formule (1-3) : 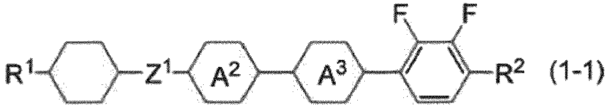 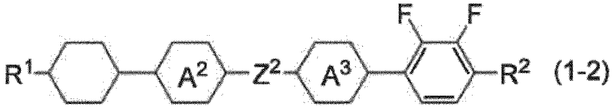 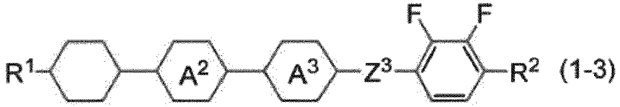 où dans la formule (1-1) à la formule (1-3),
R¹ est un alkyle ayant 1 à 10 carbones, et dans l'alkyle, au moins un élément -CH₂-peut être remplacé par -O-, et un élément -CH₂CH₂- peut être remplacé par -CH=CH-, et R² est un alkyle ayant 1 à 10 carbones, un alcoxy ayant 1 à 10 carbones, ou un alcényle ayant 2 à 10 carbones ;
le cycle A² et le cycle A³ sont indépendamment, 1,4-cyclohexylène, 1,4-phénylène, tétrahydropyran-2,5-diyle, 1,3-dioxan-2,5-diyle, pyridin-2,5-diyle ou pyrimidin-2,5-diyle, et
Z¹, Z² et Z³ sont indépendamment une simple liaison, -COO-, -OCO- ou -CH₂CH₂-.

4. Composé selon la revendication 1, représenté par l'une quelconque de la formule (1-4) à la formule (1-7) : 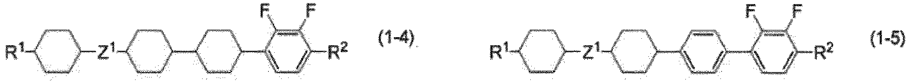 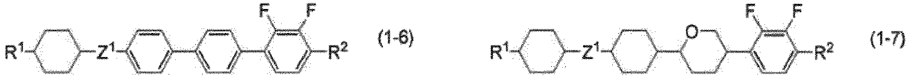 où dans la formule (1-4) à la formule (1-7), R¹ et R² sont indépendamment, alkyle ayant 1 à 10 carbones, alcoxy ayant 1 à 10 carbones ou alcényle ayant 2 à 10 carbones, et Z¹ est -COO-, -OCO- ou -CH₂CH₂-.

5. Composé selon la revendication 1, représenté par l'une quelconque de la formule (1-8) à la formule (1-10) : 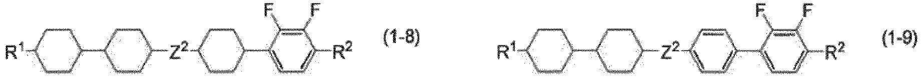 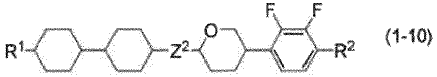 où dans la formule (1-8) à la formule (1-10), R¹ et R² sont indépendamment, alkyle ayant 1 à 10 carbones, alcoxy ayant 1 à 10 carbones ou alcényle ayant 2 à 10 carbones, et Z² est -COO-, -OCO- ou -CH₂CH₂-.

6. Composé selon la revendication 1, représenté par l'une quelconque de la formule (1-11) ou la formule (1-12) : 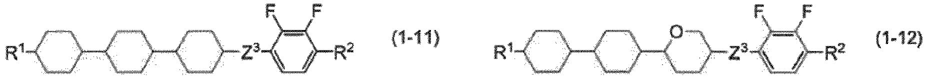 où dans la formule (1-11) ou la formule (1-12), R¹ et R² sont, alkyle ayant 1 à 10 carbones, alcoxy ayant 1 à 10 carbones ou alcényle ayant 2 à 10 carbones, et Z³ est -COO-, -OCO- ou -CH₂CH₂-.

7. Composé selon la revendication 1, représenté par l'une quelconque de la formule (1-13) à la formule (1-15), la formule (1-19) à la formule (1-22) et la formule (1-24) : 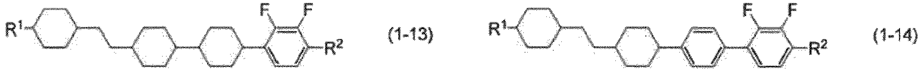 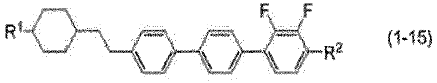 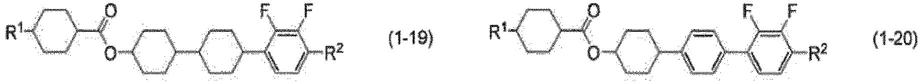 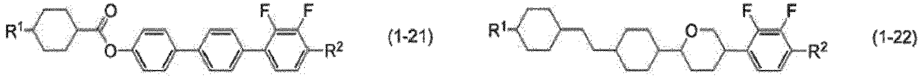 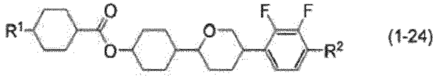 où dans la formule (1-13) à la formule (1-15), la formule (1-19) à la formule (1-22) et la formule (1-24), R¹ est alcényle ayant 2 à 5 carbone, et R² est alkyle ayant 1 à 5 carbones, alcoxy ayant 1 à 5 carbones ou alcényle ayant 2 à 5 carbones.

8. Composé selon la revendication 1, représenté par l'une quelconque de la formule (1-25), la formule (1-26) et la formule (1-29) à la formule (1-31) : 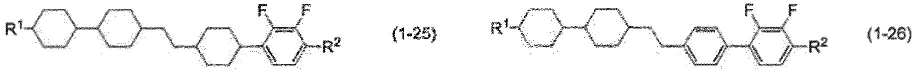 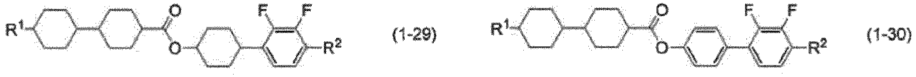 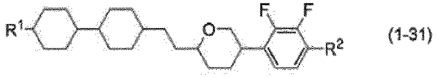 où dans la formule (1-25), la formule (1-26) et la formule (1,29) à la formule (1-31), R¹ est alcényle ayant 2 à 5 carbones, et R² est, alkyle ayant 1 à 5 carbones, alcoxy ayant 1 à 5 carbones ou alcényle ayant 2 à 5 carbones.

9. Composé selon la revendication 1, représenté par l'une quelconque de la formule (1-32), la formule (1-34) et la formule (1-36) 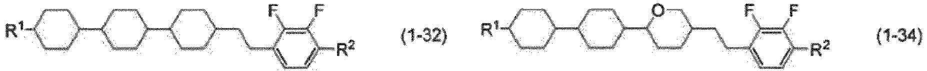 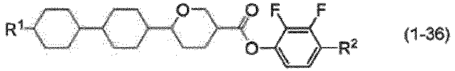 où dans la formule (1-32), la formule (1-34) et la formule (1-36), R¹ est alcényle ayant 2 à 5 carbones, et R² est, alkyle ayant 1 à 5 carbones, alcoxy ayant 1 à 5 carbones ou alcényle ayant 2 à 5 carbones.

10. Composition de cristal liquide contenant au moins un composé selon l'une quelconque des revendications 1 à 9.

11. Composition de cristal liquide selon la revendication 10, contenant en outre au moins un composé choisi parmi le groupe de composés représentés par les formules (2) à (4) : 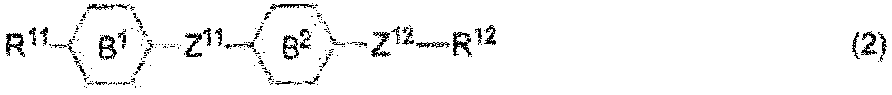 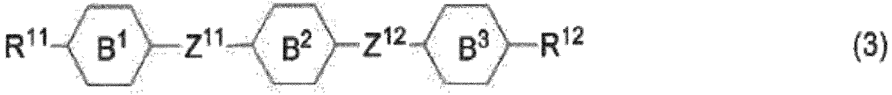 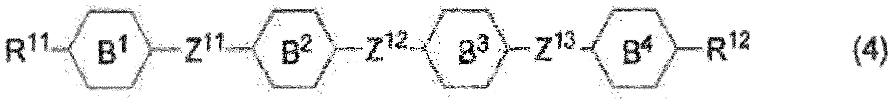 où dans les formules (2) à (4),
R¹¹ et R¹² sont indépendamment alkyle ayant 1 à 10 carbones ou alcényle ayant 2 à 10 carbones, et dans l'alkyle et l'alcényle, au moins un élément -CH₂- peut être remplacé par -O-, et dans les groupes, au moins un hydrogène peut être remplacé par fluor ;
le cycle B¹, le cycle B², le cycle B³ et le cycle B⁴ sont indépendamment, 1,4-cyclohexylène, 1,4-phénylène, 2-fluoro-1,4-phénylène, 2,5-difluoro-1,4-phénylène ou pyrimidin-2,5-diyle ; et Z¹¹, Z¹² et Z¹³ sont indépendamment une simple liaison, -COO-, -CH₂CH₂-, -CH=CH- ou - C≡C-.

12. Composition de cristal liquide selon la revendication 11, contenant en outre au moins un composé choisi parmi le groupe de composés représentés par les formules (5) à (11) : 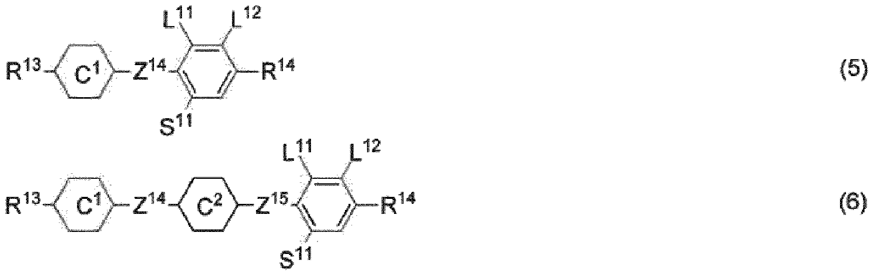 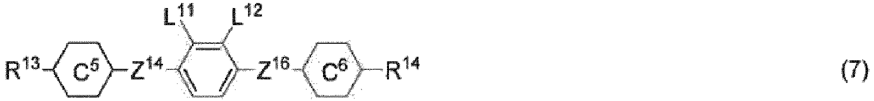 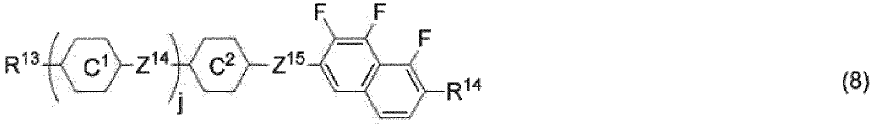 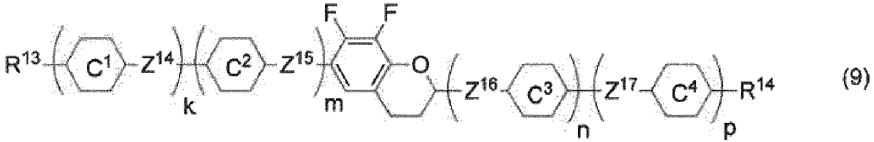 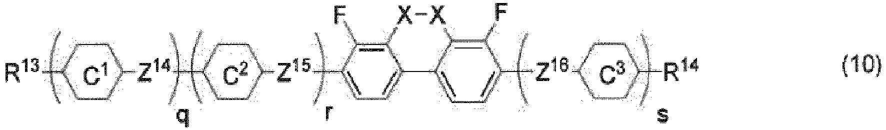 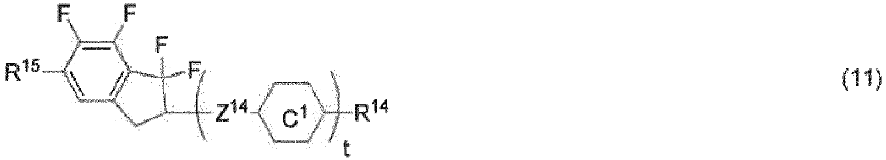 où dans les formules (5) à (11),
R¹³, R¹⁴ et R¹⁵ sont indépendamment alkyle ayant 1à 10 carbones ou alcényle ayant 2 à 10 carbones, et dans l'alkyle et l'alcényle, au moins un élément -CH₂- peut être remplacé par -O-, et dans les groupes, au moins un hydrogène peut être remplacé par fluor, et R¹⁵ peut être hydrogène ou fluor ;
le cycle C¹, le cycle C², le cycle C³ et le cycle C⁴ sont indépendamment, 1,4-cyclohexylène, 1,4-cyclohexènylène, 1,4-phénylène, 1,4-phénylène dans lequel au moins un hydrogène est remplacé par fluor, tétrahydropyran-2,5-diyle ou décahydronaphtalène-2,6-diyle;
le cycle C⁵ et le cycle C⁶ sont indépendamment, 1,4-cyclohexylène, 1,4-cyclohexènylène, 1,4-phénylène, tétrahydropyran-2,5-diyle ou décahydronaphtalène-2,6-diyle ;
Z¹⁴, Z¹⁵, Z¹⁶ et Z¹⁷ sont indépendamment une simple liaison, -COO-, -CH₂O-, -OCF₂-, - CH₂CH₂- ou -OCF₂CH₂CH₂- ;
L¹¹ et L¹² sont indépendamment, fluor ou chlore ;
S¹¹ est hydrogène ou méthyle ;
X est -CHF- ou -CF₂-; et
j, k, m, n, p, q, r et s sont indépendamment, 0 ou 1, et la somme de k, m, n et p vaut 1 ou 2, et la somme de q, r et s vaut 0, 1, 2 ou 3, et t est 1, 2 ou 3.

13. Composition de cristal liquide selon la revendication 11 ou 12, contenant en outre au moins un composé choisi parmi le groupe de composés représentés par les formules (12) à (14) : 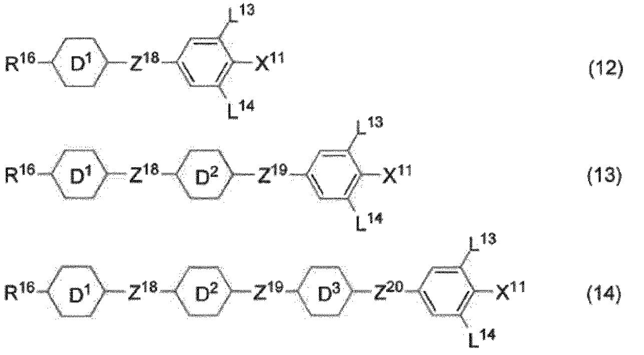 où dans les formules (12) à (14),
R¹⁶ est alkyle ayant 1à 10 carbones ou alcényle ayant 2 à 10 carbones, et dans l'alkyle et l'alcényle, au moins un élément -CH₂- peut être remplacé par -O-, et dans les groupes, au moins un hydrogène peut être remplacé par fluor ;
X¹¹ est fluor, chlore, -CF₃, -CHF₂, -CH₂F, -OCF₃, -OCHF₂, -OCF₂CHF₂ ou -OCF₂CHFCF₃ ; le cycle D¹, le cycle D² et le cycle D³ sont indépendamment, 1,4-cyclohexylène, 1,4-phénylène, 1,4-phénylène dans lequel au moins un hydrogène est remplacé par fluor, tétrahydropyran-2,5-diyle, 1,3-dioxan-2,5-diyle ou pyrimidin-2,5-diyle ;
Z¹⁸, Z¹⁹ et Z²⁰ sont indépendamment une simple liaison, -COO-, -CH₂O-, -CF₂O-, -OCF₂-, - CH₂CH₂-, -CH=CH-, -C≡C- ou -(CH₂)₄- ; et
L¹³ et L¹⁴ sont indépendamment, hydrogène ou fluor.

14. Composition de cristal liquide selon l'une quelconque des revendications 11 à 13, contenant en outre au moins un composé choisi parmi le groupe de composés représentés par la formule (15) : 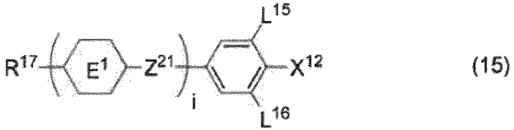 où dans la formule (15),
R¹⁷ est alkyle ayant 1 à 10 carbones ou alcényle ayant 2 à 10 carbones, et dans l'alkyle et l'alcényle, au moins un élément -CH₂- peut être remplacé par -O-, et dans les groupes, au moins un hydrogène peut être remplacé par fluor;
X¹² est -C≡N ou -C≡C-C≡N ;
le cycle E¹ est 1,4-cyclohexylène, 1,4-phénylène, 1,4-phénylène dans lequel au moins un hydrogène est remplacé par fluor, tétrahydropyran-2,5-diyle, 1,3-dioxan-2,5-diyle ou pyrimidin-2,5-diyle ;
Z²¹ est une simple liaison, -COO-, -CH₂O-, -CF₂O-, -OCF₂-, -CH₂CH₂- ou -C≡C- ;
L¹⁵ et L¹⁶ sont indépendamment, hydrogène ou fluor ; et
i est 1, 2, 3 ou 4.

15. Dispositif d'affichage à cristal liquide, comprenant la composition à cristal liquide selon l'une quelconque des revendications 10 à 14.
